# EUROPEAN PATENT APPLICATION

(11) **EP 1 364 653 A1**
(43) Date of publication of application: **26.11.2003**
(21) Application number: 02700664.2
(22) Date of filing: 21.02.2002
(51) Int. Cl.: A61K 38/17, A61K 31/711, A61K 48/00, A61P 43/00, A61P 31/18, A61P 25/28, A61P 25/16, A61P 21/02, A61P 27/02, A61P 25/00, A61P 7/06, A61P 9/10, A61P 9/02, A61P 37/06

(54) **CASOASE 3 INHIBITORS**

(30) Priority: 23.02.2001 JP 2001049453
(71) Applicant: Takeda Chemical Industries, Ltd., Osaka-shi, Osaka 541-0045 (JP)
(72) Inventor: MATSUI, Hideki, Tsukuba-shi, Ibaraki 305-0044 (JP); SHINTANI, Yasushi, Osaka-shi, Osaka 532-0033 (JP); HIKICHI, Yukiko, Tsukuba-shi, Ibaraki 305-0035 (JP)
(74) Representative: Keller, Günter, Dr.
(86) International application number: JP0201537
(87) International publication number: WO02066050

(57) **Abstract**

The invention is intended to develop a drug capable of directly or indirectly inhibiting the activation of caspase. Specifically, the invention provides a caspase 3 inhibitor comprising a protein comprising the same or substantially the same amino acid sequence as that represented by SEQ ID NO: 1, 2, 3 or 31 or a salt thereof. The protein of the invention, a partial peptide or a salt thereof is useful as a pharmaceutical such as a prophylactic and/or therapeutic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.

Sequence Listing

<110> Takeda Chemical Industries, Ltd.

<120> Caspase 3 Inhibitor

<130> P02-0017PCT

<140> PCT/JP02/01537
<141> 2002-02-21

<150> JP 2001-49453
<151> 2001-02-23

<160> 34

<210> 1
<211> 240
<212> PRT
<213> Human

<400> 1
Met Glu Glu Ser Val Val Arg Pro Ser Val Phe Val Val Asp Gly Gln
1               5                   10                  15
Thr Asp Ile Pro Phe Thr Arg Leu Gly Arg Ser His Arg Arg Gln Ser
            20                  25                  30
Cys Ser Val Ala Arg Val Gly Leu Gly Leu Leu Leu Leu Leu Met Gly
        35                  40                  45
Ala Gly Leu Ala Val Gln Gly Trp Phe Leu Leu Gln Leu His Trp Arg
    50                  55                  60
Leu Gly Glu Met Val Thr Arg Leu Pro Asp Gly Pro Ala Gly Ser Trp
65                  70                  75                  80
Glu Gln Leu Ile Gln Glu Arg Arg Ser His Glu Val Asn Pro Ala Ala
                85                  90                  95
His Leu Thr Gly Ala Asn Ser Ser Leu Thr Gly Ser Gly Gly Pro Leu
            100                 105                 110

Leu Trp Glu Thr Gln Leu Gly Leu Ala Phe Leu Arg Gly Leu Ser Tyr
        115                 120                 125
His Asp Gly Ala Leu Val Val Thr Lys Ala Gly Tyr Tyr Tyr Ile Tyr
    130                 135                 140
Ser Lys Val Gln Leu Gly Gly Val Gly Cys Pro Leu Gly Leu Ala Ser
145                 150                 155                 160
Thr Ile Thr His Gly Leu Tyr Lys Arg Thr Pro Arg Tyr Pro Glu Glu
                165                 170                 175
Leu Glu Leu Leu Val Ser Gln Gln Ser Pro Cys Gly Arg Ala Thr Ser
            180                 185                 190
Ser Ser Arg Val Trp Trp Asp Ser Ser Phe Leu Gly Gly Val Val His
        195                 200                 205
Leu Glu Ala Gly Glu Lys Val Val Val Arg Val Leu Asp Glu Arg Leu
    210                 215                 220
Val Arg Leu Arg Asp Gly Thr Arg Ser Tyr Phe Gly Ala Phe Met Val
225                 230                 235                 240

<210> 2
<211> 239
<212> PRT
<213> Murine

<400> 2
Met Glu Ser Val Val Gln Pro Ser Val Phe Val Val Asp Gly Gln Thr
 1               5                  10                  15
Asp Ile Pro Phe Arg Arg Leu Glu Gln Asn His Arg Arg Arg Arg Cys
            20                  25                  30
Gly Thr Val Gln Val Ser Leu Ala Leu Val Leu Leu Leu Gly Ala Gly
        35                  40                  45
Leu Ala Thr Gln Gly Trp Phe Leu Leu Arg Leu His Gln Arg Leu Gly
    50                  55                  60
Asp Ile Val Ala His Leu Pro Asp Gly Gly Lys Gly Ser Trp Glu Lys
65                  70                  75                  80
Leu Ile Gln Asp Gln Arg Ser His Gln Ala Asn Pro Ala Ala His Leu
                85                  90                  95
Thr Gly Ala Asn Ala Ser Leu Ile Gly Ile Gly Gly Pro Leu Leu Trp

            100                 105                 110
Glu Thr Arg Leu Gly Leu Ala Phe Leu Arg Gly Leu Thr Tyr His Asp
        115                 120                 125
Gly Ala Leu Val Thr Met Glu Pro Gly Tyr Tyr Tyr Val Tyr Ser Lys
    130                 135                 140
Val Gln Leu Ser Gly Val Gly Cys Pro Gln Gly Leu Ala Asn Gly Leu
145                 150                 155                 160
Pro Ile Thr His Gly Leu Tyr Lys Arg Thr Ser Arg Tyr Pro Lys Glu
                165                 170                 175
Leu Glu Leu Leu Val Ser Arg Arg Ser Pro Cys Gly Arg Ala Asn Ser
            180                 185                 190
Ser Arg Val Trp Trp Asp Ser Ser Phe Leu Gly Gly Val Val His Leu
        195                 200                 205
Glu Ala Gly Glu Glu Val Val Val Arg Val Pro Gly Asn Arg Leu Val
    210                 215                 220
Arg Pro Arg Asp Gly Thr Arg Ser Tyr Phe Gly Ala Phe Met Val
225 230 235 239

<210> 3
<211> 239
<212> PRT
<213> Rat

<400> 3
Met Glu Ser Val Val Gln Pro Ser Val Phe Val Val Asp Gly Gln Thr
1               5                   10                  15
Asp Ile Pro Phe Arg Arg Leu Gly Gln Asn His Arg Arg Arg His Cys
            20                  25                  30
Gly Thr Val Gln Val Ser Leu Ala Leu Leu Leu Leu Leu Gly Ala Gly
        35                  40                  45
Leu Ala Thr Glu Gly Trp Phe Leu Leu Arg Leu His Gln Arg Leu Gly
    50                  55                  60
Asp Ile Val Ala His Leu Pro Asp Gly Gly Lys Gly Ser Trp Glu Lys
65                  70                  75                  80
Leu Ile Gln Asp Gln Arg Ser His Gln Pro Asn Pro Ala Ala His Leu
                85                  90                  95

Thr Gly Ala Asn Ala Ser Leu Ile Gly Ile Gly Gly Pro Leu Leu Trp
            100                 105                 110
Glu Thr Gln Leu Gly Leu Ala Phe Leu Arg Gly Leu Thr Tyr His Asp
        115                 120                 125
Gly Ala Leu Val Thr Thr Glu Ala Gly Tyr Tyr Tyr Val Tyr Ser Lys
    130                 135                 140
Val Gln Leu Ser Gly Val Gly Cys Pro Gln Gly Leu Ala Asn Gly Leu
145                 150                 155                 160
Pro Ile Thr His Gly Leu Tyr Lys Arg Thr Ser Arg Tyr Pro Lys Glu
                165                 170                 175
Leu Glu Leu Leu Val Ser Arg Arg Ser Pro Cys Gly Arg Ala Asn Ser
            180                 185                 190
Ser Arg Val Trp Trp Asp Ser Ser Phe Leu Gly Gly Val Val His Leu
        195                 200                 205
Glu Ala Gly Glu Glu Val Val Val Arg Val Pro Gly Asn Arg Leu Val
    210                 215                 220
Arg Pro Arg Asp Gly Thr Arg Ser Tyr Phe Gly Ala Phe Met Ile
225                 230                 235             239

<210> 4
<211> 720
<212> DNA
<213> Human

<400> 4
atggaggaga gtgtcgtacg gccctcagtg tttgtggtgg atggacagac cgacatccca    60
ttcacgaggc tgggacgaag ccaccggaga cagtcgtgca gtgtggcccg ggtgggtctg   120
ggtctcttgc tgttgctgat gggggctggg ctggccgtcc aaggctggtt cctcctgcag   180
ctgcactggc gtctaggaga gatggtcacc cgcctgcctg acggacctgc aggctcctgg   240
gagcagctga tacaagagcg aaggtctcac gaggtcaacc cagcagcgca tctcacaggg   300
gccaactcca gcttgaccgg cagcgggggg ccgctgttat gggagactca gctgggcctg   360
gccttcctga ggggcctcag ctaccacgat ggggcccttg tggtcaccaa agctggctac   420
tactacatct actccaaggt gcagctgggc ggtgtgggct gcccgctggg cctggccagc   480
accatcaccc acggcctcta caagcgcaca ccccgctacc ccgaggagct ggagctgttg   540
gtcagccagc agtcaccctg cggacgggcc accagcagct cccgggtctg gtgggacagc   600
agcttcctgg gtggtgtggt acacctggag gctggggaga aggtggtcgt ccgtgtgctg   660

gatgaacgcc tggttcgact gcgtgatggt acccggtctt acttcggggc tttcatggtg 720

<210> 5
<211> 1491
<212> DNA
<213> Human

<400> 5
cgagactcca tctcaaaaac aaaacaaata aacgaacaaa aaaacccaca acgtattatt 60
ttcttgttta cgaggtttct tgtctctctg gctccaccag aagaggagca gggacccttc 120
ttgctgttgt tcattgctgc atcccccaca ccgagagcag agcctggcat gggcagaaag 180
tcctcagtcg atatttggtg gccccaagcg aatgaagcat ccaagaaggg aaagctgggg 240
gctccccact gcacttgcca cctgagtcac attttcagaa gcctctggaa agtcgtgcac 300
agcccaggag tgttgagcaa tttcggtttc ctctgaggtt gaaggaccca ggcgtgtcag 360
ccctgctcca gacaccttgg gcatggagga gagtgtcgta cggccctcag tgtttgtggt 420
ggatggacag accgacatcc cattcacgag gctgggacga agccaccgga gacagtcgtg 480
cagtgtggcc cgggtgggtc tgggtctctt gctgttgctg atgggggctg ggctggccgt 540
ccaaggctgg ttcctcctgc agctgcactg gcgtctagga gagatggtca cccgcctgcc 600
tgacggacct gcaggctcct gggagcagct gatacaagag cgaaggtctc acgaggtcaa 660
cccagcagcg catctcacag gggccaactc cagcttgacc ggcagcgggg ggccgctgtt 720
atgggagact cagctgggcc tggccttcct gaggggcctc agctaccacg atggggccct 780
tgtggtcacc aaagctggct actactacat ctactccaag gtgcagctgg gcggtgtggg 840
ctgcccgctg ggcctggcca gcaccatcac ccacggcctc tacaagcgca caccccgcta 900
ccccgaggag ctggagctgt tggtcagcca gcagtcaccc tgcggacggg ccaccagcag 960
ctcccgggtc tggtgggaca gcagcttcct gggtggtgtg gtacacctgg aggctgggga 1020
gaaggtggtc gtccgtgtgc tggatgaacg cctggttcga ctgcgtgatg gtacccggtc 1080
ttacttcggg gctttcatgg tgtgaaggaa ggagcgtggt gcattggaca tgggtctgac 1140
acgtggagaa ctcagagggt gcctcagggg aaagaaaact cacgaagcag aggctgggcg 1200
tggtggctct cgcctgtaat cccagcactt tgggaggcca aggcaggcgg atcacctgag 1260
gtcaggagtt cgagaccagc ctggctaaca tggcaaaacc ccatctctac taaaaataca 1320
aaaattagcc ggacgtggtg gtgcctgcct gtaatccagc tactcaggag gctgaggcag 1380
gataattttg cttaaacccg ggaggcggag gttgcagtga gccgagatca caccactgca 1440
ctccaacctg ggaaacgcag tgagactgtg cctcaaaaaa aaaaaaaaaa a 1491

<210> 6
<211> 1353

<212> DNA
<213> Human

<400> 6
cccacgcgtc cgcccacgcg tccgctgagg ttgaaggacc caggcgtgtc agccctgctc 60
cagacacctt gggcatggag gagagtgtcg tacggccctc agtgtttgtg gtggatggac 120
agaccgacat cccattcacg aggctgggac gaagccaccg gagacagtcg tgcagtgtgg 180
cccgggtggg tctgggtctc ttgctgttgc tgatgggggc tgggctggcc gtccaaggct 240
ggttcctcct gcagctgcac tggcgtctag gagagatggt cacccgcctg cctgacggac 300
ctgcaggctc ctgggagcag ctgatacaag agcgaaggtc tcacgaggtc aacccagcag 360
cgcatctcac aggggccaac tccagcttga ccggcagcgg ggggccgctg ttatgggaga 420
ctcagctggg cctggccttc ctgaggggcc tcagctacca cgatggggcc cttgtggtca 480
ccaaagctgg ctactactac atctactcca aggtgcagct gggcggtgtg ggctgcccgc 540
tgggcctggc cagcaccatc acccacggcc tctacaagcg cacaccccgc taccccgagg 600
agctggagct gttggtcagc cagcagtcac cctgcggacg ggccaccagc agctcccggg 660
tctggtggga cagcagcttc ctgggtggtg tggtacacct ggaggctggg gagaaggtgg 720
tcgtccgtgt gctggatgaa cgcctggttc gactgcgtga tggtacccgg tcttacttcg 780
gggctttcat ggtgtgaagg aaggagcgtg gtgcattgga catgggtctg acacgtggag 840
aactcagagg gtgcctcagg ggaaagaaaa ctcacgaagc agaggctggg attacaggcg 900
tgagccactg ttcccagcag gaatttcttt tttatagtat tggataaagt ttggtgtttt 960
tacagaggag aagcaatggg tcttagctct ttctctatta tgttatcatc ctcccttttt 1020
tgtacaatat gttgtttacc tgaaaggaag gtttctattc gttggttgtg gacctggaca 1080
aagtccaagt ctgtggaact taaaaccttg aaggtctgtc ataggactct ggacaatctc 1140
acaccttagc tattcccagg gaaccccagg gggcaactga cattgctcca agatgttctc 1200
ctgatgtagc ttgagatata aaggaaaggc cctgcacagg tggctgtttc ttgtctgtta 1260
tgtcagagga acagtcctgt tcagaaaggg gctcttctga gcagaaatgg ctaataaact 1320
ttgtgctgat ctggaaaaaa aaaaaaaaaa aaa 1353

<210> 7
<211> 717
<212> DNA
<213> Murine

<400> 7
atggagagtg tggtacagcc ttcagtgttt gtggtggatg gacagacgga catcccattc 60
aggcggctgg aacagaacca ccggagacgg cgctgtggca ctgtccaggt cagcctggcc 120

ctggtgctgc tgctaggtgc tgggctggcc actcagggct ggtttctcct gagactgcat 180
caacgtcttg gagacatagt agctcatctg ccagatggag gcaaaggctc ctgggagaag 240
ctgatacaag atcaacgatc tcaccaggcc aacccagcag cacatcttac aggagccaac 300
gccagcttga taggtattgg tggacctctg ttatgggaga cacgacttgg cctggccttc 360
ttgaggggct tgacgtatca tgatggggcc ctggtgacca tggagcccgg ttactactat 420
gtgtactcca aagtgcagct gagcggcgtg ggctgccccc aggggctggc caatggcctc 480
cccatcaccc atggactata caagcgcaca tcccgctacc cgaaggagtt agaactgctg 540
gtcagtcggc ggtcaccctg tggccgggcc aacagctccc gagtctggtg ggacagcagc 600
ttcctgggcg gcgtggtaca tctggaggct ggggaagagg tggtggtccg cgtgcctgga 660
aaccgcctgg tcagaccacg tgacggcacc aggtcctatt tcggagcttt catggtc 717

<210> 8
<211> 795
<212> DNA
<213> Murine

<400> 8
tctgctctgg catggagagt gtggtacagc cttcagtgtt tgtggtggat ggacagacgg 60
acatcccatt caggcggctg gaacagaacc accggagacg gcgctgtggc actgtccagg 120
tcagcctggc cctggtgctg ctgctaggtg ctgggctggc cactcagggc tggtttctcc 180
tgagactgca tcaacgtctt ggagacatag tagctcatct gccagatgga ggcaaaggct 240
cctgggagaa gctgatacaa gatcaacgat ctcaccaggc caacccagca gcacatctta 300
caggagccaa cgccagcttg ataggtattg gtggacctct gttatgggag acacgacttg 360
gcctggcctt cttgaggggc ttgacgtatc atgatggggc cctggtgacc atggagcccg 420
gttactacta tgtgtactcc aaagtgcagc tgagcggcgt gggctgcccc caggggctgg 480
ccaatggcct ccccatcacc catggactat acaagcgcac atcccgctac ccgaaggagt 540
tagaactgct ggtcagtcgg cggtcaccct gtggccgggc caacagctcc cgagtctggt 600
gggacagcag cttcctgggc ggcgtggtac atctggaggc tggggaagag gtggtggtcc 660
gcgtgcctgg aaaccgcctg gtcagaccac gtgacggcac caggtcctat ttcggagctt 720
tcatggtctg aaggctgcgg tgacaatgta ttttgtggag ggacctctcc aggactcacc 780
tcaaacccag caata 795

<210> 9
<211> 9058
<212> DNA
<213> Murine

<400> 9
   tctagattgc attaacagag agaagaccct gagtatgggt ggagtcatct cagggctgag 60
   gtcctgggct gaatgaaaat gacagagtga gcagagcacc cgtgttcttt ggtttctgct 120
   acctcactgt ggatttgaca tgaccaactg cctcgtgcta cctcccctct ccccactttg 180
   acttctccac tgtgatagac acttctcact atgagccaag atttttcttc ctcccagtct 240
   tagttggggt ttctactgct gtgctaaaac accatgacca aaagcaactt ggagagaaga 300
   gggtttattt cagttacatt tccaggttat agttcatcac tgaataaaat caggacagga 360
   attctaattg tgcaggatcc tggaggcagt aggtgacgca gaggccatga ggatgctgct 420
   tgctagtttg ctccccatgg cttgcttgct cagcctgctt tcttatagaa cccaggatca 480
   tcagcccagg ggatggtgcc atccacagtg gactgggccc ttccgcatca atcactaatg 540
   gagaaaatgc cccacgggct taaatgcatc ctgatcttat ggaggcattt ttttttcaac 600
   tgaggctccc tcctctcaga tgatttttgc tcgtgtcaag ttgacataaa actatccagc 660
   accctgtacc ctttgtcatc ttgacacaca aacacaacac tagtaagcca caacctttcc 720
   tttcttgtca tctccaagat caaacattaa tatcaacgtc acaacataaa aacattttgt 780
   gggctagaga gatggcttgg aggctaagag cactggctgt ccttcaagaa aatgagcaat 840
   tcccagcacc cacacggcag ctcacaactg tctttaattc attttccagg ggatccaaat 900
   ccctcacaca tgcaagcaaa acactaatgt acagaaaata gaaataaata aatttttaaa 960
   cattttgcaa acttaaaaaa aaaaaaagtc ccaccacttt acaaattcaa acaggttaaa 1020
   atttagtgtc tttaaaactc caaagtttaa agttgaaaaa cctttaaaat ctaaaatatt 1080
   ttaaaagcta gcctctcagc tgtgggatcc tataacattg aaaacaagct taatacttcc 1140
   ttatttcaag agggaagaac cacggcacag tcaacaacct gaacaagcaa aaccaaatac 1200
   cagctgtgta aataattcga tttccagtgt ctgggattca aacatgatct tctgggctcc 1260
   tccaaaggtc ctgagtgcct tctctggtgc tgccctatgc agccctcaca tcttgtcttc 1320
   taggctgggg ctggctccac cccaaggctg ctgctgctgc tgctgctgct ggtgatcatc 1380
   ccaagacacc agcatcttca aaatactgtg gtcttctgct gccacggagg ctgcactttc 1440
   accaacagcc tctcctggtc ccacacagtg ccatgcctca gcttctctcc atggcctcct 1500
   tatccttcca aacccagcat catctgagga taaccttgtt caccttaaca gcacagcttc 1560
   tgtgtactgg ttctctcagg aaacactccc cagatttcac ctcagtgatg caggtctctt 1620
   ctaaatcacg gccagttctg cagaacaagc taagcagaac taagaatctc atttcaaata 1680
   tcaaacatcc ccaatgctct ccttgttgtt gtttttgttt taaaatagaa tccctttata 1740
   gttcttgctc tcttggaact cactatgtag aataggctgg ttttgatctc acaggagatc 1800
   catctgcctc tgcctcccaa aggtgtgtac taccctaccc aggaaatcgt ggtggtcttt 1860
   aagaaactct caaacttccc tctgtaattt cacaaggcag gcctccatca tctgccgtat 1920
   tctcagcatt ttgaacttcc aagctcccac agaacagccc actgagctct gcatggaaca 1980
   gtttctccag tccaaagttg agaggccgta caatcttctc aataccatag tcaggtcttt 2040
   cctattccta acccacacct gattccaatt tgtgtcttgg ctaggatttc tattgctgtc 2100
   ataaaacagc atgatcaaaa gcaacctcag cttacacttt caggtcacac cccatccact 2160
   gagggaggtc aggacaggaa cctggagaca ggagctgatg cagagactat ggaagaacac 2220
   tgcttactgg cttgtgtcct atgacttgtt cagcctgctt tcttatagaa ctcaggaatg 2280
   ccagtgcagg gaaggcccag tttacagtga gcacggcctc ccacaagcca atctgttggg 2340
   ggtgttttct ccattgaagt ttcctcttcc aaaatgaatg tagcttgtgt caaattgaca 2400
   ctggccagca cagaccatga gagattagag aacgcttcca ggcagatcct ttatggagta 2460
   tcagatactc cgggctgctg agtaatttgc acagttccag gaatggacct gagttgggca 2520
   acagcaaaga gatgaagaaa cgacaaacac agacatccag gaatcctggg gtcatgtgat 2580
   ctgggccttc tgcacacgag ttctcttagc acgttattat atagagtcga gtggagaagt 2640
   ggggttattg cctaccactg aaaaaggcaa cttattacat acagttaagc aaggaggcag 2700
   ggtttatatg cacaatggga tggggaaggc aggttgagtt agtctccata agaactgtcc 2760
   ctggaaggga gctatgttca tgcgtcagca ctcaaggggg aggatggtga acacattcta 2820
   cacacactgg cgacatgcac atccatgcgt ggaccagaag agaaggctat gtttccctct 2880
   gggtctgagg ctctggagtt gttgacaagc tcatgtcaac agcacacatc ccttcagcac 2940
   ctcacagacc tgggcttcct ctgaagggct ctgttgctga ggcaggtgga tgggtgggtc 3000
   ctgcaggggg acaggagtct cagcagtact ttcccctgga cccttggtta gagaacccat 3060
   gaatcagcag cgtggcagag cactggggtt ctgtggtcac agcatcgcca gccctgtgtc 3120
   aagacaaaca ctacagcatg ggggccattg acattgagaa ggggcgctgg tgattcttat 3180
   aactgaaccc ccaagtcttc agtatctggg agggtgtctg atgggggtaa gctccaagct 3240
   gaagatttgg ggacaaggtg gtatctgaaa atctacccca gcaccgtctc cagtggacac 3300
   acatatgaac acatctgagg ttcatgtgtg catgtgctaa tagatgatag acactgtagt 3360
   gtgtgtgtgt gtgtgtgtcc atatatggac ctgcacacac atcaggaaat gcctgtgtgc 3420
   ttgttccagc acttgcagac acatatcaga acatctacac ctgtgtgcac acatccacga 3480
   accgtgcatc tgtacaagca cctgcaccca tatgcacaga ctaaagcaaa cacagaggcc 3540
   tctgcttgtc agcgcgtgca aatgcaggtg cggtgcaaga tgcttgcatg gaacccacag 3600
   aagttctttt gagggaaaca aaagccacca agtacatcgg agcaggggct gccccatcca 3660
   tcccacctga gtcacatttt ctggaaagtg tgagctatgg tggcctcagt gagagtgatc 3720
   gaccgggggc tggccttctg gggcacaggg aagaagagga ggtacgtgag gaaaggggag 3780
   gcaccagact tcagctttaa agtgagtcct aggggtgaca ggaacctttt gcagtttgca 3840
   cagcccgagc gtgttgggca attgtggttt cctccggaga ggaggaactc aggcttgcca 3900
   acccttcccc ctgggcttcg gagcctcagc tgctctggca tggagagtgt ggtacagcct 3960
   tcagtgtttg tggtggatgg acagacggac atcccattca ggcggctgga acagaaccac 4020
   cggagacggc gctgtggcac tgtccaggtc agcctggccc tggtgctgct gctaggtgct 4080
   gggctggcca ctcagggctg gtttctcctg agactgcatc aacgtcttgg agacatagta 4140
   gctcatctgc cagtaagtgg ggcttgggga accagcgagt cttctccagt acctgtccct 4200
   ttatggttgt gtattgtgtg ctagaaccca gggcttcggt tagcctgtct ctctgaccct 4260
   cagtcttctc atctttacat gaggatctga tacacgtgca gtgcccggca gtttccttgg 4320
   gattcaccta ctcggttgtt gtttccagcc caaacctttg tccaccactg gcttcatggt 4380
   caccctgctt cactcgcagc tctctcatgt gcttgctcta ctgtttcctt tcagacaagc 4440
   catgggtgtg tcacagtatg ctcccgagtc tttgcctata ttttcttttt aagttttttt 4500
   ttttaattac atttacttat ttagtgtgta gacatgaaaa agtatgcatg tgccgcagca 4560
   tgtatgtgga acacctctat ggaatcactt ctctgtgcct tcctttgtgg agggtcctag 4620
   ggactgaact caggtagcca ggcttggcag caagcacttt taccgcctga gccctctcac 4680
   cggccctcaa ctatccaaaa acctcgaaaa cagttccgaa acaattctag tctcaagcat 4740
   ttcagacact cgacttgact agttaattcc tcatcccagc acttgggcag gggaggcaga 4800
   agactgaggg agttcaaggt catcctaggc tatttagcaa gtctgaggcc agactgggct 4860
   acatgagagc ctatcttgga aatacaacag atctaattag gtgtgtgccc ctccccctgc 4920
   atctgtgtgt atgtatgtga tatgtgcatg tgtggctctg catacatgtc catgggtggt 4980
   gtctgtggcc atggccagac agtctcatca ccacatacca gtgagactcg gagcggcatg 5040
   tccttaccaa acatccctgt ctgcatttca ggatggaggc aaaggctcct gggagaagct 5100
   gatacaaggt gagtttgggc cccagtccct ttgaagcagg tggaatggtt tccgttcagc 5160
   acctccccac accaactctg ggtcatcacc cagtgtcaca ttgtaggcct cgcaggtgcc 5220
   cctcccccag ggccagagaa gcagaaatct gggtccaaaa gggacagagc ctgacacagg 5280
   tagcctcaat tgatgtgtga gaaacttcgg gtacacacac actgagacat gcgtgcacac 5340
   acacacatac acacacacac acacacacac aatcctcttt ttgtctctaa cctttgactc 5400
   tcttcctcag atcaacgatc tcaccaggcc aacccagcag cacatcttac aggtgagagg 5460
   gaccccagat cttcacacgg aatggggttt agcatgtcct gggagactca gatctttata 5520
   agttagagca ctcgtttatg cattcattta gtgtgtcttt aaattttttt tgacatttac 5580
   ttctttttat tcatttatta ttgcacatgt gcatgccata gtgtagaggt cagaaaactc 5640
   tcaggggaac gattctttcc ttccaccttt tggcctccgt gtactgatct cagccatcag 5700
   gcttggctct aagtaccttt actctctgcg ccatcttgcc ggtcactttt ttttttctca 5760
   gtgtttacta aaaaacagcc cagagaagag ccacagggag ccatgtaaag tcaactcttc 5820
   accctgctag tcatggggac acatcccaag gtccttggag gcctcagaag atgttgggac 5880
   tcagacctac cacagtttat atttgtttgt ttgtttggtt ggttggttgg ttggttggtt 5940
   ggttggttga ttggttggtt ggttttggtt ttggagacag ggtctctctg tgtagcttgg 6000
   gctgcctggg tagacctgcc tctgtcctga gtactggaat taacaggaat taacgacgtg 6060
   ggccactaca cccagctgta tatactggtt tttcttttgg catagacagt ttaatttata 6120
   atttaacaag taagaaacta acaagaatac acataaagta tgctgtaatt aaaaactatt 6180
   tttgaaacat gatgtgggag atagggaggt ggctccatgt gcaaattgct agccactttt 6240
   gactcaacat ctgagccaaa agccaaacac acactcccgt gacagttaca gaggcagatt 6300
   cagacagaca cacactcctg tgacagtcat ggagacagag acaggcagag aggtccctag 6360
   gacctgctgt ccagcagcct tgacaaacag gggacttcca gggtcaataa gagactctgc 6420
   ctcaaaacta aggtggacag aggttgaaac tgaggaagtt gcctggtttt gacttctggg 6480
   cacacacaca cacacacaca cacatatata taacacatac atcacacaca tatacacaaa 6540
   catacagtca cacacacttt ttgagataga gtctcatgta gcctaaaccg gccttgaact 6600
   cttgatcctc cagactcgac gtcccaagat ctggatgtca ccactacacc tgatttattt 6660
   ggtactgggg ctggaatgta cagccttcgg catgctgggc gagtgttcca cctcctaaat 6720
   cctaacccca tttaaaaaaa atttttttga catttcacta ggaagcttca ataccttgtg 6780
   cctcagtttc ttctttggta ttcaagtgac tttgctcctc agtgcttcag tgtctaccct 6840
   tgcagaagga gatgggaatg aacggagtta agacacaacc accaaattac tttgcttgct 6900
   ttcctgtatc tcagtttctt ccccctgcaa gataggaatg ggctgagtca acccagacaa 6960
   gcagcctact atggtggtga aaccagaggg aactagcata aggtcacaca gaaagggtgg 7020
   gcacaatgct aaccgtgctg acgggttatc tttctctctc ctttcctccc aggagccaac 7080
   gccagcttga taggtattgg tggacctctg ttatgggaga cacgacttgg cctggccttc 7140
   ttgaggggct tgacgtatca tgatggggcc ctggtgacca tggagcccgg ttactactat 7200
   gtgtactcca aagtgcagct gagcggcgtg ggctgccccc aggggctggc caatggcctc 7260
   cccatcaccc atggactata caagcgcaca tcccgctacc cgaaggagtt agaactgctg 7320
   gtcagtcggc ggtcaccctg tggccgggcc aacagctccc gagtctggtg ggacagcagc 7380
   ttcctgggcg gcgtggtaca tctggaggct ggggaagagg tggtggtccg cgtgcctgga 7440
   aaccgcctgg tcagaccacg tgacggcacc aggtcctatt tcggagcttt catggtctga 7500
   aggctgcggt gacaatgtat tttgtggagg gacctctcca ggactcacct caaacccagc 7560
   aatagggttt gaagtcctcc ctttaaggag ccctgaactc tgcagtgctc ggggcggtgt 7620
   tcactgctga cctgctttgg gcaatcttca aatcagagac ctggagactt ggggcgtgga 7680
   gcccaggagc gaggggtcag ctcatttgcc tgatattcag gaagaaagaa tcaagctggg 7740
   gtatttatgc ttctgatgca aacactgaga tttcggcttt ctgggttttg agctggaggc 7800
   aagaaacctt cccagagtgt catcaggacc atgttggcag gacttggggc tccagacttg 7860
   ccaccacact ctggcctctc ccatccatcc gctgcattgg tttccagcca ccaaaacagc 7920
   actggccccc tggctgcaac tggccaggta cgagcttctg agcacctaca ttcctcaggg 7980
   acatcttgat gagatctcag tactcagtcc aatgcgcagc agcgacagac atgccaggaa 8040
   tggttggtca gaagggaagg gaggaaaggg aggaaagaac ggaatgcaca agagaagggg 8100
   ggaaaacaag accaaaacaa aacagcaaca acaaagcggc agggaagaag ttgacaccct 8160
   tggggatact ttagtcaaca cacttagaac agattgtgcc aggcctgttg gattcctgga 8220
   gttgatggga tcgtgggaag gcacaatggg gagcaagtgg gcttgggtta tggctcagtg 8280
   ggtaaagtgc aattatgggg atctgagttt gaatccctgg tacccatata aagacacaga 8340
   tgcggtgatg ggcacttgtg acaatgagat catcaatagg gaatggagac aggagggacc 8400
   tctggggttc actggccagg cagtctagct gaatcaaaga gctccaagtt cagtcgatag 8460
   ctcctgaaga tgacaactga ggctattctc caaaccccac acgcagggac acatgcgtaa 8520
   taaataaaat tttaaaaata ttaaataata gtgtttgaga ggcactttat atgttctaat 8580
   catttgtgct tttgtttatt tgttcaggtt tgtttgtttg tttgttggaa gacaggggtc 8640
   tcaagtagcc caggctagct ttgaactata tatttcatat attttgaggc agtcttatgc 8700
   cttttatcta ggttttcttg gcatctaaag ctatagctgt gtgttactcc aatggaacat 8760
   ctgggcagtt acccagcacc ttcaaatgca gagcccacac ctgatagggt cgggctcgcc 8820
   ctgctcaggg gtcgctgcta gtccaagcca gtccaagcgg acttcccgcg tcctgtcctt 8880
   gcaaccctgg tgggaggtgg aaaagccccc aaatacccag tctcaccctc catcggagtt 8940
   tcctttatgc ttatcacggc ctgtttccgt gtctttgatg agaccaaggt gtggggacag 9000
   tatatttata aaaagccacc agcagtttcc gctgtaagga aaaaaaaatc actctaga 9058

<210> 10
<211> 717
<212> DNA
<213> Rat

<400> 10
atggagagtg tggtacagcc ttcagtgttt gtggtggatg gacagacaga catcccattc 60
aggcggctgg gacagaacca caggagacgg cactgcggca ctgtccaggt cagcctggcc 120
ctgctgctgc tgctgggtgc tgggctggcc actgagggct ggtttctcct gagactgcat 180
cagcgtcttg gggacatagt agctcatctg ccagatggag gcaaaggctc ctgggagaag 240
ctgatacaag atcaacgatc tcaccagccc aacccagcag cacatctcac aggagctaac 300
gccagcttga taggcattgg tggacctctg ttatgggaga cacaacttgg cctggccttc 360
ctgaggggcc tgacgtatca tgatggggcc ctggtgacca ccgaggctgg ctactactac 420
gtgtactcca aagtgcagtt gagtggtgtg ggctgccccc aggggctggc caatggcctc 480
cccatcaccc acgggctgta caagcgcaca tcccgatacc ccaaggagtt agaactgctg 540
gtcagccggc ggtcaccttg tggccgggcc aacagctccc gagtctggtg ggacagtagt 600
ttcctcggcg gagtggtaca tctggaggcc ggagaagagg tggtggtccg cgtgcctgga 660
aaccgcctgg tcagaccacg tgatggcacg aggtcctatt tcggagcttt catgatc 717

<210> 11
<211> 24
<212> DNA
<213> Artificial Sequence

<220>
<223> synthetic oligonucleotide probe

<400> 11
aggtcaaccc agcagcgcat ctca     24

<210> 12
<211> 28
<212> DNA
<213> Artificial Sequence

<220>
<223> synthetic oligonucleotide primer

<400> 12
aggtcaaccc agcagcgcat ctcacagg     28

<210> 13
<211> 30
<212> DNA
<213> Artificial Sequence

<220>
<223> synthetic oligonucleotide primer

<400> 13
caaattaaac cgggtaccat cacgcagtcg     30

<210> 14
<211> 25
<212> DNA
<213> Artificial Sequence

<220>
<223> synthetic oligonucleotide primer

<400> 14
tctgctctgg catggagagt gtggt     25

<210> 15
<211> 24
<212> DNA
<213> Artificial Sequence

<220>
<223> synthetic oligonucleotide primer

<400> 15
ctattgctgg gtttgaggtg agtc     24

<210> 16
<211> 22
<212> DNA
<213> Artificial Sequence

<220>
<223> primer AP1

<400> 16
gtaatacgac tcactatagg gc     22

<210> 17
<211> 19
<212> DNA
<213> Artificial Sequence

<220>
<223> primer AP2

<400> 17
actatagggc acgcgtggt     19

<210> 18
<211> 48

<212> DNA
<213> Artificial Sequence

<220>
<223> adaptor

<400> 18
gtaatacgac tcactatagg gcacgcgtgg tcgacggccc gggctggt 48

<210> 19
<211> 27
<212> DNA
<213> Artificial Sequence

<220>
<223> synthetic oligonucleotide GSP1

<400> 19
cagcccagca cctagcagca gcaccag 27

<210> 20
<211> 27
<212> DNA
<213> Artificial Sequence

<220>
<223> synthetic oligonucleotide GSP2

<400> 20
gccgcctgaa tgggatgtcc gtctgtc 27

<210> 21
<211> 32
<212> DNA
<213> Artificial Sequence

<220>
<223> synthetic oligonucleotide primer

<400> 21
acgaattcca agagcgaagg tctcacgagg tc    32

<210> 22
<211> 35
<212> DNA
<213> Artificial Sequence

<220>
<223> synthetic oligonucleotide primer

<400> 22
agtctagact ccttccttca caccatgaaa gcccc    35

<210> 23
<211> 24
<212> DNA
<213> Artificial Sequence

<220>
<223> synthetic oligonucleotide primer

<400> 23
cctgaccctg ggcttctgag cctc    24

<210> 24
<211> 27
<212> DNA
<213> Artificial Sequence

<220>
<223> synthetic oligonucleotide primer

<400> 24
tccacaaaat ccattgtcgt catagcc          27

<210> 25
<211> 242
<212> PRT
<213> Human, Murine, Rat

<220>
<221> VARIANT
<222> 3
<223> Xaa at the 3rd position may be preferably Glu or may be deleted.

<220>
<221> VARIANT
<222> 7
<223> Xaa at the 7th position may be preferably a hydrophilic amino acid, e.g. suitably
Arg or Gln.

<220>
<221> VARIANT
<222> 22
<223> Xaa at the 22nd position may be preferably a hydrophilic amino acid, e.g.
suitably Thr or Arg.

<220>
<221> VARIANT
<222> 25
<223> Xaa at the 25th position may be preferably a hydrophilic amino acid, e.g.
suitably Gly or Glu.

<220>
<221> VARIANT
<222> 26
<223> Xaa at the 26th position may be preferably a hydrophilic amino acid, e.g.
suitably Arg or Gln.

<220>
<221> VARIANT
<222> 27
<223> Xaa at the 27th position may be preferably a hydrophilic amino acid, e.g.
suitably Ser or Asn.

<220>
<221> VARIANT
<222> 31
<223> Xaa at the 31st position may be preferably a hydrophilic amino acid, e.g.
suitably Gln or Arg.

<220>
<221> VARIANT
<222> 32
<223> Xaa at the 32nd position may be preferably a hydrophilic amino acid, e.g.
suitably Ser or Arg.

<220>
<221> VARIANT
<222> 34
<223> Xaa at the 34th position may be preferably a hydrophilic amino acid, e.g.
suitably Ser or Gly.

<220>
<221> VARIANT
<222> 35
<223> Xaa at the 35th position may be e.g. suitably Val or Thr.

<220>
<221> VARIANT
<222> 36
<223> Xaa at the 36th position may be preferably a hydrophobic amino acid, e.g.
suitably Ala or Val.

<220>
<221> VARIANT
<222> 37
<223> Xaa at the 37th position may be preferably a hydrophilic amino acid, e.g.
suitably Arg or Gln.

<220>
<221> VARIANT
<222> 39
<223> Xaa at the 39th position may be preferably a hydrophilic amino acid, e.g.
suitably Gly or Ser.

<220>
<221> VARIANT
<222> 41
<223> Xaa at the 41st position may be e.g. suitably Gly or Ala.

<220>
<221> VARIANT
<222> 43
<223> Xaa at the 43rd position may be preferably a hydrophobic amino acid, e.g.
suitably Leu or Val.

<220>
<221> VARIANT
<222> 47
<223> Xaa at the 47th position may be preferably Met or may be deleted.

<220>
<221> VARIANT
<222> 53
<223> Xaa at the 53rd position may be e.g. suitably Val or Thr.

<220>
<221> VARIANT
<222> 60

<223> Xaa at the 60th position may be preferably a hydrophilic amino acid, e.g.
suitably Gln or Arg.

<220>
<221> VARIANT
<222> 63
<223> Xaa at the 63rd position may be e.g. suitably Trp or Gln.

<220>
<221> VARIANT
<222> 67
<223> Xaa at the 67th position may be preferably an acidic amino acid, e.g. suitably
Glu or Asp.

<220>
<221> VARIANT
<222> 68
<223> Xaa at the 68th position may be preferably a hydrophobic amino acid, e.g.
suitably Met or Ile.

<220>
<221> VARIANT
<222> 70
<223> Xaa at the 70th position may be e.g. suitably Thr or Ala.

<220>
<221> VARIANT
<222> 71
<223> Xaa at the 71st position may be preferably a basic amino acid, e.g. suitably
Arg or His.

<220>
<221> VARIANT
<222> 76
<223> Xaa at the 76th position may be e.g. suitably Pro or Gly.

<220>
<221> VARIANT
<222> 77
<223> Xaa at the 77th position may be e.g. suitably Ala or Lys.

<220>
<221> VARIANT
<222> 82
<223> Xaa at the 82nd position may be preferably a hydrophilic amino acid, e.g.
suitably Gln or Lys.

<220>
<221> VARIANT
<222> 86
<223> Xaa at the 86th position may be preferably an acidic amino acid, e.g. suitably
Glu or Asp.

<220>
<221> VARIANT
<222> 87
<223> Xaa at the 87th position may be preferably a hydrophilic amino acid, e.g.
suitably Arg or Gln.

<220>
<221> VARIANT
<222> 91
<223> Xaa at the 91st position may be preferably a hydrophilic amino acid, e.g.
suitably Glu or Gln.

<220>
<221> VARIANT
<222> 92
<223> Xaa at the 92nd position may be preferably a hydrophobic amino acid, e.g.
suitably Val or Ala.

<220>

<221> VARIANT
<222> 103
<223> Xaa at the 103rd position may be e.g. suitably Ser or Ala.

<220>
<221> VARIANT
<222> 106
<223> Xaa at the 106th position may be e.g. suitably Thr or Ile.

<220>
<221> VARIANT
<222> 108
<223> Xaa at the 108th position may be e.g. suitably Ser or Ile.

<220>
<221> VARIANT
<222> 117
<223> Xaa at the 117th position may be preferably a hydrophilic amino acid, e.g.
suitably Gln or Arg.

<220>
<221> VARIANT
<222> 127
<223> Xaa at the 127th position may be preferably a hydrophilic amino acid, e.g.
suitably Ser or Thr.

<220>
<221> VARIANT
<222> 135
<223> Xaa at the 135th position may be e.g. suitably Val or Thr.

<220>
<221> VARIANT
<222> 136
<223> Xaa at the 136th position may be e.g. suitably Thr or Met.

<220>
<221> VARIANT
<222> 137
<223> Xaa at the 137th position may be preferably a hydrophilic amino acid, e.g.
suitably Lys or Glu.

<220>
<221> VARIANT
<222> 138
<223> Xaa at the 138th position may be preferably a hydrophobic amino acid, e.g.
suitably Ala or Pro.

<220>
<221> VARIANT
<222> 143
<223> Xaa at the 143rd position may be preferably a hydrophobic amino acid, e.g.
suitably Ile or Val.

<220>
<221> VARIANT
<222> 150
<223> Xaa at the 150th position may be preferably a hydrophilic amino acid, e.g.
suitably Gly or Ser.

<220>
<221> VARIANT
<222> 156
<223> Xaa at the 156th position may be e.g. suitably Leu or Gln.

<220>
<221> VARIANT
<222> 160
<223> Xaa at the 160th position may be preferably a hydrophilic amino acid, e.g.
suitably Ser or Asn.

<220>

<221> VARIANT
<222> 161
<223> Xaa at the 161st position may be preferably a hydrophilic amino acid, e.g.
suitably Thr or Gly.

<220>
<221> VARIANT
<222> 162
<223> Xaa at the 162nd position may be preferably Leu or may be deleted.

<220>
<221> VARIANT
<222> 163
<223> Xaa at the 163rd position may be preferably Pro or may be deleted.

<220>
<221> VARIANT
<222> 173
<223> Xaa at the 173rd position may be e.g. suitably Pro or Ser.

<220>
<221> VARIANT
<222> 178
<223> Xaa at the 178th position may be preferably a hydrophilic amino acid, e.g.
suitably Glu or Lys.

<220>
<221> VARIANT
<222> 185
<223> Xaa at the 185th position may be preferably a hydrophilic amino acid, e.g.
suitably Gln or Arg.

<220>
<221> VARIANT
<222> 186
<223> Xaa at the 186th position may be preferably a hydrophilic amino acid, e.g.

suitably Gln or Arg.

<220>
<221> VARIANT
<222> 193
<223> Xaa at the 193th position may be preferably Thr or may be deleted.

<220>
<221> VARIANT
<222> 194
<223> Xaa at the 194th position may be preferably a hydrophilic amino acid, e.g.
suitably Ser or Asn.

<220>
<221> VARIANT
<222> 216
<223> Xaa at the 216th position may be preferably a hydrophilic amino acid, e.g.
suitably Lys or Glu.

<220>
<221> VARIANT
<222> 222
<223> Xaa at the 222th position may be preferably a hydrophobic amino acid, e.g.
suitably Leu or Pro.

<220>
<221> VARIANT
<222> 223
<223> Xaa at the 223th position may be preferably a hydrophilic amino acid, e.g.
suitably Asp or Gly.

<220>
<221> VARIANT
<222> 224
<223> Xaa at the 224th position may be preferably a hydrophilic amino acid, e.g.
suitably Glu or Asn.

<220>
<221> VARIANT
<222> 229
<223> Xaa at the 229th position may be preferably a hydrophobic amino acid, e.g.
suitably Leu or Pro.

<400> 25
Met Glu Xaa Ser Val Val Xaa Pro Ser Val Phe Val Val Asp Gly Gln
1               5                   10                  15
Thr Asp Ile Pro Phe Xaa Arg Leu Xaa Xaa Xaa His Arg Arg Xaa Xaa
            20                  25                  30
Cys Xaa Xaa Xaa Xaa Val Xaa Leu Xaa Leu Xaa Leu Leu Leu Xaa Gly
        35                  40                  45
Ala Gly Leu Ala Xaa Gln Gly Trp Phe Leu Leu Xaa Leu His Xaa Arg
    50                  55                  60
Leu Gly Xaa Xaa Val Xaa Xaa Leu Pro Asp Gly Xaa Xaa Gly Ser Trp
65                  70                  75                  80
Glu Xaa Leu Ile Gln Xaa Xaa Arg Ser His Xaa Xaa Asn Pro Ala Ala
                85                  90                  95
His Leu Thr Gly Ala Asn Xaa Ser Leu Xaa Gly Xaa Gly Gly Pro Leu
            100                 105                 110
Leu Trp Glu Thr Xaa Leu Gly Leu Ala Phe Leu Arg Gly Leu Xaa Tyr
        115                 120                 125
His Asp Gly Ala Leu Val Xaa Xaa Xaa Xaa Gly Tyr Tyr Tyr Xaa Tyr
    130                 135                 140
Ser Lys Val Gln Leu Xaa Gly Val Gly Cys Pro Xaa Gly Leu Ala Xaa
145                 150                 155                 160
Xaa Xaa Xaa Ile Thr His Gly Leu Tyr Lys Arg Thr Xaa Arg Tyr Pro
                165                 170                 175
Glu Xaa Leu Glu Leu Leu Val Ser Xaa Xaa Ser Pro Cys Gly Arg Ala
            180                 185                 190
Xaa Xaa Ser Ser Arg Val Trp Trp Asp Ser Ser Phe Leu Gly Gly Val
        195                 200                 205
Val His Leu Glu Ala Gly Glu Xaa Val Val Val Arg Val Xaa Xaa Xaa
    210                 215                 220

Arg Leu Val Arg Xaa Arg Asp Gly Thr Arg Ser Tyr Phe Gly Ala Phe
225                 230                 235                 240
Met Val
    242

<210> 26
<211> 34
<212> DNA
<213> Artificial Sequence

<220>
<223> synthetic oligonucleotide primer

<400> 26
gaattcgata caagagcgaa ggtctcacga ggtc        34

<210> 27
<211> 35
<212> DNA
<213> Artificial Sequence

<220>
<223> synthetic oligonucleotide primer

<400> 27
aaatctagat ccttccttca caccatgaaa gcccc        35

<210> 28
<211> 36
<212> DNA
<213> Artificial Sequence

<220>
<223> primer 1

<400> 28

aggaattcat ggaggagagt gtcgtacggc cctcag        36

<210> 29
<211> 35
<212> DNA
<213> Artificial Sequence

<220>
<223> primer 2

<400> 29
agctcgagct ccttccttca caccatgaaa gcccc        35

<210> 30
<211> 612
<212> DNA
<213> Human

<400> 30
atggaggaga gtgtcgtacg gccctcagtg tttgtggtgg atggacagac cgacatccca    60
ttcacgaggc tgggacgaag ccaccggaga cagtcgtgca gtgtggcccg ggacggacct   120
gcaggctcct gggagcagct gatacaagag cgaaggtctc acgaggtcaa cccagcagcg   180
catctcacag gggccaactc cagcttgacc ggcagcgggg ggccgttgtt atgggagact   240
cagctgggcc tggccttcct gaggggcctc agctaccatg atggggccct tgtggtcacc   300
aaagctggct actactacat ctactccaag gtgcagctgg gcggtgtggg ctgcccgctg   360
ggcctggcca gcaccatcac ccacggcctc tacaagcgca caccccgcta ccccgaggag   420
ctggagctgt tggtcagcca gcagtcaccc tgcggacggg ccaccagcag ctcccgggtc   480
tggtgggaca gcagcttcct gggtggtgtg gtacacctgg aggctgggga gaaggtggtc   540
gtccgtgtgc tggatgaacg cctggttcga ctgcgtgatg gtacccggtc ttacttcggg   600
gctttcatgg tg                                                       612

<210> 31
<211> 204
<212> PRT
<213> Human

<400> 31
Met Glu Glu Ser Val Val Arg Pro Ser Val Phe Val Val Asp Gly Gln
1               5                   10                  15
Thr Asp Ile Pro Phe Thr Arg Leu Gly Arg Ser His Arg Arg Gln Ser
            20                  25                  30
Cys Ser Val Ala Arg Asp Gly Pro Ala Gly Ser Trp Glu Gln Leu Ile
        35                  40                  45
Gln Glu Arg Arg Ser His Glu Val Asn Pro Ala Ala His Leu Thr Gly
    50                  55                  60
Ala Asn Ser Ser Leu Thr Gly Ser Gly Gly Pro Leu Leu Trp Glu Thr
65                  70                  75                  80
Gln Leu Gly Leu Ala Phe Leu Arg Gly Leu Ser Tyr His Asp Gly Ala
                85                  90                  95
Leu Val Val Thr Lys Ala Gly Tyr Tyr Tyr Ile Tyr Ser Lys Val Gln
            100                 105                 110
Leu Gly Gly Val Gly Cys Pro Leu Gly Leu Ala Ser Thr Ile Thr His
        115                 120                 125
Gly Leu Tyr Lys Arg Thr Pro Arg Tyr Pro Glu Glu Leu Glu Leu Leu
    130                 135                 140
Val Ser Gln Gln Ser Pro Cys Gly Arg Ala Thr Ser Ser Ser Arg Val
145                 150                 155                 160
Trp Trp Asp Ser Ser Phe Leu Gly Gly Val Val His Leu Glu Ala Gly
                165                 170                 175
Glu Lys Val Val Val Arg Val Leu Asp Glu Arg Leu Val Arg Leu Arg
            180                 185                 190
Asp Gly Thr Arg Ser Tyr Phe Gly Ala Phe Met Val
        195                 200             204

<210> 32
<211> 204
<212> PRT
<213> Human

<220>
<221> VARIANT
<222> 3

<223> Xaa at the 3rd position may be preferably Glu or may be deleted.

<220>
<221> VARIANT
<222> 7
<223> Xaa at the 7th position may be preferably a hydrophilic amino acid, e.g. suitably
Arg or Gln.

<220>
<221> VARIANT
<222> 22
<223> Xaa at the 22nd position may be preferably a hydrophilic amino acid, e.g.
suitably Thr or Arg.

<220>
<221> VARIANT
<222> 25
<223> Xaa at the 25th position may be preferably a hydrophilic amino acid, e.g.
suitably Gly or Glu.

<220>
<221> VARIANT
<222> 26
<223> Xaa at the 26th position may be preferably a hydrophilic amino acid, e.g.
suitably Arg or Gln.

<220>
<221> VARIANT
<222> 27
<223> Xaa at the 27th position may be preferably a hydrophilic amino acid, e.g.
suitably Ser or Asn.

<220>
<221> VARIANT
<222> 31
<223> Xaa at the 31st position may be preferably a hydrophilic amino acid, e.g.

suitably Gln or Arg.

<220>
<221> VARIANT
<222> 32
<223> Xaa at the 32nd position may be preferably a hydrophilic amino acid, e.g.
suitably Ser or Arg.

<220>
<221> VARIANT
<222> 34
<223> Xaa at the 34th position may be preferably a hydrophilic amino acid, e.g.
suitably Ser or Gly.

<220>
<221> VARIANT
<222> 35
<223> Xaa at the 35th position may be e.g. suitably Val or Thr.

<220>
<221> VARIANT
<222> 36
<223> Xaa at the 36th position may be preferably a hydrophobic amino acid, e.g.
suitably Ala or Val.

<220>
<221> VARIANT
<222> 37
<223> Xaa at the 37th position may be preferably a hydrophilic amino acid, e.g.
suitably Arg or Gln.

<220>
<221> VARIANT
<222> 40
<223> Xaa at the 40th position may be e.g. suitably Pro or Gly.

<220>
<221> VARIANT
<222> 41
<223> Xaa at the 41st position may be e.g. suitably Ala or Lys.

<220>
<221> VARIANT
<222> 46
<223> Xaa at the 46th position may be preferably a hydrophilic amino acid, e.g.
suitably Gln or Lys.

<220>
<221> VARIANT
<222> 50
<223> Xaa at the 50th position may be preferably an acidic amino acid, e.g. suitably
Glu or Asp.

<220>
<221> VARIANT
<222> 51
<223> Xaa at the 51st position may be preferably a hydrophilic amino acid, e.g.
suitably Arg or Gln.

<220>
<221> VARIANT
<222> 55
<223> Xaa at the 55th position may be preferably a hydrophilic amino acid, e.g.
suitably Glu or Gln.

<220>
<221> VARIANT
<222> 56
<223> Xaa at the 56th position may be preferably a hydrophobic amino acid, e.g.
suitably Val or Ala.

<220>

<221> VARIANT
<222> 67
<223> Xaa at the 67th position may be e.g. suitably Ser or Ala.

<220>
<221> VARIANT
<222> 70
<223> Xaa at the 70th position may be e.g. suitably Thr or Ile.

<220>
<221> VARIANT
<222> 72
<223> Xaa at the 72nd position may be e.g. suitably Ser or Ile.

<220>
<221> VARIANT
<222> 81
<223> Xaa at the 81st position may be preferably a hydrophilic amino acid, e.g.
suitably Gln or Arg.

<220>
<221> VARIANT
<222> 91
<223> Xaa at the 91st position may be preferably a hydrophilic amino acid, e.g.
suitably Ser or Thr.

<220>
<221> VARIANT
<222> 99
<223> Xaa at the 99th position may be e.g. suitably Val or Thr.

<220>
<221> VARIANT
<222> 100
<223> Xaa at the 100th position may be e.g. suitably Thr or Met.

<220>
<221> VARIANT
<222> 101
<223> Xaa at the 101st position may be preferably a hydrophilic amino acid, e.g.
suitably Lys or Glu.

<220>
<221> VARIANT
<222> 102
<223> Xaa at the 102nd position may be preferably a hydrophobic amino acid, e.g.
suitably Ala or Pro.

<220>
<221> VARIANT
<222> 107
<223> Xaa at the 107th position may be preferably a hydrophobic amino acid, e.g.
suitably Ile or Val.

<220>
<221> VARIANT
<222> 114
<223> Xaa at the 114th position may be preferably a hydrophilic amino acid, e.g.
suitably Gly or Ser.

<220>
<221> VARIANT
<222> 120
<223> Xaa at the 120th position may be e.g. suitably Leu or Gln.

<220>
<221> VARIANT
<222> 124
<223> Xaa at the 124th position may be preferably a hydrophilic amino acid, e.g.
suitably Ser or Asn.

<220>

<221> VARIANT
<222> 125
<223> Xaa at the 125th position may be preferably a hydrophilic amino acid, e.g.
suitably Thr or Gly.

<220>
<221> VARIANT
<222> 135
<223> Xaa at the 135th position may be preferably Pro or may be deleted.

<220>
<221> VARIANT
<222> 140
<223> Xaa at the 140th position may be preferably a hydrophilic amino acid, e.g.
suitably Glu or Lys.

<220>
<221> VARIANT
<222> 147
<223> Xaa at the 147th position may be preferably a hydrophilic amino acid, e.g.
suitably Gln or Arg.

<220>
<221> VARIANT
<222> 148
<223> Xaa at the 148th position may be preferably a hydrophilic amino acid, e.g.
suitably Gln or Arg.

<220>
<221> VARIANT
<222> 155
<223> Xaa at the 155th position may be preferably Thr or may be deleted.

<220>
<221> VARIANT
<222> 156

<223> Xaa at the 156th position may be preferably a hydrophilic amino acid, e.g.
suitably Ser or Asn.

<220>
<221> VARIANT
<222> 178
<223> Xaa at the 178th position may be preferably a hydrophilic amino acid, e.g.
suitably Lys or Glu.

<220>
<221> VARIANT
<222> 184
<223> Xaa at the 184th position may be preferably a hydrophobic amino acid, e.g.
suitably Leu or Pro.

<220>
<221> VARIANT
<222> 185
<223> Xaa at the 185th position may be preferably a hydrophilic amino acid, e.g.
suitably Asp or Gly.

<220>
<221> VARIANT
<222> 186
<223> Xaa at the 186th position may be preferably a hydrophilic amino acid, e.g.
suitably Glu or Asn.

<220>
<221> VARIANT
<222> 191
<223> Xaa at the 191st position may be preferably a hydrophobic amino acid, e.g.
suitably Leu or Pro.

<400> 32
Met Glu Xaa Ser Val Val Xaa Pro Ser Val Phe Val Val Asp Gly Gln
1               5                   10                  15

Thr Asp Ile Pro Phe Xaa Arg Leu Xaa Xaa Xaa His Arg Arg Xaa Xaa
            20                  25                  30
Cys Xaa Xaa Xaa Xaa Asp Gly Xaa Xaa Gly Ser Trp Glu Xaa Leu Ile
        35                  40                  45
Gln Xaa Xaa Arg Ser His Xaa Xaa Asn Pro Ala Ala His Leu Thr Gly
    50                  55                  60
Ala Asn Xaa Ser Leu Xaa Gly Xaa Gly Gly Pro Leu Leu Trp Glu Thr
65                  70                  75                  80
Xaa Leu Gly Leu Ala Phe Leu Arg Gly Leu Xaa Tyr His Asp Gly Ala
                85                  90                  95
Leu Val Xaa Xaa Xaa Xaa Gly Tyr Tyr Tyr Xaa Tyr Ser Lys Val Gln
            100                 105                 110
Leu Xaa Gly Val Gly Cys Pro Xaa Gly Leu Ala Xaa Xaa Ile Thr His
        115                 120                 125
Gly Leu Tyr Lys Arg Thr Xaa Arg Tyr Pro Glu Xaa Leu Glu Leu Leu
    130                 135                 140
Val Ser Xaa Xaa Ser Pro Cys Gly Arg Ala Xaa Xaa Ser Ser Arg Val
145                 150                 155                 160
Trp Trp Asp Ser Ser Phe Leu Gly Gly Val Val His Leu Glu Ala Gly
                165                 170                 175
Glu Xaa Val Val Val Arg Val Xaa Xaa Xaa Arg Leu Val Arg Xaa Arg
            180                 185                 190
Asp Gly Thr Arg Ser Tyr Phe Gly Ala Phe Met Val
195 200 204

<210> 33
<211> 33
<212> DNA
<213> Artificial Sequence

<220>
<223> synthetic oligonucleotide primer

<400> 33
gtagaattcg gccaacccag cagcacatct tac 33

<210> 34

<211> 33

<212> DNA

<213> Artificial Sequence

<220>

<223> synthetic oligonucleotide primer

<400> 34

aaatctagat attgctgggt ttgaggtgag tcc          33

## Description

### TECHNICAL FIELD

The present invention relates to a novel caspase 3 inhibitor and so forth.

### BACKGROUND ART

It has been recognized that apoptosis plays a physiologically important role in a mechanism for disposing of cells, which become unnecessary in the developmental process and adult body, or which become injured and hazardous to the life maintenance. Furthermore, it has been made clear that apoptosis is involved in cell proliferation and differentiation. These findings suggest the possibility of opening the path for establishment of a new concept of diseases and development of a new remedy through reconsideration of diseases in view of the abnormal programmed cell death ("A variety and a regulation of apoptosis" Clinical Immunology 27, 308-316 (1995)).

In vivo functions of apoptosis can be divided into the three primary categories. The first one is observed in the programmed cell death for morphogenesis in the developmental process, and involved in disposing of unnecessary cells in the developmental process. The second function is for maintaining the biological homeostasis. It is believed that a balance of cells in an organ is kept through two processes, i.e. proliferation and apoptosis to perform the regulation of the organ function and the turnover of cells. The third one is the biological defense to actively remove a population of cells, which are injured and become harmful by an extracellular or intracellular challenge, for example, precancerous cells or virus-affected cells, by inducing apoptosis in the cells. As mentioned above, apoptosis serves crucially for maintenance of biological functions, and accordingly it is reasonably considered that the disturbance of apoptosis will be associated with an onset and pathological condition of a certain disease.

In recent years, there have been the rising reports to support a possible therapy by the control of apoptosis. In other words, for treating diseases caused by an abnormal apoptosis, it has become possible to promote or inhibit the apoptosis by regulating molecules involved in the process. For example, clinically used are a chemical therapy, radiation therapy, hormone therapy, cytokine therapy, and the like to induce apoptosis in cancerous cells (Science 267, 1456-1462 (1995); Cancer 73, 2013-2026 (1994)). Other examples of therapies aiming at the induction of apoptosis by targeting receptors include a breast cancer therapy by a receptor antagonist, a therapy using a monoclonal antibody to Fas antigen, or repetitive dosing of autoantibody for treating autoimmune diseases. On the contrary, therapies for anemia and neutropenia using erythropoietin and granulocyte colony-stimulating factor aim at inhibition of apoptosis. All of these therapies are ones targeting signals and receptors of apoptosis.

Also studies are recently progressed on therapies targeting proteases including caspase family or protein phosphorylation or dephosphorylation. For example, an attempt is made to induce apoptosis in cancer cells using a tyrosine phosphorylation inhibitor. Further, an application of antisense nucleotides to p53 or bcl-2, which controls apoptosis, and inversely an expression induction of their genes have drawn attention for treatment of cancer or lymphoma. For example, the specific expression of bcl-2 in a tissue indicates the possibility of effectively treating a disease produced by promotion of cell death. In addition, an animal experiment has been tried to treat a breast cancer by expressing bcl-Xs, which inhibits bcl-2 function. Regulation of the apoptosis process is also possible, in which the target may be a protease, transglutaminase, endonuclease, or the like to treat symptomatically pathological conditions associated with an abnormal apoptosis, although such therapies have not been sufficiently investigated yet.

In view of the above, if the regulation of apoptosis process, in particular, the development of a medicament to inhibit directly or indirectly the activation of caspases can be achieved, the therapy of diseases caused by an abnormal apoptosis will become a reality.

### SUMMARY OF THE INVENTION

In the process where TL4, a ligand molecule ofTNF family (WO98/03648) inhibits TNFα-induced apoptosis in normal human hepatic parenchymal cells, we unexpectedly found that the TL4-induced inhibition of caspase 3 activation played a central role in the inhibitory process. This indicates a possibility that administration of TL4 may be a new remedy for diseases, which is associated with caspase 3 as a central cause. A further study made us achieve the present invention.

The present invention provides:
(1) A caspase 3 inhibitor comprising a protein comprising the same or substantially the same amino acid sequence as that represented by SEQ ID NO: 1, 2, 3 or 31 or a salt thereof.
(2) The inhibitor according to (1) wherein substantially the same amino acid sequence as that represented by SEQ ID NO: 1, 2 or 3 includes an amino acid sequence having the amino acid sequences from aa (amino acid residue) 8 to aa 21, aa 54 to aa 59, aa93 to aa 102, aa 109 to aa 116, aa 118 to aa 126, aa 128 to aa 134, aa 144 to aa 149, aa 162 to aa 170, aa 176 to aa 182, aa 184 to aa 189, aa 193 to aa 213, aa 215 to aa 219, and aa 228 to aa 240 in the amino acid sequence represented by SEQ ID NO: 1.
(3) A caspase 3 inhibitor comprising a partial peptide of the protein described in (1) or a salt thereof.
(4) The inhibitor according to (3) wherein the partial peptide of the protein described in (1) includes the peptide consisting of the amino acid sequence from aa 84 to aa 240 in the amino acid sequence represented by SEQ ID NO: 1.
(5) A caspase 3 inhibitor comprising a DNA comprising a DNA encoding the protein described in (1) or the partial peptide described in (3).
(6) The inhibitor according to (5) wherein the DNA includes a DNA having the nucleotide sequence represented by any one of SEQ ID NOs: 4 to 10 and 30.
(7) The inhibitor according to (1), (3) or (5), which is a prophylactic and/or therapeutic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.
(8) A diagnostic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect, which comprises an antibody to a protein comprising the same or substantially the same amino acid sequence as that represented by SEQ ID NO: 1, 2, 3 or 31, a partial peptide or a salt thereof.
(9) A diagnostic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect, which comprises a DNA comprising a DNA encoding a protein comprising the same or substantially the same amino acid sequence as that represented by SEQ ID NO: 1, 2, 3 or 31 or a partial peptide thereof.
(10) A method of inhibiting caspase 3 in a mammal, which comprises administering an effective amount of a protein comprising the same or substantially the same amino acid sequence as that represented by SEQ ID NO: 1, 2, 3 or 31 or a salt thereof to the mammal.
(11) A method of preventing and/or treating AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect in a mammal, which comprises administering an effective amount of a protein comprising the same or substantially the same amino acid sequence as that represented by SEQ ID NO: 1, 2, 3 or 31 or a salt thereof to the mammal.
(12) A method of inhibiting caspase 3 in a mammal, which comprises administering an effective amount of a DNA comprising a DNA encoding a protein comprising the same or substantially the same amino acid sequence as that represented by SEQ ID NO: 1, 2, 3 or 31 or a partial peptide thereof to the mammal.
(13) A method of preventing and/or treating AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect in a mammal, which comprises administering an effective amount of a DNA comprising a DNA encoding a protein comprising the same or substantially the same amino acid sequence as that represented by SEQ ID NO: 1, 2, 3 or 31 or a partial peptide thereof to the mammal.
(14) A use of a protein comprising the same or substantially the same amino acid sequence as that represented by SEQ ID NO: 1, 2, 3 or 31 or a salt thereof for the production of a caspase 3 inhibitor.
(15) A use of a protein comprising the same or substantially the same amino acid sequence as that represented by SEQ ID NO: 1, 2, 3 or 31 or a salt thereof for the production of a prophylactic and/or therapeutic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.
(16) A use of a DNA comprising a DNA encoding a protein comprising the same or substantially the same amino acid sequence as that represented by SEQ ID NO: 1, 2, 3 or 31 or a partial peptide thereof for the production of a caspase 3 inhibitor.
(17) A use of a DNA comprising a DNA encoding a protein comprising the same or substantially the same amino acid sequence as that represented by SEQ ID NO: 1, 2, 3 or 31 or a partial peptide thereof for the production of a prophylactic and/or therapeutic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.

### BRIEF DESCRIPTION OF THE DRAWINGS

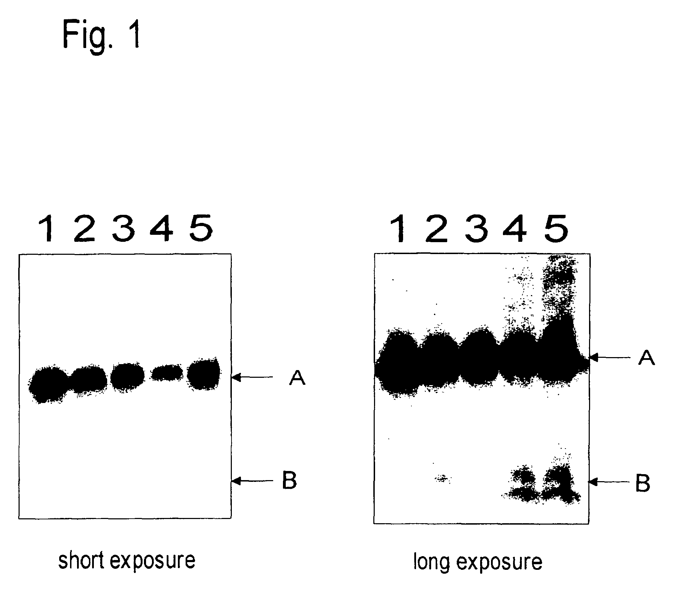

Fig. 1 shows the caspase 3 inhibiting activity of the protein of the invention, as observed in Example 1. Samples used in this figure are as follows: lane 1: no stimulation; lane 2: 4 hr after stimulation without pretreatment; lane 3: 4 hr after stimulation with pretreatment with the protein of the invention; lane 4: 14 hr after stimulation without pretreatment; lane 5: 14 hr after stimulation with pretreatment with the protein of the invention. "A" indicates the band of caspase 3 precursor, and "B" indicates the band of mature caspase 3.

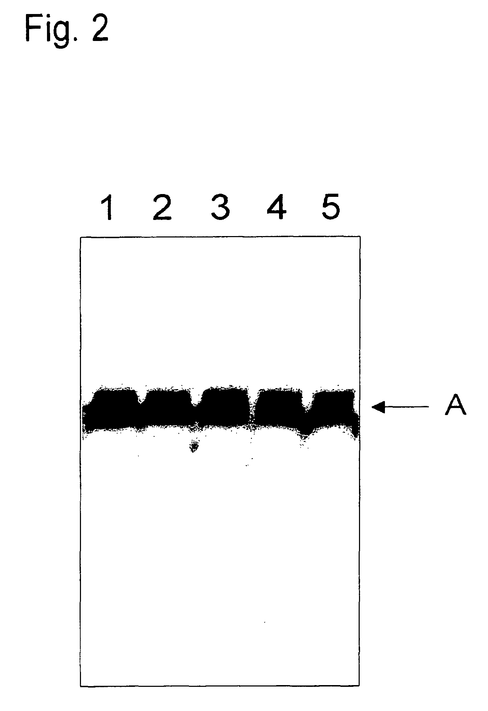

Fig. 2 shows the control experiment using an anti-actin antibody, conducted in Example 1. Samples used in this figure are as follows: lane 1: no stimulation; lane 2: 4 hr after stimulation without pretreatment; lane 3: 4 hr after stimulation with pretreatment with the protein of the invention; lane 4: 14 hr after stimulation without pretreatment; lane 5: 14 hr after stimulation with pretreatment with the protein of the invention. "A" indicates the band of actin.

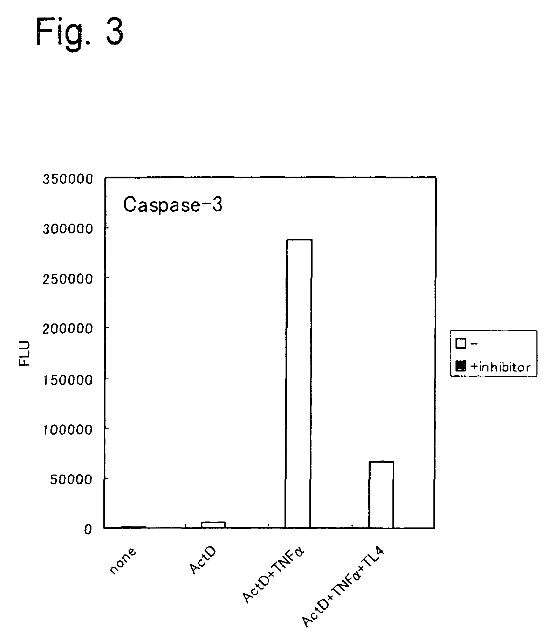

Fig. 3 shows the inhibitory activity of TL4 on the caspase 3 activation induced by Actinomycin D (ActD) and TNFα. The abscissa axis indicates the following cases: none: no addition; ActD: addition of ActD; ActD+TNFα: addition of ActD and TNFα; ActD+TNFα+TL4: addition of ActD, TNFα and hTL4-2. The ordinate axis indicates the caspase 3 activity.

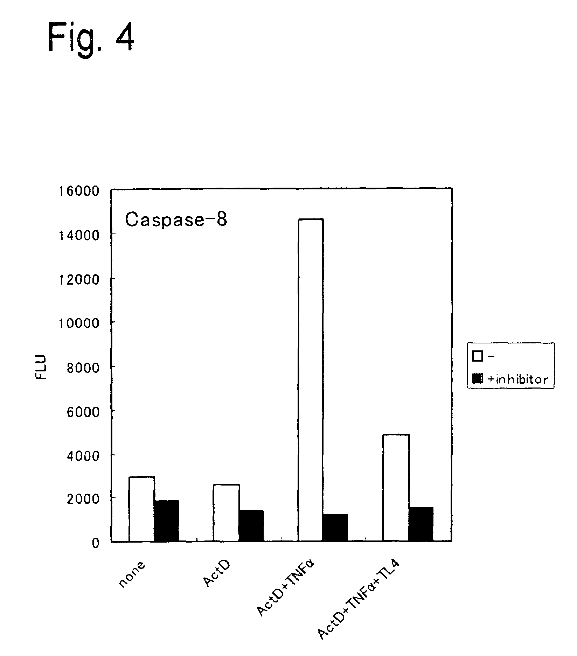

Fig. 4 shows the inhibitory activity of TL4 on the caspase 8 activation induced by ActD and TNFα. The abscissa axis indicates the following cases: none: no addition; ActD: addition of ActD; ActD+TNFα: addition of ActD and TNFα; ActD+TNFα+TL4: addition of ActD, TNFα and hTL4-2. The ordinate axis indicates the caspase 8 activity.

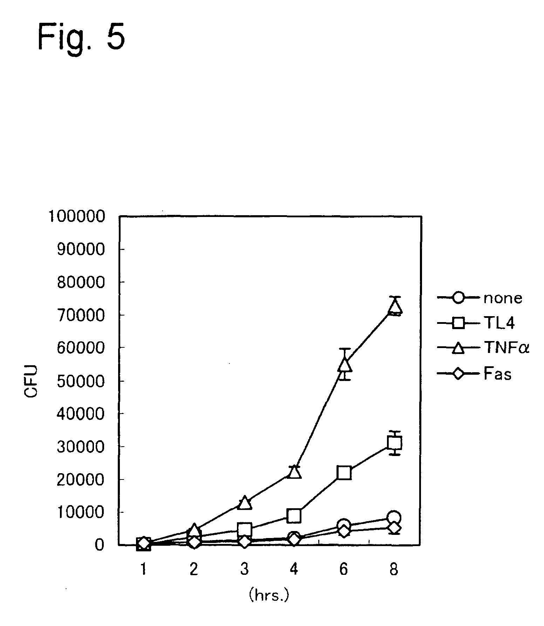

Fig. 5 shows the NF-κB activation by TL4 in hepatic parenchymal cells. There are following cases: -○-: no addition; -Δ-: addition of TL4 (hTL4-2); -□-: addition of TNFα; -◇-: addition of Fas. The ordinate axis indicates the NF-κB activity.

### BEST MODE FOR CARRYING OUT THE INVENTION

The protein contained in the caspase 3 inhibitor of the invention (hereinafter occasionally referred as to the protein of the invention) comprises the same or substantially the same amino acid sequence as that represented by SEQ ID NO: 1, 2, 3 or 31.

The protein of the invention may be derived from any cells of a human or non-human warm-blooded animals (e.g. guinea pig, rat, mouse, chicken, rabbit, swine, sheep, bovine, horse, monkey) (e.g. splenocytes, nerve cells, glial cells, pancreas beta cells, bone marrow cells, mesangial cells, Langerhans' cells, epidermic cells, epithelial cells, endothelial cells, fibroblasts, fibrocytes, myocytes, fat cells, immune cells (e.g. macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells); or any tissues where such cells are present, e.g. brain or any brain regions (e.g. olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g. large intestine, small intestine, duodenum), blood vessel, heart, thymus, spleen, submandibular gland, peripheral blood, prostate, testis, ovary, placenta, uterus, bone, joint, skeletal muscle, etc.; the protein may also be synthetic.

The protein of the invention includes proteins described in WO 98/03648 and WO 97/34911.

The protein of the invention also includes proteins (polypeptides) having the ligand activity to receptor proteins as described in J. Clin. Invest. 102, 1142-1151 (1998) and US patent No. 5,874,240.

The amino acid sequence which is substantially the same as the amino acid sequence represented by SEQ ID NO: 1, 2 or 3 includes an amino acid sequence having at least about 40% homology, preferably at least about 60% homology, more preferably at least about 80% homology, even more preferably at least about 90% homology, and most preferably at least about 95% homology to the amino acid sequence represented by SEQ ID NO: 1,2 or 3.

In particular, preferred are amino acid sequences having at least about 40%, preferably at least about 60%, more preferably at least about 80%, even more preferably at least about 90% homology to the amino acid sequence from aa 84 to aa 240 of the amino acid sequence of SEQ ID NO: 1, the amino acid sequence from aa 82 to aa 239 of the amino acid sequence of SEQ ID NO: 2, or the amino acid sequence from aa 82 to aa 239 of the amino acid sequence of SEQ ID NO: 3.

In addition, the amino acid sequence which is substantially the same as the amino acid sequence represented by SEQ ID NO: 1, 2 or 3 preferably includes an amino acid sequence having the constitutional amino acid sequences from aa 8 to aa 21, aa 55 to aa 59, aa 93 to aa 102,aa 109 to aa 116, aa 118 to aa 126, aa 128 to aa 134,aa 144 to aa 149, aa 162 to aa 170, aa 176 to aa 182, aa 184 to aa 189, aa 193 to aa 213, aa 215 to aa 219, and aa 228 to aa 239 in the amino acid sequence represented by SEQ ID NO: 1. These amino acid sequences are the common sequences among the amino acid sequences of SEQ ID NO: 1, SEQ ID NO: 2 and SEQ ID NO: 3.

In addition, the amino acid sequence which is substantially the same as the amino acid sequence represented by SEQ ID NO: 1 or 2 preferably includes an amino acid sequence having the constitutional amino acid sequences from aa 8 to aa 21, aa 54 to aa 59, aa 93 to aa 102,aa 109 to aa 116, aa 118 to aa 126, aa 128 to aa 134, aa 144 to aa 149, aa 162 to aa 170, aa 176 to aa 182, aa 184 to aa 189, aa 193 to aa 213, aa 215 to aa 219, and aa 228 to aa 240 in the amino acid sequence represented by SEQ ID NO: 1. These amino acid sequences are correspondent to the amino acid sequences aa 6 to aa 20, aa 52 to aa 57, aa 91 to aa 100, aa 107 to aa 114, aa 116 to aa 124, aa 126 to aa 132, aa 142 to aa 147, aa 162 to aa 170, aa 176 to aa 182, aa 184 to aa 189, aa 192 to aa 212, aa 214 to aa 218, and aa 227 to aa 239 in the amino acid sequence represented by SEQ ID NO: 2, and the common sequences between the amino acid sequences of SEQ ID NO: 1 and SEQ ID NO: 2.

Furthermore, the amino acid sequence which is substantially the same as the amino acid sequence represented by SEQ ID NO: 31 preferably includes an amino acid sequence having the constitutional amino acid sequences from aa 8 to aa 21, aa 57 to aa 66, aa 73 to aa 80, aa 82 to aa 90, aa 92 to aa 98, aa 108 to aa 111, aa 126 to aa 134, aa 140 to aa 146, aa 148 to aa 153, aa 157 to aa 177, aa 179 to aa 183, and aa 192 to aa 204 in the amino acid sequence represented by SEQ ID NO: 31.

The preferred protein of the invention comprising substantially the same amino acid sequence as that represented by SEQ ID NO: 1, 2, 3 or 31 includes a protein having substantially the same amino acid sequence as that represented by SEQ ID NO: 1, 2, 3 or 31 as described above and having an activity substantially equivalent in property to that of the protein comprising the amino acid sequence represented by SEQ ID NO: 1, 2, 3 or 31.

An example of "an activity substantially equivalent in property" may be the caspase 3 inhibiting activity.

The caspase 3 inhibiting activity refers to the activity to inhibit the activation of caspase 3, a protease which is activated through the partial cleavage thereof on the induction of cell death and degrades a variety of intracellular proteins to conduct the cell death.

The term "substantially equivalent in property" is used to express that the activity is the same in property (physiologically and pharmacologically). Accordingly, it is preferred that the activity including caspase 3 inhibiting activity has the same level (e.g., about 0.01 to 20 folds, preferably about 0.2 to 5 folds, more preferably about 0.5 to 2 folds), while quantitative factors such as a level of the activity and a molecular weight of the protein may be different.

The activity including caspase 3 inhibiting activity can be determined by a known method or a variation thereof (e.g. methods described in Trends Biochem. Sci. 22, 388-393 (1997); Biochem. J. 326, 1-16 (1997); or Anal. Biochem. 251, 98-102 (1997)), or a method described in the screening method or Examples described later.

The protein of the invention also includes so-called muteins such as proteins comprising (i) the amino acid sequence represented by SEQ ID NO: 1, 2, 3 or 31, from which one or more (e.g. 1 to 80, preferably 1 to 20, more preferably 1 to 9, and even more preferably several (e.g. 1 to 5)) amino acids are deleted; (ii) the amino acid sequence represented by SEQ ID NO: 1, 2, 3 or 31, to which one or more (e.g. 1 to 80, preferably 1 to 20, more preferably 1 to 9, and even more preferably several (e.g. 1 to 5)) amino acids are added; (iii) the amino acid sequence represented by SEQ ID NO: 1, 2, 3 or 31, in which one or more (e.g. 1 to 80, preferably 1 to 20, more preferably 1 to 9, and even more preferably several (e.g. 1 to 5)) amino acids are substituted by other amino acids; and (iv) an amino acid sequence having a combination of the above modifications.

When such a deletion or substitution is made in the amino acid sequence as described above, there is no special limitation to positions for the deletion or substitution. For example, these positions include:
(i) positions other than those in aa 8 to aa 21, aa 55 to aa 59 (or aa 54 to aa 59), aa 93 to aa 102, aa 109 to aa 116,aa 118 to aa 126, aa 128 to aa 134, aa 144 to aa 149, aa 162 to aa 170, aa 176 to aa 182, aa 184 to aa 189, aa 193 to aa 213, aa 215 to aa 219, and aa 228 to aa 239 (or aa 228 to aa 240) in the amino acid sequence represented by SEQ ID NO: 1, preferably positions other than those in aa 93 to aa 102, aa 109 to aa 116, aa 118 to aa 126, aa 128 to aa 134, aa 144 to aa 149, aa 162 to aa 170, aa 176 to aa 182, aa 184 to aa 189, aa 193 to aa 213, aa 215 to aa 219, and aa 228 to aa 240 in the amino acid sequence represented by SEQ ID NO: 1;
(ii) positions other than those in aa 6 to aa 19, aa 53 to aa 57 (or aa 52 to aa 57), aa 91 to aa 100, aa 107 to aa 114, aa 116 to aa 124, aa 126 to aa 132, aa 142 to aa 147, aa 162 to aa 170, aa 176 to aa 182, aa 184 to aa 189, aa 192 to aa 212, aa 214 to aa 218, and aa 227 to aa 238 (or aa 227 to aa 239) in the amino acid sequence represented by SEQ ID NO: 2, preferably positions other than those in aa 91 to aa 100, aa 107 to aa 114, aa 116 to aa 124, aa 126 to aa 132, aa 142 to aa 147, aa 162 to aa 170, aa 176 to aa 182, aa 184 to aa 189, as 192 to aa 212, aa 214 to aa 218, and aa 227 to aa 239 in the amino acid sequence represented by SEQ ID NO: 2;
(iii) positions other than those in aa 6 to aa 19, aa 53 to aa 57 (or aa 52 to aa 57), aa 91 to aa 100, aa 107 to aa 114, aa 116 to aa 124, aa 126 to aa 132, aa 142 to aa 147, aa 162 to aa 170, aa 176 to aa 182, aa 184 to aa 189, aa 192 to aa 212, aa 214 to aa 218, and aa 227 to aa 238 (or aa 227 to aa 239) in the amino acid sequence represented by SEQ ID NO: 3, preferably positions other than those in as 91 to aa 100, aa 107 to aa 114, aa 116 to aa 124, aa 126 to aa 132, aa 142 to aa 147, aa 162 to aa 170, aa 176 to aa 182, aa 184 to aa 189, aa 192 to aa 212, aa 214 to aa 218, and aa 227 to aa 239 in the amino acid sequence represented by SEQ ID NO: 3; and
(iv) positions other than those in aa 8 to aa 21, aa 57 to aa 66, aa 73 to aa 80, aa 82 to aa 90, aa 92 to aa 98, aa 108 to aa 111, aa 126 to aa 134, aa 140 to aa 146, aa 148 to aa 153, aa 157 to aa 177, aa 179 to aa 183, and aa 192 to aa 204 in the amino acid sequence represented by SEQ ID NO: 31.

Further, a preferred example of the protein comprising substantially the same amino acid sequence as that represented by SEQ ID NO: 1, 2 or 3 includes a protein having the amino acid sequence represented the following general formula (the amino acid sequence represented by SEQ ID NO: 25): 
Met Glu Xaa Ser Val Val Xaa Pro Ser Val Phe Val Val Asp Gly Gln
1               5                   10                  15
Thr Asp Ile Pro Phe Xaa Arg Leu Xaa Xaa Xaa His Arg Arg Xaa Xaa
            20                  25                  30
Cys Xaa Xaa Xaa Xaa Val Xaa Leu Xaa Leu Xaa Leu Leu Leu Xaa Gly
        35                  40                  45
Ala Gly Leu Ala Xaa Gln Gly Trp Phe Leu Leu Xaa Leu His Xaa Arg
    50                  55                  60
Leu Gly Xaa Xaa Vla Xaa Xaa Leu Pro Asp Gly Xaa Xaa Gly Ser Trp
65                  70                  75                  80
Glu Xaa Leu Ile Gln Xaa Xaa Arg Ser His Xaa Xaa Asn Pro Ala Ala
                85                  90                  95
His Leu Thr Gly Ala Asn Xaa Ser Leu Xaa Gly Xaa Gly Gly Pro Leu
            100                 105                 110
Leu Trp Glu Thr Xaa Leu Gly Leu Ala Phe Leu Arg Gly Leu Xaa Tyr
        115                 120                 125
His Asp Gly Ala Leu Val Xaa Xaa Xaa Xaa Gly Tyr Tyr Tyr Xaa Tyr
    130                 135                 140
Ser Lys Val Gln Leu Xaa Gly Val Gly Cys Pro Xaa Gly Leu Ala Xaa
145                 150                 155                 160
Xaa Xaa Xaa Ile Thr His Gly Leu Tyr Lys Arg Thr Xaa Arg Tyr Pro
 
165 170 175
Glu Xaa Leu Glu Leu Leu Val Ser Xaa Xaa Ser Pro Cys Gly Arg Ala
180 185 190
Xaa Xaa Ser Ser Arg Val Trp Trp Asp Ser Ser Phe Leu Gly Gly Val
195 200 205
Val His Leu Glu Ala Gly Glu Xaa Val Val Val Arg Val Xaa Xaa Xaa
210 215 220
Arg Leu Val Arg Xaa Arg Asp Gly Thr Arg Ser Tyr Phe Gly Ala Phe
225 230 235 240
Met Val (I)
 wherein Xaa is any amino acid residue or a bond.

In addition, in the general formula (I), one or more (for example 1 to 56, preferably 1 to 40, more preferably 1 to 20, even more preferably 1 to 9, most preferably several (1 to 5)) Xaa residues may be deleted.

An amino acid represented by Xaa may be a hydrophilic or hydrophobic amino acid, and may be an acidic, basic or neutral amino acid, and specifically includes Gly, Ala, Val, Leu, Ile, Ser, Thr, Cys, Met, Glu, Asp, Lys, Arg, His, Phe, Tyr, Trp, Pro, Asn, Gln, and so on.

In the general formula (I),
Xaa at the 3rd position may be preferably Glu or may be deleted;
Xaa at the 7th position may be preferably a hydrophilic amino acid, e.g. suitably Arg or Gln;
Xaa at the 22nd position may be preferably a hydrophilic amino acid, e.g. suitably Thr or Arg;
Xaa at the 25th position may be preferably a hydrophilic amino acid, e.g. suitably Gly or Glu;
Xaa at the 26th position may be preferably a hydrophilic amino acid, e.g. suitably Arg or Gln;
Xaa at the 27th position may be preferably a hydrophilic amino acid, e.g. suitably Ser or Asn;
Xaa at the 31st position may be preferably a hydrophilic amino acid, e.g. suitably Gln or Arg;
Xaa at the 32nd position may be preferably a hydrophilic amino acid, e.g. suitably Ser or Arg;
Xaa at the 34th position may be preferably a hydrophilic amino acid, e.g. suitably Ser or Gly;
Xaa at the 35th position may be e.g. suitably Val or Thr;
Xaa at the 36th position may be preferably a hydrophobic amino acid, e.g. suitably Ala or Val;
Xaa at the 37th position may be preferably a hydrophilic amino acid, e.g. suitably Arg or Gin;
Xaa at the 39th position may be preferably a hydrophilic amino acid, e.g. suitably Gly or Ser;
Xaa at the 41st position may be e.g. suitably Gly or Ala;
Xaa at the 43rd position may be preferably a hydrophobic amino acid, e.g. suitably Leu or Val;
Xaa at the 47th position may be preferably Met or may be deleted;
Xaa at the 53rd position may be e.g. suitably Val or Thr;
Xaa at the 60th position may be preferably a hydrophilic amino acid, e.g. suitably Gln or Arg;
Xaa at the 63rd position may be e.g. suitably Trp or Gln;
Xaa at the 67th position may be preferably an acidic amino acid, e.g. suitably Glu or Asp;
Xaa at the 68th position may be preferably a hydrophobic amino acid, e.g. suitably Met or lie;
Xaa at the 70th position may be e.g. suitably Thr or Ala;
Xaa at the 71st position may be preferably a basic amino acid, e.g. suitably Arg or His; Xaa at the 76th position may be e.g. suitably Pro or Gly;
Xaa at the 77th position may be e.g. suitably Ala or Lys;
Xaa at the 82nd position may be preferably a hydrophilic amino acid, e.g. suitably Gln or Lys;
Xaa at the 86th position may be preferably an acidic amino acid, e.g. suitably Glu or Asp;
Xaa at the 87th position may be preferably a hydrophilic amino acid, e.g. suitably Arg or Gln;
Xaa at the 91st position may be preferably a hydrophilic amino acid, e.g. suitably Glu or Gin;
Xaa at the 92nd position may be preferably a hydrophobic amino acid, e.g. suitably Val or Ala;
Xaa at the 103rd position may be e.g. suitably Ser or Ala;
Xaa at the 106th position may be e.g. suitably Thr or Ile;
Xaa at the 108th position may be e.g. suitably Ser or Ile;
Xaa at the 117th position may be preferably a hydrophilic amino acid, e.g. suitably Gln or Arg;
Xaa at the 127th position may be preferably a hydrophilic amino acid, e.g. suitably Ser or Thr;
Xaa at the 135th position may be e.g. suitably Val or Thr;
Xaa at the 136th position may be e.g. suitably Thr or Met;
Xaa at the 137th position may be preferably a hydrophilic amino acid, e.g. suitably Lys or Glu;
Xaa at the 138th position may be preferably a hydrophobic amino acid, e.g. suitably Ala or Pro;
Xaa at the 143rd position may be preferably a hydrophobic amino acid, e.g. suitably Ile or Val;
Xaa at the 150th position may be preferably a hydrophilic amino acid, e.g. suitably Gly or Ser;
Xaa at the 156th position may be e.g. suitably Leu or Gln;
Xaa at the 160th position may be preferably a hydrophilic amino acid, e.g. suitably Ser or Asn;
Xaa at the 161st position may be preferably a hydrophilic amino acid, e.g. suitably Thr or Gly;
Xaa at the 162nd position may be preferably Leu or may be deleted;
Xaa at the 163rd position may be preferably Pro or may be deleted;
Xaa at the 173rd position may be e.g. suitably Pro or Ser;
Xaa at the 178th position may be preferably a hydrophilic amino acid, e.g. suitably Glu or Lys;
Xaa at the 185th and 186th positions may be preferably a hydrophilic amino acid, e.g. suitably Gln or Arg;
Xaa at the 193th position may be preferably Thr or may be deleted;
Xaa at the 194th position may be preferably a hydrophilic amino acid, e.g. suitably Ser or Asn;
Xaa at the 216th position may be preferably a hydrophilic amino acid, e.g. suitably Lys or Glu;
Xaa at the 222th position may be preferably a hydrophobic amino acid, e.g. suitably Leu or Pro;
Xaa at the 223th position may be preferably a hydrophilic amino acid, e.g. suitably Asp or Gly;
Xaa at the 224th position may be preferably a hydrophilic amino acid, e.g. suitably Glu or Asn;
Xaa at the 229th position may be preferably a hydrophobic amino acid, e.g. suitably Leu or Pro.

Further, a preferred example of the protein comprising substantially the same amino acid sequence as that represented by SEQ ID NO: 31 includes a protein having the amino acid sequence represented the following general formula (the amino acid sequence represented by SEQ ID NO: 32): 
Met Glu Xaa Ser Val Val Xaa Pro Ser Val Phe Val Val Asp Gly Gln
1               5                   10                  15
Thr Asp Ile Pro Phe Xaa Arg Leu Xaa Xaa Xaa His Arg Arg Xaa Xaa
            20                  25                  30
Cys Xaa Xaa Xaa Xaa Asp Gly Xaa Xaa Gly Ser Trp Glu Xaa Leu Ile
        35                  40                  45
Gln Xaa Xaa Arg Ser His Xaa Xaa Asn Pro Ala Ala His Leu Thr Gly
    50                  55                  60
Ala Asn Xaa Ser Leu Xaa Gly Xaa Gly Gly Pro Leu Leu Trp Glu Thr
65                  70                  75                  80
Xaa Leu Gly Leu Ala Phe Leu Arg Gly Leu Xaa Tyr His Asp Gly Ala
                85                  90                  95
Leu Val Xaa Xaa Xaa Xaa Gly Tyr Tyr Tyr Xaa Tyr Ser Lys Val Gln
            100                 105                 110
Leu Xaa Gly Val Gly Cys Pro Xaa Gly Leu Ala Xaa Xaa Ile Thr His
        115                 120                 125
Gly Leu Tyr Lys Arg Thr Xaa Arg Tyr Pro Glu Xaa Leu Glu Leu Leu
    130                 135                 140
Val Ser Xaa Xaa Ser Pro Cys Gly Arg Ala Xaa Xaa Ser Ser Arg Val
145                 150                 155                 160
Trp Trp Asp Ser Ser Phe Leu Gly Gly Val Val His Leu Glu Ala Gly
                165                 170                 175
Glu Xaa Val Val Val Arg Val Xaa Xaa Xaa Arg Leu Val Arg Xaa Arg
            180                 185                 190
Asp Gly Thr Arg Ser Tyr Phe Gly Ala Phe Met Val     (II)
        195                 200                 204
 wherein Xaa is any amino acid residue or a bond.

In addition, in the general formula (II), one or more (e.g. 1 to 40, preferably 1 to 20, more preferably 1 to 9, most preferably several (1 to 5)) Xaa residues may be deleted.

An amino acid represented by Xaa may be a hydrophilic or hydrophobic amino acid, and may be an acidic, basic or neutral amino acid, and specifically includes Gly, Ala, Val, Leu, lle, Ser, Thr, Cys, Met, Glu, Asp, Lys, Arg, His, Phe, Tyr, Trp, Pro, Asn, Gln, and so on.

In the general formula (II),
Xaa at the 3rd position may be preferably Glu or may be deleted;
Xaa at the 7th position may be preferably a hydrophilic amino acid, e.g. suitably Arg or Gln;
Xaa at the 22nd position may be preferably a hydrophilic amino acid, e.g. suitably Thr or Arg;
Xaa at the 25th position may be preferably a hydrophilic amino acid, e.g. suitably Gly or Glu;
Xaa at the 26th position may be preferably a hydrophilic amino acid, e.g. suitably Arg or Gin;
Xaa at the 27th position may be preferably a hydrophilic amino acid, e.g. suitably Ser or Asn;
Xaa at the 31st position may be preferably a hydrophilic amino acid, e.g. suitably Gln or Arg;
Xaa at the 32nd position may be preferably a hydrophilic amino acid, e.g. suitably Ser or Arg;
Xaa at the 34th position may be preferably a hydrophilic amino acid, e.g. suitably Ser or Gly;
Xaa at the 35th position may be e.g. suitably Val or Thr;
Xaa at the 36th position may be preferably a hydrophobic amino acid, e.g. suitably Ala or Val;
Xaa at the 37th position may be preferably a hydrophilic amino acid, e.g. suitably Arg or Gln;
Xaa at the 40th position may be e.g. suitably Pro or Gly;
Xaa at the 41st position may be e.g. suitably Ala or Lys;
Xaa at the 46th position may be preferably a hydrophilic amino acid, e.g. suitably Gln or Lys;
Xaa at the 50th position may be preferably an acidic amino acid, e.g. suitably Glu or Asp;
Xaa at the 51 st position may be preferably a hydrophilic amino acid, e.g. suitably Arg or Gln;
Xaa at the 55th position may be preferably a hydrophilic amino acid, e.g. suitably Glu or Gln;
Xaa at the 56th position may be preferably a hydrophobic amino acid, e.g. suitably Val or Ala;
Xaa at the 67th position may be e.g. suitably Ser or Ala;
Xaa at the 70th position may be e.g. suitably Thr or lle;
Xaa at the 72nd position may be e.g. suitably Ser or Ile;
Xaa at the 81st position may be preferably a hydrophilic amino acid, e.g. suitably Gln or Arg;
Xaa at the 91st position may be preferably a hydrophilic amino acid, e.g. suitably Ser or Thr;
Xaa at the 99th position may be e.g. suitably Val or Thr;
Xaa at the 100th position may be e.g. suitably Thr or Met;
Xaa at the 101st position may be preferably a hydrophilic amino acid, e.g. suitably Lys or Glu;
Xaa at the 102nd position may be preferably a hydrophobic amino acid, e.g. suitably Ala or Pro;
Xaa at the 107th position may be preferably a hydrophobic amino acid, e.g. suitably Ile or Val;
Xaa at the 114th position may be preferably a hydrophilic amino acid, e.g. suitably Gly or Ser;
Xaa at the 120th position may be e.g. suitably Leu or Gin;
Xaa at the 124th position may be preferably a hydrophilic amino acid, e.g. suitably Ser or Asn;
Xaa at the 125th position may be preferably a hydrophilic amino acid, e.g. suitably Thr or Gly;
Xaa at the 135th position may be preferably Pro or may be deleted;
Xaa at the 140th position may be preferably a hydrophilic amino acid, e.g. suitably Glu or Lys;
Xaa at the 147th and 148th positions may be preferably a hydrophilic amino acid, e.g. suitably Gln or Arg;
Xaa at the 155th position may be preferably Thr or may be deleted;
Xaa at the 156th position may be preferably a hydrophilic amino acid, e.g. suitably Ser or Asn;
Xaa at the 178th position may be preferably a hydrophilic amino acid, e.g. suitably Lys or Glu;
Xaa at the 184th position may be preferably a hydrophobic amino acid, e.g. suitably Leu or Pro;
Xaa at the 185th position may be preferably a hydrophilic amino acid, e.g. suitably Asp or Gly;
Xaa at the 186th position may be preferably a hydrophilic amino acid, e.g. suitably Glu or Asn;
Xaa at the 191st position may be preferably a hydrophobic amino acid, e.g. suitably Leu or Pro.

In the present specification, proteins are shown in a conventional way for peptide description with the N-terminal (amino terminal) on the left side and the C-terminal (carboxyl terminal) on the right side. The protein of the invention usually has the C-terminal in a form of carboxyl group (-COOH) or carboxylate (-COO⁻). An amide form (-CONH₂) or an ester form (-COOR) is also possible.

Examples of the ester group R include C₁₋₆ alkyl such as methyl, ethyl, n-propyl, isopropyl, n-butyl; C₃₋₈ cycloalkyl such as cyclopentyl, cyclohexyl; C₆₋₁₂ aryl such as phenyl, α-naphthyl; C₇₋₁₄ aralkyl such as phenyl-C₁₋₂ alkyl, e.g. benzyl, phenethyl, and α-naphthyl-C₁₋₂ alkyl, e.g. α-naphthylmethyl; and pivaloyloxymethyl, which is used widely as an oral ester form.

When the protein of the invention contains a carboxyl group (or a carboxylate) at a position other than the C-terminal, it may be amidated or esterified. Such an amide or ester is also included in the protein of the invention. In this case, the same ester groups as the above-mentioned C-terminal ester groups can be used.

Furthermore, examples of the protein of the invention include variants of the above proteins, wherein the N-terminal amino group of the protein is protected with a protecting group (e.g. a C₁₋₆ acyl group such as a C₁₋₆ alkanoyl group, e.g. formyl group, acetyl group, etc); those wherein the N-terminal region is cleaved in vivo and the glutamyl group thus formed is pyroglutaminated; those wherein a substituent (e.g. -OH, -SH, amino group, imidazole group, indole group, guanidino group, etc) on the side chain of an amino acid in the molecule is protected with a suitable protecting group (e.g. a C₁₋₆ acyl group such as a C₁₋₆ alkanoyl group, e.g. formyl group, acetyl group, etc), or conjugated proteins such as glycoproteins bound to sugar chains.

More specifically, the protein of the invention includes a protein derived from a human liver having the amino acid sequence represented by SEQ ID NO: 1; a protein derived from a mouse embryo having the amino acid sequence represented by SEQ ID NO: 2; a protein derived from a rat liver having the amino acid sequence represented by SEQ ID NO: 3; and a protein derived from a human liver having the amino acid sequence represented by SEQ ID NO: 31.

The partial peptide of the protein of the invention may be any one having an activity equivalent in property to that of the protein of the invention described above, for example, the caspase 3 inhibiting activity. Preferably used are peptides having, for example, at least 20, preferably at least 50, more preferably at least 70, much more preferably at least about 100 and most preferably at least about 200 amino acid residues in the amino acid sequence which constitutes the protein of the invention.

Specifically used are:
(1) a partial peptide having at least one or more amino acid sequences selected from the group consisting of the amino acid sequences from aa 8 to aa 21, aa 55 to aa 59, aa 93 to aa 102, aa 109 to aa 116, aa 118 to aa 126, aa 128 to aa 134, aa 144 to aa 149, aa 162 to aa 170, aa 176 to aa 182, aa 184 to aa 189, aa 193 to aa 213, aa 215 to aa 219, and aa 228 to aa 239 in the amino acid sequence represented by SEQ ID NO: 1 (or a partial peptide having at least one or more amino acid sequences selected from the group consisting of the amino acid sequences from aa 6 to aa 20, aa 53 to aa 57, aa 91 to aa 100, aa 107 to aa 114, aa 116 to aa 124, aa 126 to aa 132, aa 142 to aa 147, aa 162 to aa 170, aa 176 to aa 182, aa 184 to aa 189, aa 192 to aa 212, aa 214 to aa 218, and aa 227 to aa 238 in the amino acid sequence represented by SEQ ID NO: 2 or 3);
(2) a partial peptide having at least one or more amino acid sequences selected from the group consisting of the amino acid sequences from aa 8 to aa 21, aa 54 to aa 59, aa 93 to aa 102, aa 109 to aa 116, as 118 to aa 126, aa 128 to aa 134, aa 144 to aa 149, aa 162 to aa 170, aa 176 to aa 182, aa 184 to aa 189, aa 193 to aa 213, aa 215 to aa 219, and aa 228 to aa 240 in the amino acid sequence represented by SEQ ID NO: 1 (or a partial peptide having at least one or more amino acid sequences selected from the group consisting of the amino acid sequences from aa 6 to aa 20, aa 52 to aa 57, aa 91 to aa 100, aa 107 to aa 114, aa 116 to aa 124, aa 126 to aa 132, aa 142 to aa 147, aa 162 to aa 170, aa 176 to aa 182, aa 184 to aa 189, aa 192 to aa 212, aa 214 to aa 218, and aa 227 to aa 239 in the amino acid sequence represented by SEQ ID NO: 2 or 3); and
(3) a partial peptide having at least one or more amino acid sequences selected from the group consisting of the amino acid sequences from aa 8 to aa 21, aa 57 to aa 66, aa 73 to aa 80, aa 82 to aa 90, aa 92 to aa 98, aa 108 to aa 113, aa 126 to aa 134, aa 140 to aa 146, aa 148 to aa 153, aa 157 to aa 177, aa 179 to aa 183, and aa 192 to aa 203 in the amino acid sequence represented by SEQ ID NO: 31.

More specifically, preferably used are a partial peptide having the amino acid sequence from aa 84 to aa 240 in the amino acid sequence represented by SEQ ID NO: 1; a partial peptide having the amino acid sequence from as 82 to aa 239 in the amino acid sequence represented by SEQ ID NO: 2; a partial peptide having the amino acid sequence from aa 82 to aa 239 in the amino acid sequence represented by SEQ ID NO: 3; and a partial peptide having the amino acid sequence from aa 48 to aa 204 in the amino acid sequence represented by SEQ ID NO: 31.

Furthermore, preferably used are (i) a partial peptide which has substantially the same amino acid sequence as the sequence from aa 84 to aa 240 in the amino acid sequence represented by SEQ ID NO: 1 and has an activity substantially equivalent in property to that of the partial peptide having the amino acid sequence from aa 84 to aa 240 in the amino acid sequence represented by SEQ ID NO: 1; (ii) a partial peptide which has substantially the same amino acid sequence as the sequence from aa 82 to aa 239 in the amino acid sequence represented by SEQ ID NO: 2 and has an activity substantially equivalent in property to that of the partial peptide having the amino acid sequence from aa 82 to aa 239 in the amino acid sequence represented by SEQ ID NO: 2; (iii) a partial peptide which has substantially the same amino acid sequence as the sequence from aa 82 to aa 239 in the amino acid sequence represented by SEQ ID NO: 3 and has an activity substantially equivalent in property to that of the partial peptide having the amino acid sequence from aa 82 to aa 239 in the amino acid sequence represented by SEQ ID NO: 2; and (iv) a partial peptide which has substantially the same amino acid sequence as the sequence from aa 48 to aa 204 in the amino acid sequence represented by SEQ ID NO: 31 and has an activity substantially equivalent in property to that of the partial peptide having the amino acid sequence from aa 48 to aa 204 in the amino acid sequence represented by SEQ ID NO: 1.

The amino acid sequence which is substantially the same as the amino acid sequence from aa 84 to aa 240 in the amino acid sequence represented by SEQ ID NO: 1 includes an amino acid sequence having at least about 40% homology, preferably at least about 60% homology, more preferably at least about 80% homology, even more preferably at least about 90% homology, and most preferably at least about 95% homology to the amino acid sequence from aa 84 to aa 240 in the amino acid sequence represented by SEQ ID NO: 1.

The amino acid sequence which is substantially the same as the amino acid sequence from aa 82 to aa 239 in the amino acid sequence represented by SEQ ID NO: 2 includes an amino acid sequence having at least about 40% homology, preferably at least about 60% homology, more preferably at least about 80% homology, even more preferably at least about 90% homology, and most preferably at least about 95% homology to the amino acid sequence from aa 82 to aa 239 in the amino acid sequence represented by SEQ ID NO: 2.

The amino acid sequence which is substantially the same as the amino acid sequence from aa 82 to aa 239 in the amino acid sequence represented by SEQ ID NO: 3 includes an amino acid sequence having at least about 40% homology, preferably at least about 60% homology, more preferably at least about 80% homology, even more preferably at least about 90% homology, and most preferably at least about 95% homology to the amino acid sequence from aa 82 to aa 239 in the amino acid sequence represented by SEQ ID NO: 3.

The amino acid sequence which is substantially the same as the amino acid sequence from aa 48 to aa 204 in the amino acid sequence represented by SEQ ID NO: 31 includes an amino acid sequence having at least about 40% homology, preferably at least about 60% homology, more preferably at least about 80% homology, even more preferably at least about 90% homology, and most preferably at least about 95% homology to the amino acid sequence from aa 48 to aa 204 in the amino acid sequence represented by SEQ ID NO: 31.

The term "an activity substantially equivalent in property" has the same definition as described above.

Furthermore, the partial peptide of the invention includes a partial peptide comprising:
(i) the amino acid sequence from aa 84 to aa 240 in the amino acid sequence represented by SEQ ID NO: 1, from which one or more (e.g. 1 to 80, preferably 1 to 20, more preferably 1 to 9, and even more preferably several (e.g. 1 to 5)) amino acids are deleted; to which one or more (e.g. 1 to 80, preferably 1 to 20, more preferably 1 to 9, and even more preferably several (e.g. 1 to 5)) amino acids are added; in which one or more (e.g. 1 to 80, preferably 1 to 20, more preferably 1 to 9, and even more preferably several (e.g. 1 to 5)) amino acids are substituted by other amino acids; or which has a combination of the above modifications;
(ii) the amino acid sequence from aa 82 to aa 239 in the amino acid sequence represented by SEQ ID NO: 2, from which one or more (e.g. 1 to 80, preferably 1 to 20, more preferably 1 to 9, and even more preferably several (e.g. 1 to 5)) amino acids are deleted; to which one or more (e.g. 1 to 80, preferably 1 to 20, more preferably 1 to 9, and even more preferably several (e.g. 1 to 5)) amino acids are added; in which one or more (e.g. 1 to 80, preferably 1 to 20, more preferably 1 to 9, and even more preferably several (e.g. 1 to 5)) amino acids are substituted by other amino acids; or which has a combination of the above modifications;
(iii) the amino acid sequence from aa 82 to aa 239 in the amino acid sequence represented by SEQ ID NO: 3, from which one or more (e.g. 1 to 80, preferably 1 to 20, more preferably 1 to 9, and even more preferably several (e.g. 1 to 5)) amino acids are deleted; to which one or more (e.g. 1 to 80, preferably 1 to 20, more preferably 1 to 9, and even more preferably several (e.g. 1 to 5)) amino acids are added; in which one or more (e.g. 1 to 80, preferably 1 to 20, more preferably 1 to 9, and even more preferably several (e.g. 1 to 5)) amino acids are substituted by other amino acids; or which has a combination ofthe above modifications;
(iv) the amino acid sequence from aa 48 to aa 204 in the amino acid sequence represented by SEQ ID NO: 31, from which one or more (e.g. 1 to 80, preferably 1 to 20, more preferably 1 to 9, and even more preferably several (e.g. 1 to 5)) amino acids are deleted; to which one or more (e.g. 1 to 80, preferably 1 to 20, more preferably I to 9, and even more preferably several (e.g. 1 to 5)) amino acids are added; in which one or more (e.g. 1 to 80, preferably 1 to 20, more preferably 1 to 9, and even more preferably several (e.g. 1 to 5)) amino acids are substituted by other amino acids; or which has a combination of the above modifications.

When being deleted or substituted as described above, for example, a peptide having the amino acid sequence represented by general formula (I), which is deprived of the amino acid sequence from aa 1 to aa 83, may be preferably used.

The partial peptide of the invention usually has the C-terminal in a form of carboxyl group (-COOH) or carboxylate (-COO⁻). Like the protein of the invention as described above, an amide form (-CONH₂) or an ester form (-COOR) is also possible (R is defined as above).

Furthermore, like the protein of the invention as described above, the partial peptide of the invention includes variants of the above peptides, in which the N-terminal amino group is protected by a protecting group; those in which the N-terminal glutamine residue, newly formed by cleavage in vivo, is pyroglutaminated; those in which a substituent on the side chain of an amino acid in the molecule is protected by a suitable protecting group; or a conjugated peptide such as a so-called glycopeptide bound to sugar chains.

Suitable examples of the partial peptide of the invention include the peptide having the amino acid sequence from aa 84 to aa 240 in the amino acid sequence represented by SEQ ID NO: 1; the peptide having the amino acid sequence from aa 82 to aa 239 in the amino acid sequence represented by SEQ ID NO: 2; the peptide having the amino acid sequence from aa 82 to aa 239 in the amino acid sequence represented by SEQ ID NO: 3; and the peptide having the amino acid sequence from aa 48 to aa 204 in the amino acid sequence represented by SEQ ID NO: 31.

The salts of the protein of the invention and the partial peptide thereof include ones with physiologically acceptable acids (e.g. inorganic acids, organic acids) or bases (e.g. alkaline metals). Physiologically acceptable acid addition salts are particularly preferred. Examples of such salts are salts with inorganic acids (e.g. hydrochloric acid, phosphoric acid, hydrobromic acid, sulfuric acid), and salts with organic acids (e.g. acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The protein of the invention or the salt thereof can be produced from tissues or cells of the human or non-human warm-blooded animals as described above according to a known protein purification method; or can be produced by culturing a transformant comprising the DNA encoding the protein as described below; or can be produced according to the protein synthesis method or a variation thereof as described below. Specifically, methods described in WO98/03648 and WO97/34911 can be used for the production.

When the protein of the invention is produced from tissues or cells of the human or other warm-blooded animals, the tissues or cells are homogenized and extracted with acids or the like, and then the obtained extract can be subjected to a combination of chromatography techniques such as reversed phase chromatography, ion exchange chromatography, and the like to isolate or purify the protein.

To synthesize the protein of the invention or the partial peptide, or salts or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin. Using these resins, amino acids in which α-amino groups and functional groups on the side chains are appropriately protected are condensed on the resin in the order of the sequence of the objective protein according to various condensation methods publicly known. At the end of the reaction, the protein is excised from the resin and at the same time, the protecting groups are removed. Then, intramolecular disulfide bond-forming reaction is performed in a highly diluted solution to obtain the desired protein, the partial peptide, or the amide thereof.

For condensation of the protected amino acids described above, a variety of activation reagents for protein synthesis may be used, but carbodiimides are particularly preferably employed. Examples of such carbodiimides include DCC, N,N'-diisopropylcarbodiimide, N-ethyl-N'-(3.dimethylaminopropyl)carbodiimide. For activation by these reagents, the protected amino acids in combination with a racemization inhibitor (e.g. HOBt, HOOBt) are added directly to the resin, or the protected amino acids are previously activated in the form of symmetric acid anhydrides, HOBt esters or HOOBt esters, and then added to the resin.

Solvents used to activate the protected amino acids or condense them with the resin may be chosen from solvents that are known to be usable for protein condensation. For example, N,N-dimethylformamide, N-methylpyrrolidone, chloroform, trifluoroethanol, dimethylsulfoxide, DMF, pyridine, dioxane, methylene chloride, tetrahydrofuran, acetonitrile, ethyl acetate, and an appropriate mixture thereof are usable. The reaction temperature is appropriately chosen from the range known to be applicable to protein binding reactions and is usually selected in the range of approximately -20°C to 50°C. The activated amino acid derivatives are used generally in 1.5 to 4 times excess. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc.

A carboxyl group can be protected by e.g. alkyl ester (e.g. ester groups of methyl, ethyl, propyl, butyl, t-butyl, cyclopentyl, cyclohexyl, cycloheptyl, cyclooctyl, 2-adamantyl), benzyl ester, 4-nitrobenzyl ester, 4-methoxybenzyl ester, 4-chlorobenzyl ester, benzhydryl ester, phenacyl ester, benzyloxycarbonyl hydrazide, t-butoxycarbonyl hydrazide, trityl hydrazide.

The hydroxyl group of serine can be protected through, for example, its esterification or etherification. Examples of groups appropriately used for the esterification include a lower alkanoyl group such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group.

Examples of protecting groups for the phenolic hydroxyl group of tyrosine include Bzl, Cl₂-Bzl, 2-nitrobenzyl, Br-Z, t-butyl.

Examples of protecting groups for the imidazole moiety of histidine include Tos, 4-methoxy-2,3,6-trimethylbenzenesulfonyl, DNP, benzyloxymethyl, Bum, Boc, Trt, Fmoc.

Examples of the activated carboxyl groups in the starting material include the corresponding acid anhydrides, azides, activated esters (esters with alcohols (e.g. pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). Examples of the activated amino groups in the starting material include the corresponding phosphoric amide.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of about -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group used for protecting the imidazole of histidine is eliminated by treatment with thiophenol. Formyl group used for protecting the indole of tryptophan is eliminated by the aforesaid acid treatment in the presence of 1,2-ethanedithiol or 1,4-butanedithiol, or by alkali treatment with a dilute sodium hydroxide solution and dilute ammonia.

Protection of functional groups in the starting material, which should not be involved in the reaction, protecting groups, elimination of the protecting groups and activation of functional groups involved in the reaction may be publicly known and appropriately selected.

Another method for producing an amide of the protein is as follows. At first, the α-carboxyl group of the carboxy terminal amino acid is amidated for protection, and the peptide chain is then extended to amino group side for a desired length. Then, the protein in which only the protecting group of the N-terminal α-amino group of the peptide is eliminated, and the protein in which only the protecting group of the C-terminal carboxyl group is eliminated are produced. Both the proteins are condensed in a mixture of solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the desired amidated protein.

To prepare an ester of the protein, the α-carboxyl group of the carboxy terminal amino acid can be condensed with a desired alcohol to prepare the amino acid ester, which is then processed in a similar way to the preparation of the amidated protein to give the desired esterified protein.

The partial peptide of the invention or a salt thereof can be produced by a known peptide synthesis method, or by cleaving the protein of the invention with an appropriate peptidase. The peptide synthesis method may be, for example, either solid phase synthesis method or liquid phase synthesis method. That is, a partial peptide or amino acids that can construct the protein of the invention can be condensed with the remaining part, and when the product contains protecting groups, these protecting groups are removed to give the desired peptide. Publicly known methods for condensation and elimination of protecting groups are described in the following (i) to (v):
(i) M. Bodanszky & M.A. Ondetti: Peptide Synthesis, Interscience Publishers, New York (1966).
(ii) Schroeder & Luebke: The Peptide, Academic Press, New York (1965).
(iii) Nobuo Izumiya, et al.: Peptide Gosei-no-Kiso to Jikken (Basics and experiments of peptide synthesis), published by Maruzen Co. (1975).
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977).
(v) Haruaki Yajima ed.: Zoku Iyakuhin no Kaihatsu (A sequel to Development of Pharmaceuticals), Vol. 14, Peptide Synthesis, published by Hirokawa Shoten.

After completion of the reaction, the protein of the invention can be purified and isolated by a combination of conventional purification methods such as solvent extraction, distillation, column chromatography, liquid chromatography and recrystallization. When the protein obtained by the above methods is in a free form, it can be converted into an appropriate salt by a known method. On the other hand, when the protein is obtained in a salt form, it can be converted into a free form or a different salt form by a known method.

The DNA encoding the protein of the invention includes any DNAs comprising a nucleotide sequence encoding the aforementioned protein of the invention. In addition, the DNA may be derived from genome DNAs, genome DNA libraries, cDNAs from the aforementioned tissues and cells, cDNA libraries from the aforementioned tissues and cells, or synthetic DNAs. Vectors used for the libraries may be any one of bacteriophage, plasmid, cosmid, phagemid, and the like. The DNA may also be amplified directly by reverse transcriptase polymerase chain reaction (hereinafter abbreviated as RT-PCR) from mRNA fraction prepared from the above-mentioned tissues or cells.

The DNA of the invention also includes a DNA comprising a nucleotide sequence encoding the protein of the invention described in WO98/03648 and W097/34911.

Specifically, the DNA encoding the protein having the amino acid sequence represented by SEQ ID NO: 1 includes (i) the DNA having the nucleotide sequence represented by SEQ ID NO: 4; and (ii) a DNA, which is hybridizable to the DNA having the nucleotide sequence represented by SEQ ID NO: 4 under high stringent conditions and encodes a protein having an activity equivalent in property to that of the protein having the amino acid sequence represented by SEQ ID NO: 1 (e.g. caspase 3 inhibiting activity).

Examples of the DNA that is hybridizable to the DNA having the nucleotide sequence represented by SEQ ID NO: 4 under high stringent conditions include a DNA comprising a nucleotide sequence having at least about 40% homology, preferably at least about 60% homology, more preferably at least about 80% homology, even more preferably at least about 90% homology, and most preferably at least about 95% homology to the nucleotide sequence represented by SEQ ID NO: 4.

The DNA encoding the protein having the amino acid sequence represented by SEQ ID NO: 2 includes (i) the DNA having the nucleotide sequence represented by SEQ ID NO: 7; and (ii) a DNA, which is hybridizable to the DNA having the nucleotide sequence represented by SEQ ID NO: 7 under high stringent conditions and encodes a protein having an activity equivalent in property to that of the protein having the amino acid sequence represented by SEQ ID NO: 2.

Examples of the DNA that is hybridizable to the nucleotide sequence represented by SEQ ID NO: 7 under high stringent conditions include a DNA comprising a nucleotide sequence having at least about 40% homology, preferably at least about 60% homology, more preferably at least about 80% homology, even more preferably at least about 90% homology, and most preferably at least about 95% homology to the nucleotide sequence represented by SEQ ID NO: 7.

The DNA encoding the protein having the amino acid sequence represented by SEQ ID NO: 3 includes (i) the DNA having the nucleotide sequence represented by SEQ ID NO: 10; and (ii) a DNA, which is hybridizable to the DNA having the nucleotide sequence represented by SEQ ID NO: 10 under high stringent conditions and encodes a protein having an activity equivalent in property to that of the protein having the amino acid sequence represented by SEQ ID NO: 3.

Examples of the DNA that is hybridizable to the nucleotide sequence represented by SEQ ID NO: 10 under high stringent conditions include a DNA comprising a nucleotide sequence having at least about 40% homology, preferably at least about 60% homology, more preferably at least about 80% homology, even more preferably at least about 90% homology, and most preferably at least about 95% homology to the nucleotide sequence represented by SEQ ID NO: 10.

The DNA encoding the protein having the amino acid sequence represented by SEQ ID NO: 31 includes (i) the DNA having the nucleotide sequence represented by SEQ ID NO: 30; and (ii) a DNA, which is hybridizable to the DNA having the nucleotide sequence represented by SEQ ID NO: 30 under high stringent conditions and encodes a protein having an activity equivalent in property to that of the protein having the amino acid sequence represented by SEQ ID NO: 2.

Examples of the DNA that is hybridizable to the nucleotide sequence represented by SEQ ID NO: 30 under high stringent conditions include a DNA comprising a nucleotide sequence having at least about 40% homology, preferably at least about 60% homology, more preferably at least about 80% homology, even more preferably at least about 90% homology, and most preferably at least about 95% homology to the nucleotide sequence represented by SEQ ID NO: 30.

The hybridization can be carried out by a known method or a variation thereof, for example, by the method described in Molecular Cloning, 2nd Ed., J. Sambrook et al., Cold Spring Harbor Lab. Press, 1989. A commercially available library may also be used according to the attached manufacturer's protocol. The hybridization can be carried out more preferably under high stringent conditions.

The high stringent conditions used herein refer to, for example, a sodium concentration of about 19 to 40 mM, preferably about 19 to 20 mM and a temperature of about 50 to 70°C, preferably about 60 to 65°C. In particular, the condition of a sodium concentration of about 19 mM and a temperature of about 65°C is most preferred.

More specifically, the DNA encoding a protein comprising the amino acid sequence shown by SEQ ID NO: 1 includes the DNA having the nucleotide sequence shown by SEQ ID NO: 4. A DNA comprising the DNA encoding a protein comprising the amino acid sequence shown by SEQ ID NO: 1 includes the DNA having the nucleotide sequence shown by SEQ ID NO: 5 or 6.

The DNA encoding a protein comprising the amino acid sequence shown by SEQ ID NO: 2 includes the DNA having the nucleotide sequence shown by SEQ ID NO: 7. A DNA comprising the DNA encoding a protein comprising the amino acid sequence shown by SEQ ID NO: 2 includes the DNA having the nucleotide sequence shown by SEQ ID NO: 8 or 9.

The DNA encoding a protein comprising the amino acid sequence shown by SEQ ID NO; 3 includes the DNA having the nucleotide sequence shown by SEQ ID NO: 10.

The DNA encoding a protein comprising the amino acid sequence shown by SEQ ID NO: 31 includes the DNA having the nucleotide sequence shown by SEQ ID NO: 30.

The DNA encoding the partial peptide of the invention includes any DNAs comprising a nucleotide sequence encoding the aforementioned partial peptide of the invention. In addition, the DNA may be derived from genome DNAs, genome DNA libraries, cDNAs from the aforementioned tissues and cells, cDNA libraries from the aforementioned tissues and cells, or synthetic DNAs. Vectors used for the libraries may be any one of bacteriophage, plasmid, cosmid, phagemid, and the like. The DNA may also be amplified directly by RT-PCR from mRNA fraction prepared from the above-mentioned tissues or cells.

Specifically, the DNA encoding the partial peptide having at least one amino acid sequence selected from the group consisting of the amino acid sequences from aa 8 to aa 21, aa 55 to aa 59 (or aa 54 to aa 59), aa 93 to aa 102,aa 109 to aa 116, aa 118 to aa 126, aa 128 to aa 134, aa 144 to aa 149, aa 162 to aa 170, aa 176 to aa 182, aa 184 to aa 189, aa193 to aa 213, aa 215 to aa 219, and aa 228 to aa 239 (or aa 228 to aa 240) in the amino acid sequence represented by SEQ ID NO: 1 includes a DNA having at least one nucleotide sequence selected from the group consisting of the nucleotide sequences from nt (nucleotide) 22 to nt 63, nt 163 to nt 177 (or nt 160 to nt 177), nt 277 to nt 306, nt 325 to nt 348, nt 352 to nt 378, nt 382 to nt 402, nt 430 to nt 447, nt 484 to nt 510, nt 526 to nt 546, nt 550 to nt 567, nt 577 to nt 639, nt 643 to nt 657, and nt 682 to nt 717 (or nt 682 to nt 720) in the nucleotide sequence represented by SEQ ID NO: 4.

The DNA encoding the partial peptide having at least one amino acid sequence selected from the group consisting of the amino acid sequences from aa 6 to aa 20, aa 53 to aa 57 (or aa 52 to aa 57), aa 91 to aa 100, aa 107 to aa 114, aa 116 to aa 124, aa 126 to aa 132, aa 142 to aa 147, aa 162 to aa 170, aa 176 to aa 182, aa 184 to aa 189, aa 192 to aa 212, aa 214 to aa 218, and as 227 to as 238 (or aa 227 to aa 239) in the amino acid sequence represented by SEQ ID NO: 2 includes a DNA having at least one nucleotide sequence selected from the group consisting of the nucleotide sequences from nt 16 to nt 60, nt 157 to nt 171 (or nt 154 to nt 171), nt 271 to nt 300, nt 319 to nt 342, nt 346 to nt 372, nt 376 to nt 396, nt 424 to nt 441, nt 484 to nt 510, nt 526 to nt 546, nt 550 to nt 567, nt 574 to nt 636, nt 640 to nt 654, and nt 678 to nt 714 (or nt 678 to nt 717) in the nucleotide sequence represented by SEQ ID NO: 7.

The DNA encoding the partial peptide having at least one amino acid sequence selected from the group consisting of the amino acid sequences from aa 6 to aa 20, aa 53 to aa 57 (or aa 52 to aa 57), aa 91 to aa 100, aa 107 to aa 114, aa I 16 to aa 124, aa 126 to aa 132, aa 142 to aa 147, aa 162 to aa 170, aa 176 to aa 182, aa 184 to aa 189, aa 192 to aa 212, aa 214 to aa 218, and aa 227 to aa 238 (or aa 227 to aa 239) in the amino acid sequence represented by SEQ ID NO: 3 includes a DNA having at least one nucleotide sequence selected from the group consisting of the nucleotide sequences from nt 16 to nt 60, nt 157 to nt 171 (or nt 154 to nt 171), nt 271 to nt 300, nt 319 to nt 342, nt 346 to nt 372, nt 376 to nt 396, nt 424 to nt 441, nt 484 to nt 510, nt 526 to nt 546, nt 550 to nt 567, nt 574 to nt 636, nt 640 to nt 654, and nt 678 to nt 714 (or nt 678 to nt 717) in the nucleotide sequence represented by SEQ ID NO: 10.

The DNA encoding the partial peptide having at least one amino acid sequence selected from the group consisting of the amino acid sequences from aa 8 to aa 21, aa 57 to aa 66, aa 73 to aa 80, aa 82 to aa 90, aa 92 to aa 98, aa 108 to aa 113, aa 126 to aa 134, aa 140 to aa 146, aa 148 to aa 153, aa 157 to aa 177, aa 179 to aa 183, and aa 192 to aa 203 in the amino acid sequence represented by SEQ ID NO: 31 includes a DNA having at least one nucleotide sequence selected from the group consisting of the nucleotide sequences from nt 22 to nt. 63, nt 169 to nt 198, nt 217 to nt 240, nt 244 to nt 270, nt 274 to nt 294, nt 322 to nt 339, nt 376 to nt 402, nt 418 to nt 438, nt 442 to nt 459, nt 469 to nt 531, nt 535 to nt 549, and nt 574 to nt 607 in the nucleotide sequence represented by SEQ ID NO: 30.

Further, the DNA encoding the partial peptide having the amino acid sequence from aa 84 to aa 240 in the amino acid sequence represented by SEQ ID NO: 1 includes (i) the DNA having the nucleotide sequence from nt 250 to nt 720 in the nucleotide sequence represented by SEQ ID NO: 4; and (ii) a DNA, which is hybridizable to the DNA having the nucleotide sequence from nt 250 to nt 720 in the nucleotide sequence represented by SEQ ID NO: 4 under high stringent conditions and encodes a partial peptide having an activity equivalent in property to that of the partial peptide having the amino acid sequence from aa 84 to aa 240 in the amino acid sequence represented by SEQ ID NO: 1 (e.g. caspase 3 inhibiting activity).

An example of the DNA that is hybridizable to the nucleotide sequence from nt 250 to nt 720 in the nucleotide sequence represented by SEQ ID NO: 4 under high stringent conditions includes a DNA comprising a nucleotide sequence having at least about 40% homology, preferably at least about 60% homology, more preferably at least about 80% homology, even more preferably at least about 90% homology, and most preferably at least about 95% homology to the nucleotide sequence from nt 250 to nt 720 in the nucleotide sequence represented by SEQ ID NO: 4.

The DNA encoding the partial peptide having the amino acid sequence from aa 82 to aa 239 in the amino acid sequence represented by SEQ ID NO: 2 includes (i) the DNA having the nucleotide sequence from nt 244 to nt 717 in the nucleotide sequence represented by SEQ ID NO: 7; and (ii) a DNA, which is hybridizable to the DNA having the nucleotide sequence from nt 244 to nt 717 in the nucleotide sequence represented by SEQ ID NO: 7 under high stringent conditions and encodes a partial peptide having an activity equivalent in property to that of the partial peptide having the amino acid sequence from aa 82 to aa 239 in the amino acid sequence represented by SEQ ID NO: 2 (e.g. caspase 3 inhibiting activity).

An example of the DNA that is hybridizable to the nucleotide sequence from nt 244 to nt 717 in the nucleotide sequence represented by SEQ ID NO: 7 under high stringent conditions includes a DNA comprising a nucleotide sequence having at least about 40% homology, preferably at least about 60% homology, more preferably at least about 80% homology, even more preferably at least about 90% homology, and most preferably at least about 95% homology to the nucleotide sequence from nt 244 to nt 717 in the nucleotide sequence represented by SEQ ID NO: 7.

The DNA encoding the partial peptide having the amino acid sequence from aa 82 to aa 239 in the amino acid sequence represented by SEQ ID NO: 3 includes (i) the DNA having the nucleotide sequence from nt 244 to nt 717 in the nucleotide sequence represented by SEQ ID NO: 10; and (ii) a DNA, which is hybridizable to the DNA having the nucleotide sequence from nt 244 to nt 717 in the nucleotide sequence represented by SEQ ID NO: 10 under high stringent conditions and encodes a partial peptide having an activity equivalent in property to that of the partial peptide having the amino acid sequence from aa 82 to aa 239 in the amino acid sequence represented by SEQ ID NO: 3 (e.g. caspase 3 inhibiting activity).

The DNA encoding the partial peptide having the amino acid sequence from aa 48 to aa 204 in the amino acid sequence represented by SEQ ID NO: 31 includes (i) the DNA having the nucleotide sequence from nt 142 to nt 612 in the nucleotide sequence represented by SEQ ID NO: 30; and (ii) a DNA, which is hybridizable to the DNA having the nucleotide sequence from nt 142 to nt 612 in the nucleotide sequence represented by SEQ ID NO: 30 under high stringent conditions and encodes a partial peptide having an activity equivalent in property to that of the partial peptide having the amino acid sequence from aa 48 to aa 204 in the amino acid sequence represented by SEQ ID NO: 31 (e.g. caspase 3 inhibiting activity).

An example of the DNA that is hybridizable to the nucleotide sequence from nt 244 to nt 717 in the nucleotide sequence represented by SEQ ID NO: 10 under high stringent conditions includes a DNA comprising a nucleotide sequence having at least about 40% homology, preferably at least about 60% homology, more preferably at least about 80% homology, even more preferably at least about 90% homology, and most preferably at least about 95% homology to the nucleotide sequence from nt 244 to nt 717 in the nucleotide sequence represented by SEQ ID NO: 10.

The hybridization methods and the high stringent conditions that can be used are the same as described above.

More specifically, the DNA encoding the partial peptide having the amino acid sequence from aa 84 to aa 240 in the amino acid sequence represented by SEQ ID NO: 1 includes the DNA having the nucleotide sequence from nt 250 to nt 720 in the nucleotide sequence represented by SEQ ID NO: 4. The DNA encoding the partial peptide having the amino acid sequence from aa 82 to aa 239 in the amino acid sequence represented by SEQ ID NO: 2 includes the DNA having the nucleotide sequence from nt 244 to nt 717 in the nucleotide sequence represented by SEQ ID NO: 7. The DNA encoding the partial peptide having the amino acid sequence from aa 82 to aa 239 in the amino acid sequence represented by SEQ ID NO: 3 includes the DNA having the nucleotide sequence from nt 244 to nt 717 in the nucleotide sequence represented by SEQ ID NO: 10. The DNA encoding the partial peptide having the amino acid sequence from aa 48 to aa 204 in the amino acid sequence represented by SEQ ID NO: 31 includes the DNA having the nucleotide sequence from nt 142 to nt 612 in the nucleotide sequence represented by SEQ ID NO: 30.

For cloning of the DNA encoding the protein of the invention or a partial peptide thereof, the desired DNA may be amplified from the aforementioned DNA libraries and the like by the PCR method using synthetic DNA primers having a part of the nucleotide sequence encoding the protein of the invention. Alternatively, DNAs incorporated into an appropriate vector may be selected for the desired DNA by hybridization with a labeled form of a DNA fragment or a synthetic DNA encoding a part or the entire region of the protein of the invention. The hybridization can be carried out according to the method described in, for example, "Molecular Cloning" (2nd Ed., J. Sambrook et al., Cold Spring Harbor Lab. Press, 1989). Commercially available libraries may be used according to their attached manufacturer's instructions.

The nucleotide substitution of the DNA can be carried out according to a known method such as the ODA-LA PCR method, the Gapped duplex method, the Kunkel method, or variations thereof, and using a known kit such as Mutan™-super Express Km (TaKaRa Shuzo Co., Ltd.) or Mutan™-K (TaKaRa Shuzo Co., Ltd.).

The thus cloned DNA encoding the protein of the invention or a partial peptide thereof can be used for any purpose as it is or if desired, after digestion with a restriction enzyme or after addition of a linker thereto. The DNA may contain ATG as a translation initiation codon at the 5' end and TAA, TGA or TAG as a translation termination codon at the 3' end. These translation initiation and termination codons may also be added by using an appropriate synthetic DNA adapter.

The expression vector for the protein of the invention or a partial peptide thereof can be produced, for example, by (a) excising the desired DNA fragment from a DNA, e.g. cDNA encoding the protein of the invention, (b) and then inserting the DNA fragment downstream of a promoter in an appropriate vector.

Examples of the vector to be used include plasmids derived form E. coli (e.g. pBR322, pBR325, pUC12, pUC13), plasmids derived from Bacillus subtilis (e.g. pUB 110, pTP5, pC194), plasmids derived from yeast (e.g. pSH19, pSH15), bacteriophages such as λ phage, animal viruses such as retrovirus, vaccinia virus, baculovirus, as well as pAl-11, pXT1, pRc/CMV, pRc/RSV, pcDNAI/Neo.

The promoter to be used in the invention may be any suitable one working in a host used for the gene expression. When animal cells are used as the host, SRα promoter, SV40 promoter, LTR promoter, CMV(cytomegalovirus) promoter, HSV-TK promoter can be used. Among them, CMV promoter or SRα promoter is preferably used. When Escherichia bacteria are used as the host, preferred are trp promoter, lac promoter, recA promoter, λPL promoter, lpp promoter; when Bacillus bacteria are used as the host, preferred are SPO1 promoter, SPO2 promoter, penP promoter; when yeasts are used as the host, preferred are AOX1 promoter, PHO5 promoter, PGK promoter, GAP promoter, ADH promoter; when insect cells are used as the host, preferred are polyhedrin promoter, and P10 promoter.

In addition to the foregoing, the expression vector may further optionally contain an enhancer, a splicing signal, a poly A signal, a selection marker, SV40 replication origin (hereinafter abbreviated as SV40ori), and the like. Examples of the selection marker include dihydrofolate reductase (hereinafter abbreviated as dhfr) gene, ampicillin resistant gene (hereinafter abbreviated as Amp^{r}), and neomycin resistant gene (hereinafter abbreviated as Neo^{r}, G418 resistance). Dhfr gene confers methotrexate (MTX) resistance, and Neo confers G418 resistance. In particular, when dhfr gene is used as the selection marker in CHO(dhfr⁻) cells, the selection may be made for the desired gene in thymidine-free medium.

If necessary, a signal sequence suitable for a host may be added to the N-terminal of the protein. For example, PhoA signal sequence, OmpA signal sequence in Escherichia bacteria as the host; α-amylase signal sequence, subtilisin signal sequence in Bacillus bacteria as the host; MFα signal sequence, SUC2 signal sequence in yeasts as the host; and insulin signal sequence, α-interferon signal sequence, antibody molecule signal sequence in animal cells as the host can be used respectively.

A cell can be transfected with the vector comprising the DNA encoding the protein as constructed in this way to produce a transforinant.

For example, Escherichia bacteria, Bacillus bacteria, yeasts, insect cells, insects and animal cells can be used as the host.

Examples of Escherichia bacteria include Escherichia coli K12 DH1 (Proc. Natl.Acad. Sci. U.S.A. 60, 160, 1968), JM 103 (Nucleic Acids Research 9, 309, 1981), JA221 (Journal of Molecular Biology 120, 517, 1978), HB101 (Journal of Molecular Biology 41, 459, 1969), C600 (Genetics 39, 440, 1954).

Examples of Bacillus bacteria include Bacillus subtilis MI114 (Gene, 24, 255, 1983), 207-21 (Journal of Biochemistry 95, 87, 1984).

Examples of yeasts include Saccharomyces cereviseae AH22, AH22R⁻, NA87-11A, DKD-5D, 20B-12, Schizosaccharomyces pombe NCYC1913, NCYC2036, Pichia pastoris KM71.

Examples of insect cells include, for the virus AcNPV, Spodoptera frugiperda cell (Sf cell), MG1 cell derived from mid-intestine of Trichoplusia ni, High Five™ cell derived from egg of Trichoplusia ni, cells derived from Mamestra brassicae, cells derived from Estigmena acrea; and for the virus BmNPV, Bombyx mori N cell (BmN cell). Examples of the Sf cell include Sf9 cell (ATCC CRL 1711) and Sf21 cell (Vauglm, J. L. et al., In Vitro 13, 213-217, 1977).

Examples of insects include a larva of Bombyx mori (Maeda et al., Nature 315, 592, 1985).

Examples of animal cells include monkey COS-7 cell, Vero cell, Chinese hamster cell (CHO cell), dhfr gene-deficient Chinese hamster cell (CHO(dhfr⁻) cell), mouse L cell, mouse AtT-20 cell, mouse myeloma cell, rat GH3, human FL cell, 293 cell, C 127 cell, BALB3T3 cell, Sp-2 cell. Among them preferred are CHO cell, CHO(dhfr⁻) cell, and 293 cell.

Escherichia bacteria can be transformed, for example, by the method described in Proc. Natl. Acad. Sci. U.S.A. 69, 2110 (1972), or Gene 17, 107 (1982).

Bacillus bacteria can be transformed, for example, by the method described in Molecular & General Genetics 168, 111 (1979).

Yeasts can be transformed, for example, by the method described in "Methods in Enzymology" 194, 182-187 (1991) or Proc. Natl. Acad. Sci. U.S.A. 75, 1929 (1978).

Insect cells or insects can be transformed, for example, according to the method described in Bio/Technology 6, 47-55 (1988).

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by *Shujunsha;* or Virology 52, 456 (1973).

To introduce the expression vector into cells, for example, the calcium phosphate method (Graham, F.L. and van der Eb, A.J., Virology 52, 456-467, 1973) or the electroporation method (Nuemann, E. et al., EMBO. J. 1, 841-845, 1982) can be used.

In this way, a transformant, which is transformed with the expression vector comprising the DNA encoding the protein of the invention, can be obtained.

To express the protein of the invention in a stable manner using animal cells, the animal cell clone can be selected, which incorporates the introduced expression vector into the chromosome. To be specific, using the above selection marker as an index, such a transformant can be selected. It is also possible to obtain a stable animal cell strain highly expressing the protein of the invention by repeating the clonal selection of animal cells once obtained using the selection marker. In addition, when using dhfr gene as a selection marker, an animal cell strain showing much higher expression can be obtained by culturing transformed cells in gradually increasing concentrations of MTX; and selecting an MTX-resistant cell strain to amplify the DNA encoding the protein as well as dhfr gene in the cell.

The protein of the invention or a partial peptide thereof can be produced by culturing the above-mentioned transformant under condition allowing the expression of the DNA encoding the protein or the partial peptide; and producing and accumulating the protein or the partial peptide.

When an Escherichia or Bacillus bacterium is used as the host, the transformant can be appropriately cultured in a liquid medium, which contains materials required for the transformant growth such as carbon sources, nitrogen sources, and inorganic materials. Examples of the carbon sources include glucose, dextrin, soluble starch, and sucrose. Examples of the nitrogen sources include inorganic or organic materials such as ammonium salts, nitrates, corn steep liquor, peptone, casein, meat extract, soybean cake, and potato extract. Examples of the inorganic materials include calcium chloride, sodium dihydrogenphosphate, and magnesium chloride. In addition, yeast extracts, vitamins, and growth-stimulating factors may also be added. Desirably, the medium has a pH of about 5 to 8.

A preferred example of the medium for culturing Escherichia bacteria is M9 medium containing glucose and casamino acids (Miller, Journal of Experiments in Molecular Genetics, 431-433, Cold Spring Harbor Laboratory, New York, 1972). If necessary, a chemical such as 3β-indolylacry]ic acid can be added to the medium thereby to increase the promoter efficiency.

When an Escherichia bacterium is used as the host, the transformant is usually cultured at about 15 to 43°C for about 3 to 24 hours. If necessary, the culture may be aerated or agitated.

When a Bacillus bacterium is used as the host, the transformant is cultured generally at about 30 to 40°C for about 6 to 24 hours. If necessary, the culture can be aerated or agitated.

When a yeast is used as the host, the medium for culturing the transformant may be Burkholder's minimal medium (Bostian, K. L. et al., Proc. Natl. Acad. Sci. U.S.A. 77, 4505, 1980) or SD medium containing 0.5% Casamino acids (Bitter, G. A. et al., Proc. Natl. Acad. Sci. U.S.A. 81, 5330, 1984). Preferably, pH of the medium is adjusted to about 5 to 8. In general, the transformant is cultured at about 20 to 35°C for about 24 to 72 hours. If necessary, the culture can be aerated or agitated.

When an insect cell is used as the host, the medium for culturing the transformant may be Grace's Insect Medium (Grace, T.C.C., Nature 195, 788, 1962) supplemented with an appropriate additive such as 10% inactivated bovine serum. Preferably, pH of the medium is adjusted to about 6.2 to 6.4. Generally, the transformant is cultured at about 27 °C for about 3 to 5 days and, if necessary, the culture can be aerated or agitated.

When an animal cell is used as the host, the medium for culturing the transformant may be MEM medium (Science 122, 501, 1952), DMEM medium (Virology 8, 396, 1959), RPMI 1640 medium (The Journal of the American Medical Association 199, 519, 1967) or 199 medium (Proceeding of the Society for the Biological Medicine 73, 1, 1950), which contain about 5 to 20 % fetal bovine serum. Preferably, pH of the medium is about 6 to 8. The transformant is usually cultured at about 30 to 40 °C for about 15 to 72 hours and, if necessary, the culture can be aerated or agitated.

In particular, when using dhfr gene as a selection marker in CHO (dhfr⁻) cells, preferably used is DMEM medium containing dialyzed fetal bovine serum and almost no thymidine.

The protein of the invention can be isolated or purified from the culture described above by the following procedures.

To extract the protein of the invention from the culture of bacteria or cells, after the culture is completed, the bacteria or cells are collected by a known method and suspended in an appropriate buffer. The bacteria or cells are disrupted by a known method such as ultrasonication, lysozyme treatment and/or freeze-thaw cycling, and then subjected to the centrifugation or filtration to obtain the crude peptide extract. The buffer may contain a protein denaturing agent such as urea or guanidine hydrochloride, or a surfactant such as Triton X-100™.

When the protein is secreted into the culture broth, after the culture is completed, the supernatant can be separated and collected from bacteria or cells by a known method.

The protein of the invention contained in the supernatant or the extract thus obtained can be purified by an appropriate combination of known isolation or purification methods. Such isolation or purification methods include a method utilizing difference in solubility such as salting out, solvent precipitation; a method utilizing difference mainly in molecular weight such as dialysis, ultrafiltration, gel filtration, SDS-polyacrylamide gel electrophoresis; a method utilizing difference in electric charge such as ion exchange chromatography; a method utilizing specific affinity such as affinity chromatography; a method utilizing difference in hydrophobicity such as reversed phase high performance liquid chromatography; a method utilizing difference in isoelectric point such as isoelectrofocusing; and the like.

When the protein of the invention thus obtained is in a free form, it can be converted into a salt form by a known method or a variation thereof. On the other hand, when the protein is obtained in a salt form, it can be converted into the free form or a different salt form by a known method or a variation thereof.

The protein of the invention produced by the transformant can be treated, before or after the purification, with an appropriate protein-modifying enzyme so that the protein can be appropriately modified or deprived of a partial peptide. Examples of the protein-modifying enzyme include trypsin, chymotrypsin, arginyl endopeptidase, protein kinase, glycosidase and the like.

The thus produced protein of the invention can be detected by an enzyme immunoassay using a specific antibody.

An antibody to the protein of the invention, a partial peptide or a salt thereof, may be any polyclonal or monoclonal antibody, which are capable of recognizing the protein, the partial peptide or a salt thereof.

The antibody to the protein of the invention (hereinafter occasionally referred to as the antibody of the invention) may be produced by a known method for producing antibodies or antisera, using as an antigen the protein of the invention.

### [Preparation of monoclonal antibody]

### (a) Preparation of monoclonal antibody-producing cells

The protein of the invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once every two to six weeks and two to ten times in total. Examples of the applicable warm-blooded animals are monkeys, rabbits, dogs, guinea pigs, mice, rats, sheep, goats and chickens, with the use of mice and rats being preferred.

In the preparation of monoclonal antibody-producing cells, a warm-blooded animal, e.g., mouse, immunized with an antigen wherein the antibody titer is noted is selected, then spleen or lymph node is collected after two to five days from the final immunization and antibody-producing cells contained therein are fused with myeloma cells from homozoic or heterozoic animal to give monoclonal antibody-producing hybridomas. Measurement of the antibody titer in antisera may be carried out, for example, by reacting a labeled protein, which will be described later, with the antiserum followed by assaying the binding activity of the labeling agent bound to the antibody. The fusion may be carried out, for example, by the known method by Koehler and Milstein [Nature 256, 495 (1975)]. Examples of the fusion promoter are polyethylene glycol (PEG) and Sendai virus, and preferably PEG.

Examples of the myeloma cells are those collected from warm-blooded animals such as NS-1, P3U1, SP2/0, AP-1. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by culturing at 20 to 40°C, preferably at 30 to 37°C for 1 to 10 minutes, an efficient cell fusion can be carried out.

Various methods can be used for screening of a monoclonal antibody-producing hybridoma. Examples of such methods include a method which comprises adding the supernatant of hybridoma to a solid phase (e.g. microplate) adsorbed with the protein antigen directly or together with a carrier, adding an anti-immunoglobulin antibody (if mouse cells are used for the cell fusion, anti-mouse immunoglobulin antibody is used) labeled with a radioactive substance or an enzyme or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase.

The monoclonal antibody can be selected according to publicly known methods or their modifications. In general, the selection can be effected in a medium for animal cells supplemented with HAT (hypoxanthine, aminopterin and thymidine). Any selection and growth medium can be employed as far as the hybridoma can grow there. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at 37°C, for about 5 days to about 3 weeks, preferably I to 2 weeks, normally in 5% CO₂. The antibody titer of the culture supernatant of a hybridoma can be determined as in the assay for the antibody titer in antisera described above.

### (b) Purification of monoclonal antibody

Separation and purification of a monoclonal antibody can be carried out by known methods, such as methods for separation and purification of immunoglobulins, for example, salting-out, alcohol precipitation, isoelectric point precipitation, electrophoresis, adsorption and desorption with ion exchangers (e.g. DEAE), ultracentrifugation, gel filtration, or a specific purification method which comprises collecting only an antibody with an activated adsorbent such as an antigen-binding solid phase, Protein A or Protein G, and dissociating the binding to obtain the antibody.

### [Preparation of polyclonal antibody]

The polyclonal antibody of the invention can be manufactured by publicly known methods or modifications thereof. For example, a warm-blooded animal is immunized with an immunogen (the protein antigen) per se, or a complex of immunogen and a carrier protein is formed and a warm-blooded animal is immunized with the complex in a manner similar to the method described above for the manufacture of monoclonal antibodies. The product containing the antibody to the protein of the invention is collected from the immunized animal followed by separation and purification of the antibody.

In the complex of immunogen and carrier protein used to immunize a warm-blooded animal, the type of carrier protein and the mixing ratio of carrier to hapten may be any type and in any ratio, as long as the antibody is efficiently produced to the hapten immunized by crosslinking to the carrier. For example, bovine serum albumin, bovine thyroglobulin or hemocyanin is coupled to hapten in a carrier-to-hapten weight ratio of approximately 0.1 to 20, preferably about 1 to about 5.

A variety of condensation agents can be used for the coupling of carrier to hapten. Glutaraldehyde, carbodiimide, maleimide-activated ester, activated ester reagents containing thiol group or dithiopyridyl group, and others are used for the coupling.

The condensation product is administered to warm-blooded animals either solely or together with carriers or diluents to the site that can produce the antibody by the administration. In order to potentiate the antibody productivity upon the administration, complete Freund's adjuvant or incomplete Freund's adjuvant may be administered. The administration is usually made once every about 2 to 6 weeks about 3 to 10 times in total.

The polyclonal antibody can be collected from the blood, ascites, etc., preferably from the blood of warm-blooded animals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as in the separation and purification of monoclonal antibodies described above.

The antisense DNA having a nucleotide sequence substantially complementary to the DNA or mRNA encoding the protein or the partial peptide of the invention may be any oligonucleotides or derivatives thereof, which have a nucleotide sequence substantially complementary to the DNA or mRNA sequence encoding the protein or the partial peptide or to a partial sequence thereof, and also have an activity to inhibit the expression of the protein or the partial peptide.

The nucleotide sequence substantially complementary to the DNA or mRNA includes a nucleotide sequence having at least about 40%, preferably at least about 60%, more preferably at least about 80%, and even more preferably at least about 90% homology to the whole or a part of the nucleotide sequence complementary to the DNA or mRNA (i.e. a complementary strand of the DNA or mRNA). Particularly preferred is an antisense DNA having at least about 40%, preferably at least about 60%, more preferably at least about 80%, and even more preferably at least about 90% homology to the complementary strand of the partial nucleotide sequence encoding the N-terminal region of the protein of the invention (e.g. the partial nucleotide sequence around an initiation codon) in the complementary strand of the whole nucleotide sequence of the DNA or mRNA of the invention.

The protein of the invention, a partial peptide or a salt thereof has an activity such as the caspase 3 inhibiting activity, and thus can be used for any applications based on the above activity.

Hereinafter, utilities of the protein of the invention, a partial peptide or a salt thereof (sometimes referred to as the protein of the invention); the DNA encoding the protein of the invention or the partial peptide (sometimes referred to as the DNA of the invention), and the antibody to the protein of the invention or the partial peptide (sometimes referred to as the antibody of the invention); and the antisense DNA are described.

### [1] Pharmaceuticals such as a prophylactic and/or therapeutic agent for various diseases based on the caspase 3 inhibiting activity

The caspase 3 inhibiting activity refers to the activity to inhibit the activation of caspase 3, a protease which is activated through the partial cleavage thereof on the induction of cell death and degrades a variety of intracellular proteins to conduct the cell death. Thus, the protein and the DNA of the invention show the activity to inhibit the processing from the precursor of caspase 3 to the activated form of caspase 3.

For example, for a patient who does not show a sufficient nor normal level of the caspase 3 inhibiting activity because of decrease or deficiency in the protein of the invention in vivo, the function of the protein can be restored to a sufficient or normal level by such means as (a) administering the DNA of the invention to the patient to express the protein of the invention in the body; (b) incorporating the DNA of the invention into cells to express the protein of the invention, and then transplanting said cells to the patient; and (c) administering the protein of the invention to the patient.

Therefore, the protein and the DNA of the invention can be used as a caspase 3 inhibitor and thus are useful as a pharmaceutical such as a prophylactic and/or therapeutic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.

When the DNA of the invention is used as said prophylactic and/or therapeutic agent, the DNA can be administered to a human or a warm-blooded animal in a conventional manner as it is or after inserted into a proper vector such as retrovirus vector, adenovirus vector, adenovirus-associated virus vector. The DNA can be administered using a gene gun or a catheter such as a hydrogel catheter, as it is or as a formulation with a physiologically acceptable carrier such as an auxiliary material for increasing uptake.

When the protein of the invention is used as said prophylactic and/or therapeutic agent, it is preferred to use it at a purity of at least 90%, preferably at least 95%, more preferably at least 98%, and even more preferably at least 99%.

When the protein is used as said pharmaceutical, for example, it can be used orally in a form of tablet, optionally sugar-coated, capsule, elixir or microcapsule; or parenterally in a form of injection such as a sterile solution and suspension in water or other pharmaceutically acceptable liquid. These formulations can be produced by mixing the protein of the invention with a physiologically acceptable carrier, a flavoring agent, an excipient, a vehicle, an antiseptic agent, a stabilizer, a binder, and the like in a unit dosage form required for a generally accepted pharmaceutical practice. The amount of the active ingredient in the formulation is adjusted appropriately within the specified range. When the DNA of the invention is used as said pharmaceutical, the DNA can be administered in a conventional manner as it is or after inserted into a proper vector such as retrovirus vector, adenovirus vector, adenovirus-associated virus vector.

Additives miscible in a tablet, a capsule, etc. include a binder such as gelatin, corn starch, tragacanth gum, and gum arabic; an excipient such as crystalline cellulose; a swelling agent such as corn starch, gelatin and alginic acid; a lubricant such as magnesium stearate; a sweetening agent such as sucrose, lactose and saccharin; and a flavoring agent such as peppermint, akamono oil and cherry. When the unit dosage form is a capsule, liquid carriers such as oils and fats may further be used together with the additives described above. An injectable sterile composition may be formulated by conventional procedures, for example, by dissolving or suspending the active ingredient in a vehicle such as water for injection with a naturally occurring vegetable oil such as sesame oil, coconut oil.

Examples of an aqueous medium for injection include a physiological saline and an isotonic solution containing glucose and other auxiliary agents (e.g. D-sorbitol, D-mannitol, sodium chloride), which may be used in combination with an appropriate dissolution aid such as an alcohol (e.g. ethanol), a polyalcohol (e.g. propylene glycol, polyethylene glycol), a nonionic surfactant (e.g. polysorbate 80™, HCO-50). Examples of the oily medium include sesame oil and soybean oil, which may be used in combination with a dissolution aid such as benzyl benzoate and benzyl alcohol.

The composition may further contain a buffer (e.g. phosphate buffer, sodium acetate buffer), a soothing agent (e.g. benzalkonium chloride, procaine hydrochloride), a stabilizer (e.g. human serum albumin, polyethylene glycol), a preservative (e.g. benzyl alcohol, phenol), an antioxidant, and the like. An appropriate ampoule is usually filled with the thus prepared injection liquid.

The vector into which the DNA of the invention is incorporated is also formulated in the same manner as described above and are usually administered parenterally.

Since the thus obtained pharmaceutical formulation is safe and low toxic, it can be administered to a mammal (e.g. human, rat, mouse, guinea pig, rabbit, sheep, swine, bovine, horse, cat, dog, monkey).

The dose of the protein of the invention varies depending on the target disease, the subject, the route of administration, and etc. For example, when the protein of the invention is administered orally as a remedy for aplastic anemia, the daily dose for an adult (as 60 kg body weight) is normally about 0.1-100 mg, preferably about 1.0-50 mg, more preferably about 1.0-20 mg. When the protein is administered parenterally, the single dose also varies depending on the subject, the target disease, etc. For example, when the protein of the invention is administered in a injection form as a remedy for aplastic anemia to an adult (as 60 kg body weight), it is advantageous to inject the protein to the patient in a daily dose of about 0.01-30 mg, preferably about 0.1-20 mg, more preferably about 0.1-10 mg. For other animals, the corresponding dose as converted per 60 kg body weight can be administered.

### [2] Genetic diagnosis agents

An abnormality of the DNA encoding the protein of the invention (gene abnormality) can be detected in a mammal (e.g. human, rat, mouse, guinea pig, rabbit, sheep, swine, bovine, horse, cat, dog, monkey) by using the DNA of the invention as a probe. Therefore, the DNA of the invention is useful as a genetic diagnosis agent for various diseases associated with the protein of the invention.

For example, when a damage or defect of the DNA or mRNA encoding the protein of the invention or an decreased expression of the protein is detected in a subject, indicating the insufficiency of the caspase 3 inhibiting activity, the subject can be diagnosed as suffering from diseases such as AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.

The genetic diagnosis described above using the DNA of the invention can be carried out by, for example, the well-known northern hybridization assay or the PCR-SSCP assay (Genomics 5, 874-879 (1989); Proceedings of the National Academy of Sciences of the United States of America 86, 2766-2770 (1989)).

For example, when a decreased expression of the mRNA is detected in a subject by the northern hybridization assay, indicating the insufficiency of the caspase 3 inhibiting activity, the subject can be diagnosed as having or likely to have in the future diseases such as AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.

### [3] Diagnosis of various diseases based on the caspase 3 inhibiting activity of the protein of the invention by quantifying the protein, a partial peptide or a salt thereof

The antibody of the invention is capable of specifically recognizing the protein of the invention and thus can be used for conducting diagnosis of various diseases based on the caspase 3 inhibiting activity of the protein of the invention by quantifying the protein of the invention in a test liquid sample, in particular, quantifying by sandwich immunoassay.

The method of quantifying the protein of the invention includes:
(i) a method of quantifying the protein of the invention in a test liquid sample, which comprises competitively reacting the antibody of the invention with the test liquid sample and a labeled form of the protein of the invention, and measuring the ratio of the labeled protein of the invention; and,
(ii) a method of quantifying the protein of the invention in a test liquid sample, which comprises reacting the test liquid sample simultaneously or sequentially with the antibody of the invention immobilized on a carrier and a labeled form of another antibody of the invention, and then measuring the activity of the label on the immobilizing carrier.

In the quantification method (ii) described above, it is preferred that one antibody is capable of recognizing the N-terminal region of the protein of the invention, while another antibody is capable of reacting the C-terminal region of the protein of the invention.

The monoclonal antibody to the protein of the invention (sometimes referred to as the monoclonal antibody) can be used to quantify the protein of the invention. Moreover, the protein of the invention can be detected by a tissue staining method as well. For these purposes, the antibody molecule per se may be used or F(ab')₂, Fab' or Fab fractions of the antibody molecule may also be used.

There is no particular limitation for the type of quantification method using the antibody to the protein of the invention, and any assay methods can be used whereby the amount of antibody, antigen, or antibody-antigen complex corresponding to the amount of antigen (e.g., the amount of the protein) in the test liquid can be detected by chemical or physical means and the amount of the antigen can be calculated from a standard curve prepared from standard solutions containing known amounts of the antigen. Advantageously used are, for example, nephrometry, competitive method, immunometric method and sandwich method. In terms of sensitivity and specificity, the sandwich method, which will be described later, is particularly preferred.

Examples of the labeling agent used in the assay using the labeled substance include radioisotopes, enzymes, fluorescent substances and luminescent substances. Examples of the radioisotope include [¹²⁵I], [¹³¹I], [³H] and [¹⁴C]. Examples of the enzyme include preferably a stable enzyme having a high specific activity, such as β-galactosidase, β-glucosidase, alkaline phosphatase, peroxidase, and malate dehydrogenase. Examples of the fluorescent substance include fluorescamine and fluorescein isothiocyanate. Examples of the luminescent substance include luminol, luminol derivatives, luciferin, and lucigenin. Furthermore, the biotin-avidin system may also be used for coupling of an antibody or antigen to a labeling agent.

For the immobilizatio of antigens or antibodies, physical adsorption may be used. Alternatively, chemical binding that is conventionally used for immobilization of proteins or enzymes may be used as well. Examples of the carrier include insoluble polysaccharides such as agarose, dextran and cellulose; synthetic resins such as polystyrene, polyacrylamide, silicone; or glass.

In the sandwich method, a test liquid sample is reacted with an immobilized monoclonal antibody of the invention (primary reaction), then reacted with another labeled monoclonal antibody of the invention (secondary reaction) and the activity of the label on the immobilizing carrier is assayed, whereby the amount of the protein of the invention in the test liquid sample can be quantified. The primary and secondary reactions may be carried out in a reversed order, simultaneously or sequentially with an interval. The type of the labeling agent and the method for immobilization may be the same as those described above. In the immunoassay by the sandwich method, it is not always necessary that one type of the antibody is used for the immobilized or labeled antibody, but a mixture of two or more antibodies may also be used for the purpose of improving the measurement sensitivity.

In the quantification of the protein of the invention by the sandwich method, it is preferred that the monoclonal antibodies of the invention used for the primary and secondary reactions have different binding sites on the protein of the invention, respectively. Thus, in respect to the antibodies used in the primary and secondary reactions, for example, when the antibody used in the secondary reaction recognizes the C-terminal region of the protein of the invention, the antibody used in the primary reaction preferably recognize a region other than the C-terminal region, for example, the N-terminal region.

The monoclonal antibody of the invention may be used in an assay system other than the sandwich method, such as a competitive method, an immunometric method, a nephrometry.

In the competitive method, an antigen in a test liquid sample and a labeled antigen are competitively reacted with an antibody, then the unreacted labeled antigen (F) and the labeled antigen bound to the antibody (B) are separated (i.e. B/F separation), and the amount of the label present in either B or F is measured to determine the amount of the antigen in the test liquid sample. In the reactions for such a method, there are a liquid phase method in which a soluble antibody is used as the antibody and the B/F separation is effected by polyethylene glycol and a second antibody to the antibody, and a solid phase method in which an immobilized antibody is used as the first antibody or a soluble antibody is used as the first antibody while an immobilized antibody is used as the second antibody.

In the immunometric method, an antigen in a test liquid sample and an immobilized antigen are competitively reacted with a given amount of the labeled antibody, followed by separating the solid phase from the liquid phase; or an antigen in a test liquid sample and an excess amount of the labeled antibody are reacted, then an immobilized antigen is added to bind the unreacted labeled antibody to the solid phase and the solid phase is separated from the liquid phase. Thereafter, the amount of the label in either of the phases is measured to determine the antigen amount in the test liquid sample.

In the nephrometry, the amount of insoluble sediment, which is produced as a result of the antigen-antibody reaction in a gel or in a solution, is measured. Even when the amount of an antigen in a test liquid sample is small and only a small amount of the sediment is obtained, a laser nephrometry utilizing laser scattering can be suitably used.

For applying these immunological methods to the quantification method of the invention, any special conditions or procedures are not required. A system for quantifying the protein of the invention may be constructed according to the combination of the usual technical consideration in the art and the conventional conditions and procedures. For the details of these general technical means, reference can be made to any reviews and textbooks.

For example, see Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by lgaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immunochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies))(all published by Academic Press).

As described above, the protein of the invention can be quantified with high sensitivity, using the antibody of the invention.

These quantification methods as described above can be used to conduct diagnosis of various diseases associated with the protein of the invention.

For example, when a decreased concentration of the protein of the invention is detected in a subject, indicating the insufficiency of the caspase 3 inhibiting activity, the subject can be diagnosed as having or likely to have in the future diseases such as AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.

### [4] Pharmaceuticals containing the antisense DNA

The antisense DNA capable of binding complementally to the DNA of the invention and suppressing the expression of the DNA can inhibit in vivo the function of the protein or the DNA of the invention and thus can be used as, for example, the prophylactic and therapeutic agent for diseases caused by overexpression of the protein of the invention.

When used as the prophylactic and therapeutic agent as described above, the antisense DNA can be used in a similar manner to the prophylactic and therapeutic agent for various diseases comprising the DNA of the invention as described above.

For example, when applicable, the antisense DNA can be administered in a conventional manner, as it is or after inserted into a proper vector such as retrovirus vector, adenovirus vector, adenovirus-associated virus vector. The antisense DNA can be administered using a gene gun or a catheter such as a hydrogel catheter, as it is or as a formulation with a physiologically acceptable carrier such as an auxiliary material for increasing uptake.

Further, the antisense DNA can be used as a diagnostic oligonucleotide probe to examine the existence or expression profile of the DNA of the invention in tissues or cells.

In the specification and drawings, bases, amino acids, and the like are abbreviated in accordance with the IUPAC-IUB Commission on Biochemical Nomenclature or the conventional usage in the art, as shown below for example. When an amino acid has optical isomers, its L form is selected unless otherwise indicated.

| | |
|---|---|
| DNA | deoxyribonucleic acid |
| cDNA | complementary deoxyribonucleic acid |
| A | adenine |
| T | thymine |
| G | guanine |
| C | cytosine |
| RNA | ribonucleic acid |
| mRNA | messenger ribonucleic acid |
| dATP | deoxyadenosine triphosphate |
| dTTP | deoxythymidine triphosphate |
| dGTP | deoxyguanosine triphosphate |
| dCTP | deoxycytidine triphosphate |
| ATP | adenosine triphosphate |
| EDTA | ethylenediaminetetra acetic acid |
| SDS | sodium dodecyl sulfate |
| Gly | glycine |
| Ala | alanine |
| Val | valine |
| Leu | leucine |
| Ile | isoleucine |
| Ser | serine |
| Thr | threonine |
| Cys | cysteine |
| Met | methionine |
| Glu | glutamic acid |
| Asp | aspautic acid |
| Lys | lysine |
| Arg | arginine |
| His | histidine |
| Phe | phenylalanine |
| Tyr | tyrosine |
| Trp | tryptophan |
| Pro | proline |
| Asn | asparagine |
| Gin | glutamine |
| pGlu | pyroglutamic acid |

Substituents, protecting groups and reagents used often in this specification are shown by the following codes.

| | |
|---|---|
| Me | methyl |
| Et | ethyl |
| Bu | butyl |
| Ph | phenyl |
| TC | thiazolidine-4(R)-carboxamido |
| Tos | p-toluenesulfonyl |
| CHO | formyl |
| Bzl | benzyl |
| Cl₂-Bzl | 2,6-dichlorobenzyl |
| Bom | benzyloxymethyl |
| Z | benzyloxycarbonyl |
| Cl-Z | 2-chlorobenzyloxycarbonyl |
| Br-Z | 2-bromobenzyl oxycarbonyl |
| Boc | t-butoxycarbonyl |
| DNP | dinitrophenol |
| Trt | trityl |
| Bum | t-butoxymethyl |
| Fmoc | N-9-fluorenyl methoxycarbonyl |
| HOBt | 1-hydroxybenztriazole |
| HOOBt | 3,4-dihydro-3-hydroxy-4-oxo-1,2,3-benzotriazine |
| HONB | 1-hydroxy-5-norbornene-2,3-dicarboxyimide |
| DCC | N,N'-dichlorohexylcarbodiimide |

SEQ ID NOs in Sequence Listing in the specification indicate the following sequences, respectively.

### [SEQ ID NO:1]

This shows the whole amino acid sequence of the human-derived protein of the invention.

### [SEQ ID NO: 2]

This shows the whole amino acid sequence of the mouse-derived protein of the invention.

### [SEQ ID NO: 3]

This shows the whole amino acid sequence of the rat-derived protein of the invention.

### [SEQ ID NO: 4]

This shows the base sequence of cDNA encoding the human-derived protein of the invention having the amino acid sequence of SEQ ID NO: 1.

### [SEQ ID NO: 5]

This shows the base sequence of DNA comprising the cDNA encoding the human-derived protein of the invention having the amino acid sequence of SEQ ID NO: 1, as inserted into plasmid pTB1939.

### [SEQ ID NO: 6]

This shows the base sequence of DNA comprising the cDNA encoding the human-derived protein of the invention having the amino acid sequence of SEQ ID NO: 1, as inserted into plasmid pTB 1940.

### [SEQ ID NO: 7]

This shows the base sequence of cDNA encoding the mouse-derived protein of the invention having the amino acid sequence of SEQ ID NO: 2.

### [SEQ ID NO: 8]

This shows the base sequence of DNA comprising the cDNA encoding the mouse-derived protein of the invention having the amino acid sequence of SEQ ID NO: 2, as inserted into plasmid pTB1958.

### [SEQ ID NO: 9]

This shows the base sequence of genome DNA encoding the mouse-derived protein of the invention having the amino acid sequence of SEQ ID NO: 2.

### [SEQ ID NO: 10]

This shows the base sequence of cDNA encoding the rat-derived protein of the invention having the amino acid sequence of SEQ ID NO: 3.

### [SEQ ID NO: 11]

This shows the base sequence of a synthetic oligonucleotide used for the cloning of DNA encoding the human-derived protein of the invention.

### [SEQ lD NO: 12]

This shows the base sequence of a primer used for the cloning of DNA encoding the human-derived protein of the invention.

### [SEQ ID NO: 13]

This shows the base sequence of a primer used for the cloning of DNA encoding the human-derived protein of the invention.

### [SEQ ID NO: 14]

This shows the base sequence of an oligonucleotide used for the cloning of DNA encoding the mouse-derived protein of the invention.

### [SEQ ID NO: 15]

This shows the base sequence of an oligonucleotide used for the cloning of DNA encoding the mouse-derived protein of the invention.

### [SEQ ID NO: 16]

This shows the base sequence of a synthetic oligonucleotide used for the analysis of the base sequence around the start codon in the DNA encoding the mouse-derived protein of the invention.

### [SEQ ID NO: 17]

This shows the base sequence of a synthetic oligonucleotide used for the analysis of the base sequence around the start codon in the DNA encoding the mouse-derived protein of the invention.

### [SEQ ID NO: 18]

This shows the base sequence of an adaptor linked to both terminals of mouse chromosomal DNA fragments used for the analysis of the base sequence around the start codon in the DNA encoding the mouse-derived protein of the invention.

### [SEQ ID NO: 19]

This shows the base sequence of a synthetic oligonucleotide used for the analysis of the base sequence around the start codon in the DNA encoding the mouse-derived protein of the invention.

### [SEQ ID NO: 20]

This shows the base sequence of a synthetic oligonucleotide used for the analysis of the base sequence around the start codon in the DNA encoding the mouse-derived protein of the invention.

### [SEQ ID NO: 21]

This shows the base sequence of a primer used for the cloning of DNA encoding the extracellular region of the human-derived protein of the invention.

### [SEQ ID NO: 22]

This shows the base sequence of a primer used for the cloning of DNA encoding the extracellular region of the human-derived protein of the invention.

### [SEQ ID NO: 23]

This shows the base sequence of a primer used for the cloning of DNA encoding the rat-derived protein of the invention.

### [SEQ ID NO:24]

This shows the base sequence of a primer used for the cloning of DNA encoding the rat-derived protein of the invention.

### [SEQ ID NO: 25]

This shows the general formula (I) of the amino acid sequence of the protein of the invention.

### [SEQ ID NO: 26]

This shows the base sequence of a primer used in Reference Example 6.

### [SEQ ID NO: 27]

This shows the base sequence of a primer used in Reference Example 6.

### [SEQ ID NO: 28]

This shows the base sequence of primer 1 used in Reference Example 7.

### [SEQ ID NO: 29]

This shows the base sequence of primer 2 used in Reference Example 7.

### [SEQ ID NO: 30]

This shows the base sequence of 612 by cDNA obtained in Ref. Example 7.

### [SEQ ID NO: 31]

This shows the amino acid sequence of hTL4-2 obtained in Ref. Example 7.

### [SEQ ID NO: 32]

This shows the general formula (II) of the amino acid sequence of the protein of the invention.

### [SEQ ID NO: 33]

This shows the base sequence of a primer used in Reference Example 8.

### [SEQ ID NO: 34]

This shows the base sequence of a primer used in Reference Example 8.

The transformants Escherichia coli DH10B/pTB1939 and Escherichia coli DH10B/pTB1940, obtained in Reference Example 1 is deposited at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Numbers FERM BP-5595 and FERM BP-5596 respectively since July 17, 1996; and at Institute for Fermentation, Osaka (IFO), located at 2-17-85 Jyuso-Honmati, Yodogawa-ku, Osaka-shi, Osaka 532-8686, Japan, under Accession Numbers IFO 15997 and IFO 15998 respectively since July 11, 1996.

The transformants Escherichia coli DH5α/pTB1958 obtained in Reference Example 2 is deposited at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Numbers FERM BP-5805 since January 30, 1997; and at Institute for Fermentation, Osaka (IFO), located at 2-17-85 Jyuso-Honmati, Yodogawa-ku, Osaka-shi, Osaka 532-8686, Japan, under Accession Numbers IFO 16054 since January 31, 1997. In this case, Receipt of the Deposit, issued by NIBH, contained a wrong description "Escherichia coli DH10B/pTB1958" in the item "Identification of the Microorganism". The correct description is "Escherichia coli DH5α/pTB1958". A request for change of the description was filed as of February 5, 1997.

The transformants Escherichia coli DHSα/pTB2011 obtained in Reference Example 3 is deposited at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Numbers FERM BP-6012 since July 8, 1997; and at Institute for Fermentation, Osaka (IFO), located at 2-17-85 Jyuso-Honmati, Yodogawa-ku, Osaka-shi, Osaka 532-8686, Japan, under Accession Numbers IFO 16109 since July 7, 1997.

The transformants Escherichia coli DH5α/pTB2012 obtained in Reference Example 5 is deposited at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Numbers FERM BP-6013 since July 8, 1997; and at Institute for Fermentation, Osaka (IFO), located at 2-17-85 Jyuso-Honmati, Yodogawa-ku, Osaka-shi, Osaka 532-8686, Japan, under Accession Numbers IFO 16110 since July 7, 1997.

The transformants Escherichia coli DH5α/hTL4-pCR2.1 containing the nucleotide sequence encoding hTL4-2, obtained in Reference Example 7 is deposited at International Patent Organism Depositary, National Institute of Advanced Industrial Science and Technology (now-defunct National Institute of Bioscience and Human Technology (NIBH), Agency of Industrial Science and Technology, Ministry of International Trade and Industry), located at Center No. 6, 1-1-1 Higasi, Tukuba-shi, Ibaraki 305-8566, Japan, under Accession Numbers FERM BP-6958 since December 6, 1999; and at Institute for Fermentation, Osaka (IFO), located at 2-17-85 Jyuso-Honmati, Yodogawa-ku, Osaka-shi, Osaka 532-8686, Japan, under Accession Numbers IFO 16329 since October 27, 1999.

### EXAMPLES

The invention is specifically described below with reference to the following Reference Examples and Examples, but it is not limited thereto. The genetic procedures using Escherichia coli were based on methods described in "Molecular Cloning".

### REFERENCE EXAMPLE 1

### Cloning of cDNA Encoding Human-Derived TL4 Protein

The cloning of the cDNA was performed using GeneTrapper™ cDNA Positive Selection System (Gibco BRL).

The *Escherichia coli* DH12S strain of Superscript™ human liver cDNA library (Gibco BRL) was cultured in 100 µg/ml ampicillin-containing Terrific Broth [12 g/L Bacto-tryptone (Difco), 24 g/L Bacto-yeast extract (Difco), 2.3 g/L potassium monohydrogenphosphate, 12.5 g/L dipotassium hydrogenphosphate, 0.4% glycerol] at 30°C for 16 hr. Then, cells were harvested and a plasmid cDNA library was prepared using Qiagen Plasmid Kit (Qiagen). The plasmid cDNA library was purified and digested with Genell and ExoIII (both from Gibco BRL) to thereby prepare a single-stranded cDNA library.

On the other hand, a synthetic oligonucleotide (SEQ ID NO: 11) was used as a probe for screening the cDNA library. The probe was labeled by biotinylating its 3' end with TdT and biotin-14-dCTT (Gibco BRL). The single-stranded cDNA library was treated at 95°C for 1 min and then quickly cooled in ice. The biotinylated probe was added thereto, and hybridization was performed at 37°C for 1 hr and then at room temperature. After the hybridization, streptavidin beads in the GeneTrapper™ cDNA Positive Selection System (Gibco BRL) were added to the hybridization solution, which was then left at room temperature for 30 min while agitating every two minutes. Subsequently, the hybridization solution was placed in the magnet rack of the GeneTrapper™ cDNA Positive Selection System (Gibco BRL) and left for 2 min. The supernatant was discarded, and the magnet beads were washed with the wash buffer of the GeneTrapper™ cDNA Positive Selection System (Gibco BRL) three times. Subsequently, the magnetic beads were left in the magnet rack, and the supernatant was discarded. The elution buffer of the GeneTrapper™ cDNA Positive Selection System was added to the beads, which were then left for 5 min at room temperature and left in the magnetic rack for 5 min. Then, the resultant supernatant (DNA solution) was recovered.

To the thus obtained DNA solution, the synthetic oligonucleotide (SEQ ID NO: 11) was added as a primer and treated at 95° for 1 min. The repair enzyme of the GeneTrapper™ cDNA Positive Selection System was added to the solution, which was then left at 70°C for 15 min to thereby synthesize double-stranded DNA. The thus synthesized double-stranded DNA was introduced into *E. coli* DH10B using an electroporation apparatus (BioRad).

Screening was performed by colony PCR using the resultant transformant and two oligonucleotides (SEQ ID NOS: 12 and 13) as primers. Three clones (#9, #33 and #81) from which a 434 bp fragment had been amplified by PCR were selected as positive clones.

The thus selected *E. coli* clones were cultured, followed by DNA extraction. The DNA was reacted using Taq Dye Deoxyterminator Cycle Sequencing Kit (PerkinElmer), followed by determination of the nucleotide sequences of the cDNA fragments using ABI PRISM™ 377 DNA Sequencer (PerkinElmer). Of the three clones obtained, clone #9 and clone #33 contained the same DNA fragment and had a nucleotide sequence of 1491 bp with a poly(A)+ chain as shown in SEQ ID NO: 5. Clone #81 had a nucleotide sequence of 1353 bp with a poly(A)+ chain and a polyadenylation signal (AATAA) as shown in SEQ ID NO: 6. These cDNA fragments of the three clones contained one identical gene, which encoded TL4 protein consisting of the 240 amino acids as shown in SEQ ID NO: 1. Further, from the results of Kyte-Doolittle analysis, it was predicted that the hydrophobic region from Val³⁵ to Trp⁶³ was a transmembrane domain of this protein. This protein showed the highest homology to human lymphotoxin β with 33% homology at the amino acid level. This protein also showed 31% homology to human Fas ligand at the amino acid level. However, the results of phylogenetic tree analysis by J. Hein's method (based on PAM250 residue weight table) indicated that this protein had higher homology to human Fas ligand than to human lymphotoxin β.

Plasmid pTB1939 retaining clone #9, which is one of the DNAs encoding the protein of the invention, and plasmid pTB1940 retaining clone #81, which is another DNA encoding the protein of the invention, were introduced separately into *Escherichia coli* DH10B to thereby obtain transformed *Escherichia coli* DH10B/pTB1939 and *Escherichia coli* DH 10B/pTB1940.

### REFERENCE EXAMPLE 2

### Cloning of cDNA Encoding Mouse-Derived TL4 Protein

The cloning of the cDNA was performed by PCR. The *Escherichia coli* DH12S strain of Superscript™ mouse 8.5 day embryo-derived cDNA library (Gibco BRL) was cultured in 100 µg/ml ampicillin-containing Super Broth [32 g/L Bacto-tryptone (Difco), 20 g/L Bacto-yeast extract (Difco), 0.2 g/L NaCI] at 30°C for 16 hr. Then, a plasmid cDNA library was prepared using Qiagen Plasmid Kit (Qiagen), and used as a template.

The following two synthetic oligonucleotides were used for primers:

5'-TCTGCTCTGGCATGGAGAGTGTGGT-3' (SEQ ID NO: 14);

5'-CTATTGCTGGGTTTGAGGTGAGTC-3' (SEQ ID NO: 15).

A PCR reaction was performed in a system containing TaKaRa Ex Taq (Takara Shuzo) using a thermal cycler (GeneAmpR PCR System 2400; PerkinElmer) under the following conditions: 1 cycle of 94°C, 20 sec; 30 cycles of 94°C, 20 sec /55°C, 30 sec /72°C, 2 min; and leaving at 4°C.

The resultant amplified fragment was inserted into pT7Blue T-vector (Novagen) using DNA ligation kit version 2 (Takara Shuzo), and the resultant vector was introduced into *Escherichia coli* DH5α.

Plasmid DNA was extracted from the resultant transformant and reacted using Dye Terminator Cycle Sequencing FS Ready Reaction Kit (PerkinElmer), followed by determination of the nucleotide sequence of the cDNA fragment using 373A DNA Sequencer (PerkinElmer).

The resultant clone had the 795 bp nucleotide sequence as shown in SEQ ID NO: 8 containing the 717 bp nucleotide sequence as shown in SEQ ID NO: 7. This nucleotide sequence encoded mouse-derived TL4 protein consisting of the 239 amino acids as shown in SEQ ID NO: 2. This mouse-derived TL4 protein and the human-derived TL4 protein obtained in Reference Example 1 having the amino acid sequence as shown in SEQ ID NO: 1 has 78% homology at the amino acid level. The DNAs encoding them had 77% homology at the nucleotide level.

Plasmid pTB1958 retaining the resultant DNA encoding the mouse-derived TL4 protein was introduced into *Escherichia coli* DH5α to thereby obtain a transformant, *Escherichia coli* DH5α/pTB1958.

Subsequently, the nucleotide sequence around the initiation codon of the DNA encoding the mouse-derived TL4 protein of the invention was analyzed using Promoter Finder DNA Walking Kit (Clontech).

The mouse genomic DNA used was pre-digested with a restriction enzyme Scal, and an adaptor sequence was ligated to the 5' and 3' ends of the resultant fragments to enable the binding of primer AP1 (Clontech) and primer AP2 (Clontech).

(i) Primer AP1: (SEQ ID NO: 16)
5'-GTAATACGACTCACTATAGGGC-3'

(ii) Primer AP2: (SEQ ID NO: 17)
5'-ACTATAGGGCACGCGTGGT-3'

(iii) Adaptor sequence: (SEQ ID NO: 18)
5'-GTAATACGACTCACTATAGGGCACGCGTGGTCGACGGCCCGGGCTGGT-3'

The primary PCR was performed in a reaction solution containing this mouse genomic DNA solution, TaKaRa LA PCR Kit version 2 (Takara Shuzo), primer AP1 and synthetic oligonucleotide GSP1 using a thermal cycler (GeneAmpR PCR System 2400; PerkinElmer) under the following conditions: 94°C, 2 sec; 7 cycles of 72°C, 3 min; 94°C, 2 sec; 37 cycles of 68°C, 3 min; 68°C, 4 min; and leaving at 4°C.

(iv) Synthetic oligonucleotide GSP1: (SEQ ID NO: 19)
5'-CAGCCCAGCACCTAGCAGCAGCACCAG-3'

Subsequently, the resultant reaction solution was diluted 50-fold and supplied to the secondary PCR. The secondary PCR was performed in a reaction solution containing this primary PCR reaction solution, TaKaRa LA PCR Kit version 2 (Takara Shuzo), primer AP2 and synthetic oligonucleotide GSP2 using a thermal cycler (GeneAmpR PCR System 2400; PerkinElmer) under the following conditions: 94°C, 2 sec; 5 cycles of 72°C, 3 min; 94°C, 2 sec; 25 cycles of 68°C, 3 min; 68°C, 4 min; and leaving at 4°C.

(v) Synthetic oligonucleotide GSP2: (SEQ ID NO: 20)
5'-GCCGCCTGAATGGGATGTCCGTCTGTC-3'

An amplified fragment of approx. 1.1 kbp obtained from the Scal-digested genomic DNA solution was inserted into pT7 Blue T vector (Novagen) using DNA ligation kit version 2 (Takara Shuzo). The resultant vector was introduced into *E. coli* DH5α to thereby obtain transformant clones.

Plasmid DNA was extracted from the resultant transformant clones and reacted using Dye Terminator Cycle Sequencing FS Ready Reaction Kit (PerkinElmer), followed by determination of a part of the nucleotide sequence of the amplified fragment using 373A DNA sequencer (PerkinElmer). In the resultant clone, a sequence completely identical with the nucleotide sequence encoding from Met¹ (initiation codon) to Asp¹³ of the mouse-derived protein of the invention (i.e. the sequence spanning from position 1 to position 39 of the nucleotide sequence as shown in SEQ ID NO: 7) was contained. Therefore, it was confirmed that the synthetic oligonucleotide sequence (SEQ ID NO: 14) used in the cloning of the cDNA encoding the mouse-derived protein of the invention was a part of the actual DNA sequence encoding the mouse-derived protein of the invention.

### REFERENCE EXAMPLE 3

### Cloning of a Chromosomal Gene Comprising the Coding Region of the Mouse-Derived TL4 Protein Gene

A chromosomal DNA fragment encoding a region comprising the open reading frame of the mouse-derived TL4 protein was isolated by plaque hybridization using Lambda FIX™ II library (Stratagene) in which fragments of 129SVJ mouse chromosomal DNA partially digested with Sau3AI had been incorporated and, as a probe, a labeled mouse-derived TL4 protein cDNA. First, to the phage solution diluted to give a concentration of 1⁻¹⁰ x 10⁴ pfu (plaque-forming unit)/ml, an equal volume of culture broth of *E. coli* XL1-Blue MRA cultured in LB medium supplemented with 0.2% maltose and 10 mM MgSO₄ overnight at 30°C was added and mixed. Then, this mixture was incubated at 37°C for 10 min. To 200 µl of this mixture, 5 ml of top agarose (NZY medium [5 g/L NaCl, 2 g/L MgSO₄ · 7H₂O, 5 g/L yeast extract, 10 g/L NZ amine (adjusted to pH 7.5)] to which agarose was added to give a concentration of 0.7%) pre-warmed to 50°C was added, and overlayered uniformly on an NZY plate (1.5% agarose, 9 cm dish). Then, the plate was left stationary at 37°C for 9 hr. A nylon transfer membrane Hybond™-N+ (Amersham) on which the location of the plate had been marked was pressed on the plate for 1 min to thereby transfer phage particles appearing on the plate to the membrane. Then, the membrane was placed on Whatman 3MM filter paper (Whatman International) impregnated with a denaturing solution (1.5 M NaCI, 0.5 M NaOH) for 7 min with the phage-transferred surface upside. Subsequently, the membrane was left on the filter paper impregnated with a neutralizing solution (1.5 M NaCl, 0.5 M Tris-HCl (pH 7.2), 1 mM EDTA) for 3 min with the phage-transferred surface upside. After repeating this neutralization treatment again, the membrane was washed with 2 x SSC solution (0.3 M NaCl, 0.03 M sodium citrate). After air drying, the membrane was placed on the filter paper impregnated with 0.4 M NaOH for 20 min with the phage-transferred surface upside, washed with 5 x SSC solution (0.75 M NaCl, 75 mM sodium citrate) and placed in a hybridization bag. Five milliliters of the hybridization buffer of ECL gene detection system (Amersham) was added to this bag, and pre-hybridization was performed at 42°C for 1 hr.

On the other hand, the PCR-amplified DNA fragment corresponding to the open reading frame (720 bp) of the cDNA of the mouse-derived TL4 protein was thermally denatured. To the resultant DNA fragment, equal volumes of the labeling reagent of the ECL gene detection system and glutaraldehyde were added and incubated at 37°C for 5 min to thereby label the DNA fragment. A 10 µl aliquot of this labeled DNA fragment was added to each pre-hybridization bag and incubated at 42°C for 1 hr. Then, the membrane was taken out of the bag and washed with the primary washing buffer (6 M urea, 4 g/L SDS, 25 ml/L 20 x SSC), which was pre-warmed and retained at 42 °C, for 20 min. This washing was repeated again. Subsequently, the membrane was washed at room temperature with the secondary washing buffer (2 x SSC) for 5 min. This washing was repeated again. Subsequently, the membrane was soaked in the detection reagent of the ECL gene detection system for 1 min, placed upon an X-ray film, and exposed to light. After 1 hr, the membrane was removed from the film, which was then developed to select positive clones. The thus selected clones were further subjected to the secondary screening that was performed in the same manner as described above. Finally, five candidate clones (#2, #3, #4, #5 and #6) were obtained. The results of PCR reactions revealed that clone #1 and clone #6 encompassed the entire region of the gene encoding the mouse-derived TL4 protein.

Subsequently, subcloning was performed for the purpose of determining the nucleotde sequence of the chromosomal DNA comprising the coding region of the mouse-derived TL4 protein gene. First, clone #6 obtained above was digested with a restriction enzyme XbaI and electrophoresed using 0.7% agarose gel. A DNA fragment of approx. 9 kb which was believed to contain the coding region of the mouse-derived TL4 protein gene was cut out, and recovered/purified using QIAquick Gel Extraction Kit (Qiagen). On the other hand, a cloning vector pUC 19 was digested with a restriction enzyme Xbal and electrophoresed using 1.0% agarose gel. A DNA fragment corresponding to 2.7 kb was cut out and recovered/purified using QIAquick Gel Extraction Kit (Qiagen), followed by dephosphorylation of the ends with bovine small intestine-derived alkaline phosphatase CIAP (Takara Shuzo). To this CIAP-treated pUC19, the above-prepared DNA fragment derived from clone #6 was ligated using DNA Ligation Kit Ver. 2 (Takara Shuzo). The resultant vector was introduced into *E. coli* DH5α. From the resultant ampicillin resistant clones, plasmid DNAs into which the DNA fragment of interest had been inserted were selected and isolated. With respect to the nucleotide sequence of the cloned DNA fragment derived from clone #6, sequencing reactions using various synthetic oligo-DNAs as primers and Dye Terminator Cycle Sequencing FS Ready Reaction Kit (PerkinElmer) were performed in GeneAmpR PCR System 2400 according to the protocol attached. The resultant sample was sequenced by DNA Sequencer 373A (PerkinElmer). The nucleotide sequence thus obtained was confirmed with a gene analysis software Lasergene (DNA STAR). As a result, it was found that the chromosomal gene encoding the mouse-derived TL4 protein is composed of four exons.

The thus obtained plasmid retaining the Xbal DNA fragment derived from clone #6 comprising the coding region of the mouse-derived TL4 protein was designated pTB2011. The transformant obtained by introducing this plasmid into *Escherichia coli* DH5α was designated *Escherichia coli* DH5α/pTB2011.

### REFERENCE EXAMPLE 4

### Expression of the Extracellular Domain of Human-Derived TL4 Protein in Pichia Yeast Host and Western Blot Analysis

pPICZ αA (Invitrogen) was used for allowing the expression of the extracellular domain of the human-derived TL4 protein in *Pichia pastoris,* a yeast. This vector contains a gene encoding the secretion signal α-factor of *Saccharomyces cerevisiae* (budding yeast) capable of functioning even in *Pichia* yeast downstream of the promoter of *Pichia pastoris'*s alcohol oxidase gene (AOX1), and a multi-cloning site that follows the above-mentioned secretion signal gene. Thus, this vector is capable of allowing the recombinant protein to be secreted into the medium.

First, in order to prepare a DNA fragment encoding the extracellular domain of the human-derived TL4 protein of the invention by PCR, the following two primers were synthesized with a DNA synthesizer (Oligo 1000M; Beckman). 
(i) 5'-primer: (SEQ ID NO: 21)
5'-ACGAATTCCAAGAGCGAAGGTCTCACGAGGTC-3'
 (This primer has an EcoRI recognition sequence and 24 nucleotides, located 3' to the recognition sequence, encoding the eight amino acids starting from the N-terminal Gln⁸⁵ of the extracellular domain of the human-derived TL4 protein. 
(ii) 3'-primer: (SEQ ID NO: 22)
5'-AGTCTAGACTCCTTCCTTCACACCATGAAAGCCCC-3'
 (This primer has an Xbal recognition sequence and a sequence, located 3' to the recognition sequence, complementary to a termination codon (TGA) and the 15 nucleotides encoding the five C-terminal amino acids of the extracellular domain of the human-derived TL4 protein.)

A 50 µl solution containing 50 pmol each of the resultant primers, 100 ng of plasmid pTB1939 obtained in Reference Example 1, 10 nmol each of dATP, dCTP, dGTP and dTTP, 2.5 units of native Pfu DNA polymerase (Stratagene) and 5 µl of native Pfu buffer (Stratagene) was prepared, and PCR was performed using a thermal cycler (GeneAmpR PCR System 2400; PerkinElmer) under the following conditions: 94°C, 1 min; 30 cycles of 98°C, 20 sec /55°C, 30 sec /68°C, 2 min; and finally 72°C, 5 min. PCR product was recovered from the resultant reaction solution, digested with EcoRI and XbaI, and ligated to pPICZ αA pre-digested and linearized with EcoRI and XbaI, to thereby obtain a circularized plasmid. This plasmid DNA was cut again at the SacI unique restriction site of the AOX1 locus, linearized, and introduced into *Pichia pastoris* KM71 by electroporation. From the resultant transformants, several Zeocin™ resistant clones were selected which were capable of growing on 100 µl/ml Zeocin™-containing YPD agar medium [1% yeast extract (Difco), 2% Bacto-peptone (Difco), 2% glucose (Wako Purechemical), 2% agar powder (Wako Purechemical)]. Chromosomal DNA was prepared from each clone. Using the chromosomal DNA as a template, PCR was performed in order to confirm the integration of the introduced plasmid DNA into the chromosome. Those clones in which this integration had been confirmed were selected as transformants for expressing the recombinant protein of interest.

The recombinant protein was expressed by the following procedures. Briefly, one platinum loopful of colony of the transformant for expressing the human-derived TL4 protein was inoculated into 25 ml of BMGY medium [1% yeast extract, 2% peptone, 100 mM potassium phosphate (pH 6.0), 1.34% yeast nitrogen base with ammonium sulfate without amino acids (Difco), 4 x 10⁻⁵ % biotin, 1% glycerol] and cultured at 30°C for 20 hr. Then, cells were harvested by centrifugation, resuspended in BMMY medium [1% yeast extract, 2% peptone, 100 mM potassium phosphate (pH 6.0), 1.34% yeast nitrogen base with ammonium sulfate without amino acids (Difco), 4 x 10⁻⁵ % biotin, 0.5% methanol] to give an OD600 value of 1.0, and cultured at 30°C. After one or two days, the culture broth was sampled and centrifuged to thereby obtain a culture supernatant.

Western blotting using this culture supernatant was performed as described below. First, a peptide comprising a part of the amino acid sequence of the extracellular domain of the human-derived TL4 protein (i.e. the amino acid sequence spanning from position 166 to position 180 of the amino acid sequence as shown in SEQ ID NO: 1) was synthesized. Then, a rabbit antiserum that recognizes this peptide was prepared according to known methods. Subsequently, 5 µl of the above culture supernatant was mixed with 5 µl of a sample treating solution (0.25 M Tris-HCl, 2% SDS, 30% glycerol, 10% β-mercaptoethanol, 0.01% bromophenol blue, pH 6.8), treated at 95°C for 5 min, and subjected to SDS-polyacrylamide gel electrophoresis (using 10-20% gradient gel). After the electrophoresis, the protein was transferred onto a nitrocellulose membrane (Phannacia) using a protein blotting apparatus (SemiPhor™; Hoefer Phannacia BioTech). The membrane was blocked with 3% gelatin-containing TBS (20 mM Tris, 500 mM NaCl, pH 7.5), washed with TTBS (0.05% Tween 20-containing TBS), and then reacted with the above-mentioned rabbit antiserum diluted 2000-fold with 1.0% gelatin-containing TTBS at room temperature for 2 hr. After completion of the reaction, the membrane was washed with TTBS twice and then reacted with an alkaline phosphatase (AP)-labeled goat anti-rabbit IgG antibody diluted 3000-fold with 1.0% gelatin-containing TTBS at room temperature for 1 hr. The membrane was washed with TTBS twice and further washed with TBS once, followed by detection of the protein with an AP color development kit (BioRad).

A major band was recognized at around 20 kD in the culture supernatant from the expression vector-introduced clones, and the intensity of its signal increased with the passage of time. On the other hand, no signal was recognized in the culture supernatant from control clones into which pPICZ αA was introduced.

### REFERENCE EXAMPLE 5

### Cloning of cDNA Encoding Rat-Derived TL4 Protein

The cloning of a cDNA encoding rat-derived TL4 protein was performed by PCR.

The *Escherichia coli* DH12S strain containing Superscript™ rat liver cDNA library (Gibco BRL) was cultured in 100 µg/ml ampicillin-containing Terrific Broth [12 g/L Bacto-tryptone (Difco), 24 g/L Bacto-yeast extract (Difco), 2.3 g/L potassium monohydrogenphosphate, 12.5 g/L dipotassium hydrogenphosphate, 0.4% glycerol] at 30°C for 16 hr. Then, cells were harvested and a plasmid cDNA library was prepared using Qiagen Plasmid Kit (Qiagen).

PCR was performed in a reaction system using the above DNA as a template, the following two synthetic oligonucleotides as primer DNAs and TaKaRa LA Taq (Takara Shuzo) as a DNA polymerase.

5'-CCTGACCCTGGGCTTCTGAGCCTC-3' (SEQ ID NO: 23)

5'-TCCACAAAATCCATTGTCGTCATAGCC-3' (SEQ ID NO: 24)

The reaction was performed using a thermal cycler (GeneAmpR PCR System 2400; PerkinElmer) under the following program: 1 cycle of 94°C, 1 min; 35 cycles of 98°C, 20 sec /55°C, 30 sec /72°C, 3 min; 1 cycle of 72°C, 2 min; and leaving at 4°C. An aliquot of the resultant reaction solution was electrophoresed on 1.0% agarose gel. A single band amplified by PCR corresponding to the DNA fragment was confirmed. Subsequently, the DNA fragment was recovered from the gel using QIAquick Gel Extraction Kit (Qiagen) and ligated to the T cloning site of pT7Blue T-vector (Novagen) using DNA Ligation Kit Ver. 2 (Takara Shuzo) in order to determine the nucleotide sequence. The ligation solution was introduced into *E. coli* DH5α. From the resultant transformants, two clones of ampicillin resistant transformants growing on ampicillin-containing LB agar medium were selected, followed by preparation of plasmid DNA from each each clone. In order to determine the nucleotide sequence of the insert DNA in each clone, cycle sequencing reactions were performed using each plasmid DNA as a template, two commercial primer DNAs (PRM-007 and PRM-008; Toyobo) and oligo-DNAs synthesized with a DNA synthesizer (Oligo 1000M; Beckman) as primer DNAs, and Thermo Sequenase™ dye terminator cycle sequencing pre-mix kit (Amersham) in GeneAmpR PCR System 2400 according to the conditions described in the attached protocol. The resultant sample was analyzed with DNA Sequencer 373A (Perkin Elmer).

The resultant nucleotide sequences were analyzed with a gene analysis software Lasergene (DNASTAR). As a result, it was found that both clones contained at their T cloning site a 784 bp DNA fragment comprising an open reading frame consisting of the 717 base pairs as shown in SEQ ID NO: 10 encoding the rat-derived TL4 protein consisting of the 239 amino acids as shown in SEQ ID NO: 3. This rat-derived TL4 protein and the human-derived TL4 protein obtained in Reference Example I having the amino acid sequence as shown in SEQ ID NO: 1 had 75% homology at the amino acid level, and the DNAs encoding the two proteins had 74% homology at the nucleotide level. Also, this rat-derived TL4 protein and the mouse-derived TL4 protein obtained in Reference Example 2 having the amino acid sequence as shown in SEQ ID NO: 2 had 96% homology at the amino acid level, and the DNAs encoding the two proteins had 94% homology at the nucleotide level.

Plasmid pTB2012 retaining the resultant DNA encoding the rat-derived TL4 protein was introduced into *Escherichia coli* DH5α to thereby obtain a transformant *Escherichia coli* DH5α/pTB2012.

### REFERENCE EXAMPLE 6

### Production of Soluble Human TL4 in Insect Cell Expression System

PCR was performed using as a template plasmid pTB 1940 into which a DNA encoding the human TL4 protein was inserted and as primers a synthetic oligonucleotide 5'-GAATTCGATACAAGAGCGAAGGTCTCACGAGGTC-3' (SEQ ID NO: 26) in which a restriction site of EcoRI is added to the 5' end and another synthetic oligonucleotide 5'-AAATCTAGATCCTTCCTTCACACCATGAAAGCCCC-3' (SEQ ID NO: 27) in which a restriction site of Xbal is added to the 3' end. As a result, an amplified DNA fragment for a soluble TL4 protein encoding a sequence from Ile⁸⁴ to Val²⁴⁰ (which corresponds to the extracellular domain of the TL4 protein) was obtained. The PCR reaction was performed in DNA Thermal Cycler 9600 at 94°C for 1 min, and then 25 cycles of 98°C, 10 sec /55°C, 5 sec /72°C, 1 min were repeated using Ex Taq DNA polymerase. The thus obtained amplified fragment was digested with restriction enzymes EcoRI and XbaI. pCMV-FLAG plasmid was also digested with restriction enzymes EcoRI and XbaI to thereby obtain a DNA fragment encoding the signal sequence of preprotrypsin and FLAG protein added as a tag for the purpose of facilitating purification and detection. The amplified DNA fragment encoding a soluble TL4 digested with restriction enzymes was ligated to the 3' end of the DNA fragment encoding the preprotrypsin-FLAG protein. The resultant DNA fragment encoding the preprotrypsin-FLAG protein-soluble human TL4 protein was digested with restriction enzymes Sacl and XbaI, and inserted into an insect cell expression vector pFAST Bac1 (Gibco BRL Lifetech) digested with the same restriction enzymes Sacl and Xbal. The resultant TL4 expression plasmid pFAST Bacl/shTL4 was expected to allow the TL4 protein to be secreted into cell culture supernatant utilizing preprotrypsin in insect cells and to allow production of the TL4 protein as a fusion protein with a FLAG tag added thereto.

The operations described so far were performed using Bac-to-Bac Baculovirus Expression Systems (Gibco BRL Lifetech), and the experimental methods were in accordance with the attached protocol. Briefly, the resultant recombinant plasmid pFAST Bacl/shTL4 into which the DNA encoding the human TL4 protein had been inserted was introduced into *E. coli* DH10Bac contained in the Systems to thereby obtain transformant cells. From these cells, recombinant Bacmids were recovered. The resultant Bacmid was transfected into Sf9 insect cells using Cell-Fectin reagent appended to the Systems to thereby obtain a recombinant baculovirus. The Sf9 insect cells were re-infected with this baculovirus and cultured for 4 or 5 days. The FLAG-human TL4 fusion protein secreted into the culture supernatant was purified with an anti-FLAG antibody column and designated shTL4.

### REFERENCE EXAMPLE 7

### Cloning of cDNA Encoding Human Liver-Derived Novel Fas Ligand-Like Soluble Protein and Nucleotide Sequence Thereof

PCR was performed using human liver cDNA as a template and primer 1 (SEQ ID NO: 28) and primer 2 (SEQ ID NO: 29). The composition of the reaction solution was as follows: 33.5 ng of the above-mentioned cDNA as a template, 1/50 volume of Advantage 2 Polymerase Mix (Clontech), 20 µM each of primer 1 (SEQ ID NO: 28) and primer 2 (SEQ ID NO: 29), 2.5 mM dNTPs, and 1/10 volume of the buffer appended to the enzyme. The total volume of the reaction solution was 50 µl. The PCR reaction was performed as follows: (i) 1 cycle of 95°C, 30 min, then (ii) 30 cycles of 95°C, 10 sec /58°C, 10 sec /72°C, 45 sec, and finally (iii) extension reaction at 72°C for 2 min. As a result of the PCR, two reaction products of 723 bp and 615 bp were generated. The reaction product of 615 bp was recovered from the gel and purified using QIAquick Gel Extraction Kit (Qiagen) according to the manufacturer's instructions. The purified product was subcloned into a plasmid vector pCR2.1-TOPO Vector according to the instructions of TA Cloning Kit (Invitrogen). The resultant vector was introduced into *E. coli* DH5α. Those clones having a cDNA of interest were selected in ampicillin-containing LB agar medium. Then, the sequences of the individual clones were analyzed. As a result, a cDNA sequence of 612 bp (SEQ ID NO: 30) encoding a novel Fas ligand-like soluble protein was obtained. The novel Fas ligand-like soluble protein having the amino acid sequence deduced from this cDNA (SEQ ID NO: 31) was designated hTL4-2.

### REFERENCE EXAMPLE 8

### Production of Soluble Mouse TL4 in Insect Cell Expression System

PCR was performed using as a template plasmid pTB 1958 into which a DNA encoding the mouse TL4 protein (SEQ ID NO: 2) was inserted and as primers a synthetic oligonucleotide: 5'-GTAGAATTCGGCCAACCCAGCAGCACATCTTAC-3' (SEQ ID NO: 33) in which a restriction site ofEcoRi is added to the 5' end and another synthetic oligonucleotide: 5'-AAATCTAGATATTGCTGGGTTTGAGGTGAGTCC-3' (SEQ ID NO: 34) in which a restriction site of Xbal is added to the 3' end. As a result, an amplified DNA fragment for a soluble TL4 protein encoding a sequence from Ala⁹⁰ to Val²³⁹ (which corresponds to the extracellular domain of the TL4 protein) was obtained. The PCR reaction was performed in DNA Thermal Cycler 9600 at 94°C for 1 min, and then 25 cycles of 98°C, 10 sec → 60°C, 5 sec → 72°C, 1.5 min were repealed using Ex Taq DNA polymerase. The thus obtained amplified fragment was digested with restriction enzymes EcoRI and Xbal. pCMV-FLAG plasmid was also digested with restriction enzymes EcoRI and Xbal to thereby obtain a DNA fragment encoding the signal sequence of preprotrypsin and FLAG protein added as a tag for the purpose of facilitating purification and detection. The amplified DNA fragment encoding a soluble TL4 digested with restriction enzymes was ligated to the 3' end of the DNA fragment encoding the preprotrypsin-FLAG protein. The resultant DNA fragment encoding the preprotrypsin-FLAG protein-soluble mouse TL4 protein was digested wth restriction enzymes SacI and Xbal, and inserted into an insect cell expression vector pFAST Bac1 (Gibco BRL Lifetech) digested with the same restriction enzymes Sacl and XbaI. The resultant TL4 expression plasmid pFAST Bac1/smTL4 was expected to allow the TL4 protein to be secreted into cell culture supernatant utilizing preprotrypsin in insect cells and to allow production of the TL4 protein as a fusion protein with a FLAG tag added thereto.

The operations described so far were performed using Bac-to-Bac Baculovirus Expression Systems (Gibco BRL Lifetech), and the experimental methods were in accordance with the attached protocol. Briefly, the resultant recombinant plasmid pFAST Bac1/smTL4 into which the DNA encoding the mouse TL4 protein had been inserted was introduced into *E. coli* DH10Ba contained in the Systems to thereby obtain transformant cells. From these cells, recombinant Bacmids were recovered. The resultant Bacmid was transfected into Sf9 insect cells using Cell-Fectin reagent appended to the Systems to thereby obtain a recombinant baculovirus. The Sf9 insect cells prepared to give a concentration of 1.5 x 10⁶ cells/ml were re-infected with 1/20 volume of this recombinant baculovirus (relative to the total volume of the cells), and cultured for 2 days. The FLAG-mouse TL4 fusion protein secreted into the culture supernatant was recovered. This protein was concentrated with a UF membrane (MWCO 3000 0.1 m²) and suspended in TBS (50 mM Tris-HCl, 150 mM NaCI, pH 7.4). Then, the protein was purified with an anti-FLAG M2 antibody column, followed by purification of the eluate with a Sephadex G-25 column. Then, the eluate was again purified with an anti-FLAG M2 antibody column. The resultant fractions were examined by SDS-PAGE, and those fractions with high purity were recovered collectively. This pool of fractions was concentrated with Centriplus 10K, purified with a Sephadex G-25 column and then suspended in PBS. The resultant FLAG-mouse TL fusion protein was designated smTL4.

### EXAMPLE 1

### Inhibition of Caspase-3 Processing by TL4

A basal medium was prepared by mixing equivalent amounts of Ham's F-12 medium and Leibovitz's L-15 medium in a collagen I-coated 225 cm² culture flask (Iwaki), to which, 1% BSA, 5 mM glucose (Wako), 10⁻⁸ M dexamethasone (Wako) and 10⁻⁸ M bovine insulin (Gibco) were added (the indicated concentrations are final concentrations). Normal human hepatocytes (Cell System, #3716) were suspended in this medium at 5000 cells/well/100 ml and cultured in a CO2 incubator for 24 hr. Subsequently, the medium was replaced with the basal medium supplemented with newborn calf serum (Gibco) to give a final concentration of 1%. Apoptosis was induced by adding 1.33 mM actinomycin D and human TNFα (Genzyme) to give a final concentration of 100 ng/ml.

Six hours prior to the stimulation to induce apoptosis, hTL4-2 obtained in Reference Example 7 was added to the cells. The cells were recovered with a scraper 4 hr and 14 hr after the induction of apoptosis, and stored at -80°C until use for protein extraction. Protein extraction was performed as described below. Briefly, 0.5 ml of a cell-lysis solution [50 mM Tris-HCl (pH 8.0), 1 mM EDTA, 150 mM NaCI, 1% NP-40, 100 µM APMSF ((p-amidinophenyl) methanesulfonyl fluoride hydrochloride), 1 µg/ml pepstatin A, 50 µg/ml antipain] was added to the cells stored at -80°C. The cells were suspended and centrifuged at 15000 rpm for 10 min, followed by recovery of the supernatant as a cell extract. The proteins in each cell extract were quantitatively determined with Protein Assay Kit (BioRad). SDS-PAGE was performed with the amount of proteins being 20-80 µg per lane. Then, the proteins were transferred onto an Immobilon-P Membrane at 0.8 mA/cm² for 2 hr. The resultant membrane was blocked in Block Ace (Dainippon Pharmaceutical) at 4°C overnight. After blocking, the membrane was reacted with anti-caspase-3 antibody (Pharmingen; 65906E) diluted to 1/1000 with 25% Block Ace, for 30 min at room temperature. Then, the membrane was washed three times with PBS containing Tween 20 at a final concentration of 0.05% (PBST), reacted with HRP-labeled anti-rabbit Ig antibody (SantaCruz; sc-2004) diluted to 1/5000 for 30 min at room temperature, and washed with PBST five times. The bands of precursor caspase-3 and mature caspase-3 were detected with ECL plus (Amersham). As control, anti-actin antibody (SantaCruz; sc-1616) and HRP-labeled anti-goat Ig antibody (SantaCruz; sc-2020) as a secondary antibody were used.

The results are shown in Figs. 1 and 2. As shown in Fig. 1, the amount of mature caspase-3 in the sample pretreated with hTL4-2 was decreased, compared to the amount in the non-pretreated sample at 4 hr after the apoptosis stimulation. At 14 hr after the apoptosis stimulation, the amounts of mature caspase-3 were almost equal in these samples, but the amount of precursor caspase-3 in the sample pretreated with hTL4-2 was almost equal to the amount in the non-stimulated sample. On the other hand, the amount of precursor caspase-3 in the non-pretreated sample was clearly decreased, compared to the amount in the non-stimulated sample. Therefore, it was revealed that the processing of caspase-3 by TNFα can be inhibited by pretreatment with hTL4-2. Since the processing of caspase-3 is essential for the activation of caspase-3, it was suggested that hTL4-2 inhibits the activation of caspase-3. From these results, we can expect that hTL-4-2 not only inhibits apoptosis by TNFα but also improves diseases associated with apoptosis in which caspase-3 is mainly involved.

### EXAMPLE 2

### Inhibition of Caspase-3 or Caspase-8 Activation by TL4

From the results of Example 1, it has become clear that hTL4-2 inhibits the processing of caspase-3 by TNFα. It is known that caspases are activated by the processing thereof. Therefore, the state of activation of caspase-3 or caspase-8 was examined using its enzyme activity as an indicator. Briefly, individual enzyme activities in the cell extracts obtained in Example 1 were measured using ApoAlert caspase-3 assay kit or ApoAlert caspase-8 assay kit (Clontech). The results are shown in Figs. 3 and 4. The enzyme activity of caspase-3 or caspase-8 in the cell extract was increased by the addition of TNFα, but this increase of activity was inhibited by pretreatment with hTL4-2. Also, when an inhibitor of each caspase was added at the time of enzyme reaction, the enzyme activity was decreased to background. From these results, it has become cleat that the activation of caspase-3 or caspase-8 occurs specifically by TNFα and that hTL4-2 inhibits that activation. Thus, it has been found that TL4 inhibits cell death induced by the activation of not only caspase-3 but also caspase-8.

### EXAMPLE 3

### Activation of Nucleofactor-kB (NF-kB) by TL4

Some of cell death-inhibitory stimuli are known to activate NF-kB. Whether or not the activation of NF-kB occurs was examined according to the manner described in Example 1 using Mercury Pathway Profiling System (Clontech). Briefly, normal human hepatocytes were suspended in the 10% FCS-containing basal medium used in Example 1 and plated on collagen I-coated 24-well plates (Falcon) at 2x10⁴ cells/well/ml. Cells were cultured overnight. A mixture of 98.5 µl of DMEM, 1.5 µl of Fugene 6 (Boehringer) and 1 µl of one plasmid [pNF-kB-SEAP, pTAL-SEAP (negative control) or pSEAP2-control (positive control)] was prepared, and further mixed with 500 µl of 10% FCS-containing DMEM. The resultant mixture was added to each well. After overnight culture, cells were washed with 1% NBCS-containing basal medium twice. hTL4-2 or TNFα was diluted with a medium to give a final concentration of 100 ng/ml; and anti-Fas antibody was diluted with a medium to give a final concentration of 500 ng/ml. Then, 600 µl of one of these preparations was added to each well.

Fifty µl samples were taken at 1, 2, 3, 4, 5, 6, 8 and 24 hr after the above addition and stored at -20°C until use for the measurement of SEAP activity in culture broth.

The SERF activity was measured using Great EscAPe SEAP Reporter System (Clontech). The results are shown in Fig. 5. When hTL4-2 or TNFα was added, the SEAP activity in culture supernatant increased with time. On the other hand, when anti-Fas antibody was added, no increase in activity was observed. In other words, it has become clear that the NF-kB transcription activity is increased by hTL4-2 and TNFα in normal human hepatocytes. From these results, it has been revealed that TL4 promotes the increase in the expression of cell death inhibitory factors or survival factors, which are controlled by NF-kB.

### INDUSTRIAL APPLICABILITY

The protein of the invention, a partial peptide or a salt thereof has the caspase 3 inhibiting activity, and thus is useful as a pharmaceutical such as a prophylactic and/or therapeutic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.

## Claims

1. A caspase 3 inhibitor comprising a protein comprising the same or substantially the same amino acid sequence as that represented by SEQ ID NO: 1, 2, 3 or 31 or a salt thereof.

2. The inhibitor according to claim 1 wherein substantially the same amino acid sequence as that represented by SEQ ID NO: 1, 2 or 3 includes an amino acid sequence having the amino acid sequences from aa 8 to aa 21, aa 54 to aa 59, aa 93 to aa 102, as 109 to aa 116, as 118 to aa 126, aa 128 to aa 134, aa 144 to aa 149, aa 162 to aa 170, aa 176 to aa 182, aa 184 to aa 189, aa 193 to aa 213, aa 215 to aa 219, and aa 228 to aa 240 in the amino acid sequence represented by SEQ ID NO: 1.

3. A caspase 3 inhibitor comprising a partial peptide of the protein comprising the same or substantially the same amino acid sequence as that represented by SEQ ID NO: 1, 2, 3 or 31 or a salt thereof.

4. The inhibitor according to claim 3 wherein the partial peptide includes the peptide consisting of the amino acid sequence from aa 84 to aa 240 in the amino acid sequence represented by SEQ ID NO: 1.

5. A caspase 3 inhibitor comprising a DNA comprising a DNA encoding the protein comprising the same or substantially the same amino acid sequence as that represented by SEQ ID NO: 1, 2, 3 or 31 or a partial peptide thereof.

6. The inhibitor according to claim 5 wherein the DNA includes a DNA comprising the nucleotide sequence represented by any one of SEQ ID NOs: 4 to 10 and 30.

7. The inhibitor according to claim 1, 3 or 5, which is a prophylactic and/or therapeutic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.

8. A diagnostic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect, which comprises an antibody to a protein comprising the same or substantially the same amino acid sequence as that represented by SEQ ID NO: 1, 2, 3 or 31, a partial peptide or a salt thereof.

9. A diagnostic agent for AIDS, Alzheimer's disease, Parkinson's disease, amyotrophic lateral sclerosis, pigmentary retinopaihy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect, which comprises a DNA comprising a DNA encoding a protein comprising the same or substantially the same amino acid sequence as that represented by SEQ ID NO: 1, 2, 3 or 31 or a partial peptide thereof.

10. A method of inhibiting caspase 3 in a mammal, which comprises administering an effective amount of a protein comprising the same or substantially the same amino acid sequence as that represented by SEQ ID NO: 1, 2, 3 or 31 or a salt thereof to the mammal.

11. A method of preventing and/or treating AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect in a mammal, which comprises administering an effective amount of a protein comprising the same or substantially the same amino acid sequence as that represented by SEQ ID NO: 1, 2, 3 or 31 or a salt thereof to the mammal.

12. A method of inhibiting caspase 3 in a mammal, which comprises administering an effective amount of a DNA comprising a DNA encoding a protein comprising the same or substantially the same amino acid sequence as that represented by SEQ ID NO: 1, 2, 3 or 31 or a partial peptide thereof to the mammal.

13. A method of preventing and/or treating AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect in a mammal, which comprises administering an effective amount of a DNA comprising a DNA encoding a protein comprising the same or substantially the same amino acid sequence as that represented by SEQ ID NO: 1,2, 3 or 31 or a partial peptide thereof to the mammal.

14. A use of a protein comprising the same or substantially the same amino acid sequence as that represented by SEQ ID NO: 1, 2, 3 or 31 or a salt thereof for the production of a caspase 3 inhibitor.

15. A use of a protein comprising the same or substantially the same amino acid sequence as that represented by SEQ ID NO: 1, 2, 3 or 31 or a salt thereof for the production of a prophylactic and/or therapeutic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.

16. A use of a DNA comprising a DNA encoding a protein comprising the same or substantially the same amino acid sequence as that represented by SEQ ID NO: 1, 2, 3 or 31 or a partial peptide thereof for the production of a caspase 3 inhibitor.

17. A use of a DNA comprising a DNA encoding a protein comprising the same or substantially the same amino acid sequence as that represented by SEQ ID NO: 1, 2, 3 or 31 or a partial peptide thereof for the production of a prophylactic and/or therapeutic agent for AIDS, Alzheimer's disease, Parkinson's disease, amyotrophic lateral sclerosis, pigmentary retinopathy, cerebellar degeneration, myelodysplastic syndrome, aplastic anemia, sideroblastic anemia, myocardial ischemia, conduction disturbance, chronic cardiac failure, graft-versus-host disease, or congenital or acquired enzymatic defect.
